(19) Europäisches Patentamt
European Patent Office
Office européen des brevets

(11) EP 2 512 386 B1

(12) **EUROPEAN PATENT SPECIFICATION**

(45) Date of publication and mention of the grant of the patent:
**02.11.2016 Bulletin 2016/44**

(21) Application number: **10801705.4**

(22) Date of filing: **17.12.2010**

(51) Int Cl.:
***A61F 5/00*** *(2006.01)*

(86) International application number:
**PCT/IE2010/000076**

(87) International publication number:
**WO 2011/073970 (23.06.2011 Gazette 2011/25)**

# (54) A GASTROINTESTINAL IMPLANT DEVICE

GASTROINTESTINALE IMPLANTATVORRICHTUNG

DISPOSITIF D'IMPLANT GASTRO-INTESTINAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2009 US 287946 P**

(43) Date of publication of application:
**24.10.2012 Bulletin 2012/43**

(73) Proprietor: **Coloplast A/S**
**3050 Humlebaek (DK)**

(72) Inventor: **BEHAN, Niall**
**Kilcolgan**
**County Galway (IE)**

(56) References cited:
**WO-A1-2008/121409**
**WO-A1-2009/126268**
**WO-A2-2007/107990**
**US-A1- 2005 125 075**
**US-A1- 2005 267 499**
**US-A1- 2006 264 983**
**US-A1- 2007 198 048**
**US-A1- 2008 109 087**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Introduction

The invention relates to a gastrointestinal implant device. There are several procedures and devices for treatment of obesity. Whilst many of these devices are successful in the short term various problems can arise because the patient does not achieve a feeling of satiety (fullness) after eating.

WO2009/126268A describes a pyloric valve comprising a plurality of axially connected blocking members.

US2008/109087A describes a pyloric obesity valve including a tubular valve body according to the preamble of claim 1.

US2007/198048A describes a device for treating reflux from stomach to an oesophagus. WO2007/107990A describes a gastrointestinal device comprising a band for maintaining pyloric opening at a predetermined size.

US2005/267499A describes a prosthetic device that is positionable within the gastro-esophageal junction region.

US2005/125075A describes a sleeve to limit absorption of nutrients in the duodenum and an anchor for attaching the device to the duodenum.

WO2008/121409A describes an intra-gastric implant device.

US2006/064983A describes a gastric restrictor.

According to the invention there is provided a gastrointestinal implant device as defined in claim 1. The invention according to claim 1 is disclosed on page 30, line 13 - page 32, line 2 and Figures 72-77. Preferred embodiments are defined in dependent claims 2-15.

In one example the valve is configured to open only when a pre-set back pressure on the valve has been overcome.

In one example the support structure comprises a scaffold to which the valve is mounted. The support structure may comprise a luminal prosthesis.

In one example the support structure comprises a scaffold to which the valve is mounted and a luminal prosthesis. The scaffold may be releasably mountable to the luminal prosthesis.

In one example the sleeve is mounted to the support structure. In one case, the sleeve is releasably mountable to the support structure. In one case the support structure comprises a scaffold and the sleeve is mounted to the scaffold.

In one example the support structure comprises a stent-like structure.

In one example the support structure comprises a stent-like scaffold.

In one example the support structure comprises a luminal prosthesis for deployment at the pylorus and a scaffold to which the valve is mounted, the scaffold being releasably mountable to the pre-deployed luminal prosthesis. The scaffold may be releasably engagable with the luminal prosthesis. The scaffold may comprise engagement elements which are releasably engagable with the luminal prosthesis. In one case the engagement elements comprise protrusions which are releasably engagable with the luminal prosthesis.

In one example the luminal prosthesis comprises a mesh. The mesh may be coated with a coating. The protrusions may engage with the mesh. The protrusions may penetrate the mesh.

In one example the device comprises a release means for releasing the scaffold from engagement with a pre-deployed luminal prosthesis. The release means may comprise means for reducing the diameter of at least a portion of the scaffold. The release means may comprise a drawstring extending around the scaffold.

There may be a first drawstring extends around a proximal end of the support structure. There may be a second drawstring extends around a distal end of the support structure.

In one example the valve is mounted to the support structure. The valve may be sutured to the support structure. The valve may be bonded to the support structure. The valve may be adhesively bonded to the support structure.

In one example a proximal end of the sleeve is mounted to the support structure. The sleeve may be sutured to the support structure. The sleeve may be bonded to the support structure. The sleeve may be adhesively bonded to the support structure.

In one example the support structure comprises a scaffold which is of substantially uniform diameter.

The luminal prosthesis may comprise a distal bulbous region. The luminal prosthesis may comprise a scaffold receiving region. The scaffold receiving region may be intermediate the proximal and distal ends of the luminal prosthesis.

In one example the sleeve is of substantially uniform diameter along the length thereof.

In another example the sleeve has a first diameter at a proximal end and a second diameter at the distal end which is larger than the first diameter. The sleeve may be tapered.

In one example the retaining ring comprises a biasing means. The biasing means may comprise a flexible material which is biased into an expanded configuration.

In one example the retaining ring is oversized with respect to the sleeve.

The device may comprise release means for releasing the retaining ring from engagement. The release means

may comprise a drawstring.

In one example the sleeve has a retracted delivery configuration and an expanded deployed configuration. The sleeve may be folded in the retracted delivery configuration.

In one example the valve has a normally closed configuration and an open configuration in which the valve is opened for stomach emptying.

In one example the valve is adapted to open automatically for stomach emptying and to return automatically to the closed configuration.

The valve may be of a viscoelastic polymeric foam which may be biomimetic.

In one example the valve comprises an outer support region, at least three valve leaflets, and a main body region extending between the support region and the valve leaflets. The valve may have a region of co-aption of the valve leaflets in the closed configuration. The region of co-aption may extend for an axial length of at least 1mm.

In one example the device is adapted for placement in the pyloric sphincter or valve.

In another example the device is adapted for placement distal of the pyloric sphincter.

In one example the support is adapted for mounting to a pre-deployed sleeve which extends into the duodenum.

Also described is a delivery system for a gastrointestinal implant device, the implant device comprising an artificial valve, a duodenal sleeve and a support structure for the valve and the sleeve, the device having a retracted delivery configuration and an expanded deployed configuration, the delivery system comprising a delivery catheter having a distal pod for the implant device in the retracted configuration; and a sleeve deployment system.

The sleeve deployment system may comprise:

a distal cap;
a fluid delivery lumen for extending through the sleeve;
a distal seal between the distal cap and the lumen; and
a proximal seal,

whereby delivery of fluid through the lumen and into the sleeve causes the sleeve to expand from an axially retracted delivery configuration to an axially expanded deployed configuration.

The proximal seal may be sealingly engagable with the pod for deployment of the sleeve.

The proximal seal may be sealingly engagable with the valve for deployment of the sleeve.

In one example the pod is detachable from the delivery catheter.

The proximal seal may comprise an inflatable balloon.

The distal seal may comprise an inflatable balloon. The delivery system may include a flexible tube for inflating the distal balloon.

The delivery system in one example comprises a deployer for deploying the support structure and the valve to which the support structure is mounted. In one case the deployer comprises an abutment. The abutment may be provided by a balloon. The deployer balloon may comprise the proximal balloon.

In one example the distal cap or olive is releasably mounted to the fluid delivery lumen.

Also described is a gastrointestinal implant comprising a sleeve for extending into the duodenum, the sleeve having a pocket containing a radiopaque marker. The pocket may extend at least partially along the length of the sleeve.

In one example the sleeve has a plurality of pockets for reception of a radiopaque marker.

The radiopaque marker may comprise a fluid or gel. The fluid may comprise a silicon resin filled with a radiopaque material such as barium sulphate.

Also described is a method for treating obesity and/or diabetes comprising the steps of:-

providing a luminal prosthesis;
providing a valve mounted to a support scaffold, the valve having a retracted delivery configuration and an expanded deployed configuration;
providing a liner sleeve for lining the duodenum;
delivering the luminal prosthesis to a location at or distal of the pylorus;
deploying the luminal prosthesis at the location in the pylorus;
delivering the valve and support scaffold to the location; and
deploying the sleeve so that the sleeve extends from the valve and into the duodenum.

In one example the method comprises deploying the valve and support structure so that the support structure engages with the predeployed luminal prosthesis.

In one example the luminal prosthesis is deployed in the pyloric sphincter.

In another example the luminal prosthesis is deployed distal of the pyloric sphincter.

The method may comprise releasing the valve support structure from engagement with the luminal prosthesis;

and withdrawing the valve support structure, the valve, and the sleeve from the location. The method may comprise repeating the appropriate steps to deploy a valve, a support structure for the valve, and a sleeve at the desired location.

Also described is a method for treating obesity and/or diabetes comprising the steps of:-

providing a valve mounted to a support structure;
delivering the valve mounted to the support structure to a pre-deployed sleeve which extends into the duodenum; and
deploying the valve so that the valve is mounted to the sleeve.

The step of deploying the valve may comprise engaging the valve support with the pre-deployed luminal prosthesis.

In one example the valve support is an expandable support and the method comprises loading the support onto a delivery catheter in a retracted form and the valve support is expandable on deployment. The support may be self expandable. The support may be expanded by an expanding means such as a balloon.

In one example the method comprises the step of releasing the valve support from engagement with the luminal prosthesis. The method may comprise repositioning the valve support within the sleeve. The valve may be removed from the sleeve.

Also described is a gastrointestinal implant device comprising a pyloric valve for placement at the pylorus to control flow from the stomach into the duodenum,
the valve being of a viscoelastic foam and comprising at least three valve leaflets,
the valve having a normally closed configuration and an open configuration,
the valve leaflets being movable from the closed configuration to the open configuration for flow from the stomach.

In one example the valve is adapted to open automatically for stomach emptying and to return automatically to the closed configuration. The valve may comprise an outer support region and a main body region extending between the support region and the valve leaflets. The valve may have a region of co-aption of the valve leaflets in the closed configuration.

In one example the device comprises an anchor for anchoring the valve at the pylorus.

In one example the anchor comprises a support structure for the valve. The anchor may comprise a support scaffold for the valve and a luminal prosthesis to which the scaffold is mountable.

In one example the device comprises a sleeve for extending into the duodenum. The sleeve may be mounted to the valve or to an anchor for the valve. The device may be adapted for placement in the pyloric sphincter or may be adapted for placement distal of the pyloric sphincter.

Also described is a gastrointestinal implant device comprising a valve for placement at the pylorus to control the rate of stomach emptying.

In one example the valve has a normally closed configuration and an open configuration in which the valve is opened for stomach emptying.

There may be a support for the valve. The support may be adapted for mounting to a predeployed sleeve which extends into the duodenum.

In one example the implant device is adapted for placement in the pyloric valve.

In a further example the implant device is adapted for placement distal of the pyloric valve.

The valve support may comprise a support structure. The support structure may taper outwardly. The support structure may taper inwardly.

In another example the support structure is of generally uniform diameter along the length hereof.

The support structure may comprise a scaffold.

The support structure may comprise a stent-like structure.

In one example the device comprises mounting means for mounting the valve support to a pre-deployed luminal prosthesis.

The mounting means may be releasably engagable with a pre-deployed host support.

The device may comprise release means for releasing the valve from engagement with a pre-deployed host support. The release means may comprise means for reducing the diameter of at least portion of the valve support structure. The release means may comprise a drawstring extending around the valve support structure. There may be a first drawstring which extends around a proximal end of the support structure. There may be a second drawstring which extends around a distal end of the support structure.

In one example the valve is mounted to the support structure. The valve may be sutured to the support structure.

The valve may be bonded to the support structure. The valve may be adhesively bonded to the support structure.

In one example the valve is adapted to open automatically in the one direction.

Also described is a method for treating obesity and/or diabetes comprising the steps of:-

providing a valve mounted to a support structure;

delivering the valve mounted to the support structure to a pre-deployed sleeve which extends into the duodenum; and

deploying the valve so that the valve is mounted to the sleeve.

The step of deploying the valve may comprise engaging the valve support with the pre-deployed luminal prosthesis.

In one example the valve support an expandable support and the method comprises loading the support onto a delivery catheter in a retracted form and the valve support is expandable on deployment.

The support may be self expandable. Alternatively the support is expanded by an expanding means. The expanding means may comprise a balloon.

In one example the method comprises the step of releasing the valve support from engagement with the luminal prosthesis. The method may comprise repositioning the valve support within the sleeve.

In one example the method comprises removing the valve from the sleeve.

In one example the valve comprises a polymeric valve body having an outer support rim, at least three valve leaflets, and a main body region extending between the support rim and the valve leaflets.

Also described is a valve comprising at least four valve leaflets, the valve having a normally closed configuration in which the leaflets are engaged and an open configuration in which the leaflets are open. There may be at least five valve leaflets. There may be six valve leaflets.

The valve may comprise a valve body of polymeric material. The valve may comprise an outer support region. The valve may also have a main body region extending between the support region and the valve leaflets.

In one example the main body region is generally concave between the outer support rim and a region of co-aption of the valve leaflets.

In one example the valve leaflets have a region of co-aption and the valve body is reinforced at the region of co-aption. The valve body may be thickened at the region of co-aption.

The region of co-aption may extend for an axial length of at least 1 mm. The region of co-aption may extend for a depth of from 1 mm to 5mm.

In one example the support rim of the valve body is reinforced. The support rim of the valve may be thickened.

In one example the valve comprises three valve leaflets.

In another example the valve comprises six valve leaflets.

The valve may be mounted to the support structure.

In one example the valve rim is sutured to the support structure. Alternatively or additionally the valve rim is bonded to the support structure.

In one example the support structure comprises a luminal prosthesis.

In one example the luminal prosthesis extends proximally of the valve.

In another example the luminal prosthesis extends distally of the valve.

In one example the luminal prosthesis extends proximally and distally of the valve.

The luminal prosthesis may have a coating and/or a sleeve thereon. The coating or sleeve may be on the outside of the luminal prosthesis. Alternatively the coating or sleeve is on the inside of the luminal prosthesis.

In one example the polymeric material is stable to gastric fluid for at least 3 months, for at least 4 months, for at least 5 months, for at least 6 months, for at least 7 months, for at least 8 months, for at least 9 months, for at least 10 months, for at least 11 months, or for at least one year.

In one example the polymeric material takes up less than about 5%, less than about 10%, less than about 15%, less than about 20%, less than about 25%, or less than about 30% by weight of water at equilibrium.

In one example the polymeric material of the valve body has a % elongation of from 50% to 3000% or 200% to 1200%.

In one example the polymeric material of the valve body has a tensile strength of from 0.01 to 5 MPa or about 0.1 to 1.0 MPa, or about 0.25 to 0.5 MPa.

In one example the polymeric material has a Young's Modulus of about 0.01 to 0.6 MPa, or about 0.1 to about 0.5 MPa.

In one example the polymeric material of the valve body has a density of from 0.1 $g/cm^3$ to 1.5 $g/cm^3$, or 0.3 to 1.2$g/cm^3$, or 0.8 to 0.9$g/cm^3$, or 0.5 to 0.6$g/cm^3$.

In one example the distance between the proximal end of the support region of the valve body and the distal end of the valve leaflets is less than 50mm, or less than 40mm, or less than 30mm, or less than 25mm, or less than 20mm, or less than 15mm.

In one example the polymeric material of the valve body is of an elastic material.

In one example the polymeric material of the valve body is of a viscoelastic material.

In one example the polymeric material of the valve body comprises a foam. The polymeric material of the valve body may comprise an open cell foam.

In one example the polymeric material of the valve body comprises a polyurethane foam.

In one example the valve is adapted to be mounted to a pre-deployed support structure, for example an esophageal luminal prosthesis such as a stent.

Also described is a valve having:-

a normally closed configuration in which the valve is closed;
an open configuration in which the valve is opened for flow through the valve; and a support for the valve, the support being adapted for mounting to a pre-deployed luminal prosthesis intermediate a proximal end and a distal end of the predeployed luminal prosthesis.

In one example the luminal prosthesis has a coating and/or sleeve thereon. The coating or sleeve may be on the outside of the luminal prosthesis. Alternatively or additionally the coating or sleeve is on the inside of the luminal prosthesis.

The mounting means may be provided by the support structure. In one case the mounting means comprises protrusions extending from the support structure. The protrusions may be adapted to engage with a pre-deployed host esophageal luminal prosthesis.

In one example the protrusion comprises a loop.

In one example the apicial tip of the protrusion is rounded.

There may be release means for releasing the valve from engagement with a pre-deployed host luminal prosthesis. The release means may comprise means for reducing the diameter of at least portion of the valve support structure.

In one example the release means comprises a drawstring extending around the valve support structure. A first drawstring may extend around a proximal end of the support structure. A second drawstring may extend around a distal end of the support structure.

In one case the valve is mounted to the support structure. The valve may be sutured to the support structure. The valve may be bonded to the support structure. The valve may be adhesively bonded to the support structure.

In another example the mounting means comprises a surgical adhesive.

Also described is a method for providing a valve in a body passageway comprising the steps of:-

providing a valve mounted to a support structure;
delivering the valve mounted to the support structure to a pre-deployed luminal prosthesis in the body passageway; and
deploying the valve so that the valve is mounted to the luminal prosthesis.

In one example the step of deploying the valve comprises engaging the valve support with the pre-deployed luminal prosthesis.

The valve support may be mechanically engaged with the pre-deployed luminal prosthesis.

In one example the valve support comprises a protrusion and the method comprises aligning the protrusion with an aperture in the endoluminal prosthesis and engaging the protrusion in the aperture.

In one example the valve support is an expandable support and the method comprises loading the support onto a delivery catheter in a retracted form and the valve support is extendable on deployment.

The support may be self expandable or the support is expanded by an expanding means such as a balloon.

In one example the method comprises the step of releasing the valve support from engagement with the luminal prosthesis.

The method may involve repositioning the valve support within the prosthesis. The method may comprise removing the valve from the prosthesis.

In one example the luminal prosthesis extends proximally of the valve. The prosthesis may comprise a self expanding plastics mesh. The prosthesis may apply a radial force of less than 1.9kPa.

In one example there are anchors for mounting the prosthesis in situ. The anchors may be adapted to extend through the mesh of the prosthesis.

In one case the length of the valve from the proximal end of the support region to the distal end of the valve leaflets is less than 50 mm, less than 40 mm, less than 30 mm. The length of the valve may be approximately the same as the outer diameter of the support region of the valve. The length of the valve may be approximately 23 mm.

## Brief Description of the Drawings

The invention will be more clearly understood from the following description thereof given by way of example only, in which:-

Fig. 1 is an isometric view (from above) of a valve;

Fig. 2 is an isometric view (from below) of the valve;

Fig. 3 is a top plan view of the valve;

Fig. 4 is an underneath plan view of the valve;

Figs. 5 and 6 are elevational views of the valve;

Figs. 7 and 8 are isometric, partially cut-away sectional, views of the valve;

Figs. 9 and 10 are cross sectional views of the valve;

Fig. 11 is a cross sectional view of the valve in a normally closed configuration;

Fig. 12 is a cross sectional view of the valve in an open configuration in response to a force;

Fig. 13 is a cross sectional view of the valve returned to the closed configuration after opening to flow;

Fig. 14 is an isometric view (from above) of the valve in a normally closed configuration;

Fig. 15 is an isometric view of the valve in a partially open configuration in response to a force;

Fig. 16 is an isometric view of the valve in a fully open configuration in response to a force;

Fig. 17 is an isometric view of a prosthesis;

Fig. 18 is an elevational view of the valve of Figs. 1 to 16 being mounted to and in position on the prosthesis of Fig. 17;

Fig. 19 is another view of the valve mounted in a prosthesis;

Figs. 20 and 21 are isometric views of a sleeved or coated prosthesis;

Fig. 22 is an isometric view of the prosthesis of Figs. 20 and 21 with a valve of Figs. 1 to 16 in position;

Fig. 23 is an elevational view of part of the prosthesis of Fig. 22 in position;

Fig. 24 is an isometric view of another valve;

Fig. 25 is an elevational view of the valve of Fig. 24;

Fig. 26 is an isometric view of another valve with a distally outward tapering support structure;

Fig. 27 is an elevational view of the valve of Fig. 26.

Fig. 28 is an isometric view of another valve with a distally inward tapering support structure;

Fig. 29 is an elevational view of a luminal prosthesis with a valve and associated support structure in place;

Fig. 30 is an enlarged view of the luminal prosthesis and valve support structure of Fig. 29;

Figs. 31 and 32 are enlarged views of one mounting detail of a valve support structure to a luminal prosthesis;

Figs. 33 to 37 are views of a valve being deployed from a delivery catheter;

Figs. 38 to 40 are views of a luminal prosthesis in situ with a valve being deployed in the lumen of the luminal prosthesis.

Fig. 41 is an elevational view of a valve;

Fig. 42 is an enlarged view of a detail of the support structure of the valve of Fig. 41;

Figs. 43 and 44 are isometric views of the valve of Fig. 41 and 42 being deployed from a delivery catheter;

Fig. 45 is an elevational view of a prosthesis with the valve of Figs. 43 and 44 in situ;

Fig. 46 is an enlarged view of a detail of the engagement of the valve support structure of Figs. 41 to 45 engaged in the mesh of the prosthesis;

Fig. 47 is an enlarged view of part of the luminal prosthesis and valve support structure of Fig. 46;

Fig. 48 is an elevational view of a luminal prosthesis;

Fig. 49 is an elevational of an esophageal valve;

Figs. 50 to 55 are elevational views of steps involved in deploying the valve of Fig. 49 into a pre-deployed luminal prosthesis of Fig. 48;

Fig. 56 is an elevational view of the valve of Fig. 49 deployed in the luminal prosthesis of Fig. 55;

Fig. 57 is an elevational view similar to Fig. 56 with the valve being removed from the deployed prosthesis;

Fig. 58 is an isometric view of a valve;

Fig. 59 is an elevational view of the valve of Fig. 56;

Fig. 60 is a plan view of the valve of Figs. 58 and 59 with the valve in a closed configuration;

Fig. 61 is a plan view similar to Fig. 60 with the valve in an open configuration;

Fig. 62 and 63 are side views of the device of Fig. 60 with the valve in a closed configuration;

Figs. 64 and 65 are side views of the device of Fig. 60 with the valve in the open configuration;

Fig. 66 is an illustration of a gastrointestinal implant device;

Fig. 67 is an enlarged view of detail A of Fig. 66;

Figs. 68 and 69 are illustrations of another gastrointestinal implant device located in the pyloric sphincter;

Figs. 70 and 71 are illustrations similar to Figs. 66 and 67 with the device located distal of the pyloric sphincter;

Fig. 72 is an isometric view of a luminal prosthesis of an implant device of the invention;

Fig. 73 is an elevational view of a valve, sleeve and scaffold part of an implant device;

Fig. 74 is an elevational, partially cross sectional view of an implant device with a prosthesis located in a lumen such as the pylorus and a valve, sleeve and scaffold for mounting to the prosthesis;

Fig. 75 is an elevational view of the device of Fig. 72 assembled;

Fig. 76 is an elevational view of the device of Fig. 75 with the sleeve extended;

Fig. 77 is an elevational, partially cross sectional view of the device, in situ;

Fig. 78 is a view similar to Fig. 77 of an implant device with a sleeve in one folded delivery configuration;

Fig. 79 is a view similar to Fig. 78 with the sleeve in another folded delivery configuration;

Fig. 80 is a view similar to Fig. 79 with the sleeve in a further folded delivery configuration;

Fig. 81 is an elevational, partially cross sectional view of an implant device including a retaining ring for a sleeve;

Fig. 82 is a view similar to Fig.81 of another sleeve;

Fig. 83 is a view similar to Fig. 81 with a sleeve having a plurality of retaining rings;

Fig. 84 is cross sectional view illustrating a first stage in the delivery of an implant device to the pylorus;

Fig. 85 is a cross sectional view of the implant device in position with the sleeve in a retracted configuration;

Fig. 86 is a cross sectional view of the implant device in situ, with the sleeve partially extended;

Fig. 87 is a cross sectional view similar to Fig. 86 with the sleeve further extended;

Fig. 88 is an enlarged cross sectional view of a distal end of the delivery system;

Fig. 89 is a cross sectional view of the implant device in situ with the sleeve extended and the delivery system being removed;

Fig. 90 is an elevational view of a delivery catheter for the implant device;

Fig. 91 is a cross sectional view of the delivery catheter of Fig. 90 with a capsule containing the implant device;

Figs. 92 to 94 are views showing the delivery system at various stages;

Fig. 95 is a cross sectional view of a proximal end of the delivery system capsule;

Fig. 96 is an elevational view of part of the delivery system;

Fig. 97 is an exploded view of part of delivery system of Fig. 96;

Fig. 98 is a graph of pressure profile over time with various fixed orifice restrictors;

Fig. 99 is a graph of pressure profile over time with a fixed orifice restriction and an implant device comprising a valve;

Fig. 100 is a graph of pressure profile over time with a fixed orifice restriction and implant devices comprising valves;

Fig. 101 is an isometric view of part of a sleeve;

Fig. 102 is a cross sectional view of the sleeve of Fig. 93;

Fig. 103 is an isometric view of part of another sleeve;

Fig. 104 is a graph of valve performance over time in a simulated gastric fluid, where the performance ciriterion is the opening pressure of the valve;

Fig. 105 is a graph of mass uptake over time for biomaterials;

Fig. 106 is a comparison of the chemical stability of a BAS triblock urethane polymer;

Fig. 107 is an illustration of prior art polymers with urea and urethane linkages interspersed between homopolymer soft segments;

Fig. 108 is an illustration of a polyurethane/urea foam with urea and urethane linkages interspersed between triblock

copolymer soft segments;

Fig. 109 is an illustration of a siloxane and polypropylene oxide based triblock copolymer in different forms;

Fig. 110 is a graph of comparative mechanical properties of homo (VF130309) and triblock copolymer (VF230209A) soft segments;

Fig. 111 is a graph of comparative mechanical properties of home (VF190309) and triblock copolymer (VF090309) soft segments;

Fig. 112 is a graph illustrating the mechanical performance of triblock copolymer soft segments versus homopolymer soft segment during accelerated aging in simulated gastric fluid;

Fig. 113 depicts a gastric yield pressure test apparatus as utilized in Example 10; and

Fig. 114 and Fig. 115 depict results of accelerated stability of a valve prepared from a viscoelastic foam.

## Detailed Description

Referring to the drawings and initially to Figs. 1 to 16 thereof there is illustrated a valve 1 which can open automatically in one direction.

The valve 1 comprises a polymeric valve body having a proximal outer support region with a rim 2, at least three valve leaflets 3, 4, 5, and a main body region 6 extending between the support rim 2 and the valve leaflets 3, 4, 5. The valve leaflets 3, 4, 5 extend inwardly and distally and terminate at distal end faces 7, 8, 9 respectively. The leaflets each 3, 4, 5 have legs a, b which extend at an included angle of 120° to each other. The adjacent pairs of legs 3a; 4a; 4b; 5b; 5a; 3b; co - apt to close the gap between the valve leaflets when the valve is in the normally closed configuration.

The valve 1 has two configurations. The first configuration is a normally closed configuration in which the valve leaflets 3, 4, 5 co-apt to close the valve. The second configuration is an open configuration in which the valve leaflets 3, 4, 5 are opened such that the leaflet leg pairs 3a; 4a; 4b; 5b; 5a; 3b are opened and spaced-apart in response to a force F1 to allow flow through the valve.

The various configurations of the valve 1 are illustrated in Figs. 11 to 16. In the first or normally closed configuration (Figs 11, 14) the valve leaflets 3, 4, 5 co-apt. When a force F1 is applied to the valve leaflets 3, 4, 5 the leaflet legs pairs 3a; 4a; 4b; 5b; and 5a; 3b open to allow antegrade flow to pass (Figs. 12, 16). Fig. 15 illustrates a partially open configuration in response to flow. When the force F1 is removed the leaflets 3, 4, 5 return to the closed position under the inherent biasing of the polymeric material of the valve body (Fig. 13).

The valve leaflets 3, 4, 5 are reinforced in the region of co - aption. In this case, this is achieved by a local thickening of the polymeric material in this region. Similarly the support rim 2 is reinforced by a local thickening of the polymeric material.

The region of co-aption of the valve leaflets 3, 4, 5 has an axial extent which is typically from 1 to 5mm. This ensures positive co-aption of the leaflets across a significant interfacial area when the valve is in the normally closed configuration. The thickness of the leaflets at the region of co-aption is typically between 0.1mm and 10mm.

The valve body has a generally concave outer face and a generally convex inner face.

The valve 1 of the invention returns to its original working position after being fully opened. This is accomplished without damaging the working valve.

When the valve is opened by stomach emptying the leaflets open.

One important characteristic influencing the functioning of the valve is the leaflet legs that impinge on one another. By varying the geometry and length of the leaflets 3, 4, 5 the valve 1 can be made to open at different pressures. Opening is also dependant on the elasticity and density of the material the device is made from. Additionally, the overall diameter and the diameter to which the leaflets open influence the opening force.

The valve may be of any suitable biocompatible polymeric material. It may be of a biocompatible polymeric material having properties which allow the valve to function as described.

The materials used for the production of this valve have a % elongation between 50% and 3000%. The material also has a tensile strength of between 0.01 and 5 MPa. Addionally the material could have an antimicrobial action to prevent colonisation when in-vivo. Additionally the material can be elastic or viscoelastic and can optionally be an open cell foam. The density of the material should be between 0.1 g/cm3 to 1.5 g/cm3.

The valve may be mounted to any suitable luminal prosthesis, especially a prosthesis or stent. The rim 2 of the valve provides a mounting ring for mounting within the stent 20, for example, the valve 1 may be mounted to the stent by suturing the rim 2 to the stent mesh using sutures 21 as illustrated in Figs. 18 and 19.

The stent may be of any suitable type. An uncoated or unsleeved stent 20 is illustrated in Figs. 17 to 19. Alternatively, if it is desired to prevent tissue ingrowth a stent 30 having a sleeve 31 may be used (Figs. 20 to 23). In this case the sleeve 31 is external of the stent. In other cases there may alternatively or additionally be an internal sleeve. Further, the stent may have a coating.

A valve such as described above may also be placed into a pre-deployed luminal prosthesis.

In one case a valve 100 may have a co-axial support structure or scaffold 102 is shown in Figs. 24 and 25. The scaffold 102 is designed to engage with any suitable esophageal stent 140 as illustrated in Fig. 29. The mechanism of engagement can be by protrusions which may for example be proximal and/or distal apices 103 of the scaffold 102 which engage into the mesh of the existing pre-deployed stent 140. Alternatively or additionally, the scaffold 102 may have features 150 designed to hook onto the inside of the struts of an esophageal stent as illustrated in Figs. 31 and 32.

Referring to Figs. 26 and 27 there is illustrated a valve 110 in which the support structure or scaffold 102 tapers distally outwardly so that distal apices 111 of the scaffold engage with the mesh of the existing pre-deployed host stent 140.

Referring to Fig. 28 there is illustrated another valve 120 in which the support structure or scaffold 102 tapers distally inward so that proximal apices 121 of the scaffold 102 engage with the mesh of an existing pre-deployed stent 140.

The radial force of the scaffold 102 may exert enough friction to hold the valve in place without the necessity for protrusion. In another embodiment a surgical adhesive may be used to secure the retrofitted valve into place.

Referring to Figs. 33 to 37 a valve 100 is loaded into a delivery system 130 for deployment. The outer diameter of the delivery system 130 is smaller than the inner diameter of a pre-deployed esophageal stent 140. The delivery system 130 in this case comprises a delivery catheter having a distal pod 131 in which a valve is housed in a contracted configuration. The catheter has a tapered distal tip 132 to avoid snagging on a pre-deployed stent 140. The pod 131 is axially movable relative to the tip 132 to release the valve from the pod 131.

The delivery system 130 is used to deliver the valve to a pre-deployed stent 140 as illustrated in Fig. 38. The stent 140 has a mesh and the scaffold of the valve is adapted to engage with the mesh of the pre-deployed stent 140 on release of the valve from the delivery catheter as illustrated particularly in Figs. 39 and 40.

Referring to Figs. 29 to 32 there is illustrated an idealised stent 140 with a valve support scaffold 102 in situ. Details of a valve are omitted from these drawings for clarity. In this case the scaffold 102 is located at the upper proximal end of the stent. In this case the scaffold 102 has hook-like members 150 for engagement with the mesh of the stent 140 as illustrated in Figs 31 and 32. The interengagement between the stent 140 and the scaffold 102 ensures that the scaffold 102 and hence the valve which is fixed to it is retained in position and provides an anti-proximal migration mechanism.

In the cases illustrated the valve supporting scaffold 102 is of a self expanding material such as a shape memory material, for example Nitinol. The valve and scaffold are loaded into the delivery catheter pod 131 in a compressed / reduced diameter configuration. When the constraint of the pod 131 is removed at the deployment site, the scaffold and valve self expand to the normal configuration in which the scaffold is engaged with the pre-deployed host stent 140. In some arrangements the scaffold may be of an expensile material which is expanded by an expander such as a balloon or the like.

Referring to Figs. 41 to 44 there is illustrated another valve device 151 which is similar to that described above and like parts are assigned the same reference numerals. In this case the valve 1 is housed within a support structure or scaffold 102 and is placed into the lumen of a stent 140 as illustrated in Figs. 45 to 47. The support structure may comprise a relatively short length (typically 40mm) of a mesh made from a shape memory material such as Nitinol. The mesh may be formed by laser cutting and / or may be of woven construction. Deployment into the lumen of the host stent 140 is via self expansion from a radially collapsed state within a delivery catheter 130 as shown in Figs. 43 and 44. The device 151 is held in place within the stent 140 by means of specific interaction mechanisms that increase the axial friction of the support structure 102. Figs. 45 to 47 illustrate the interaction with the host stent 140. In this embodiment the support structure 102 has a series of loops or protrusions 155 extending perpendicularly from its surface. These protrusions 155 engage with the structure of any host stent 140 by interlocking with the existing mesh as shown in Figs. 52 and 53. The apical tip of each protrusion 155 is in this case rounded or designed so as to be non-traumatic to any tissue that may come into contact with the protrusion 155. The intrinsic radial force of the support structure 102 as well as the flexural strength of the protrusions 155 interact to effect the retention performance of the support structure 102. Thus the stiffness or flexural strength of the protrusion 155 and the radial force of the support structure 102 may be modified to change the interlocking capability and retention performance of the device.

The valve device 151 is also readily radially collapsible by distal and proximal drawstrings 170, 171. The distal drawstring 170 passes through eyelets 172 mounted to the support structure 102 at the distal end of the valve device 151. The distal drawstring 170 has an accessible pull string 173 which, on pulling, pulls the drawstring 171 inwardly and thus reduces the diameter of the distal end of the support structure 102. Similarly the proximal drawstring 171 passes through eyelets 175 mounted the support structure 102 at the proximal end of valve device 151. The proximal drawstring 171 has an accessible pull string 177 which, on pulling, pulls the drawstring 171 inwardly and thus reduces the diameter of the proximal end of the support structure 102. The pull strings 173, 177 can be readily gripped using a suitable

instrument such as a grasper to draw the proximal and distal ends of the support structure 102 inwardly for ease of removal of the valve device 151.

Referring to Figs. 48 to 57 there is illustrated another valve device 200 which is similar to that described above and like parts are assigned the same reference numerals. In this case the valve 1 is housed within a support structure or scaffold 102 and is placed into the lumen of a stent 140 as illustrated in Figs. 53 to 56. The support structure 102 may comprise a relatively short length (typically 40mm) of a mesh made from a shape memory material such as Nitinol. The mesh may be formed by laser cutting and / or may be of woven construction. Deployment into the lumen of the host stent 140 is via self expansion from a radially collapsed state within a delivery catheter 130 as shown in Figs. 50 to 55. The device 200 is held in place within the stent 140 by means of specific interaction mechanisms that increase the axial friction of the support structure 102. Fig. 56 illustrates the interaction with the host stent 140. In this embodiment the support structure 102 has a series of loops or protrusions 155 extending perpendicularly from its surface. These protrusions 155 engage with the structure of any host stent 140 by interlocking with the existing mesh as shown in Fig. 56. The apical tip of each protrusion 155 is in this case rounded or designed so as to be non-traumatic to any tissue that may come into contact with the protrusion 155. The intrinsic radial force of the support structure 102 as well as the flexural strength of the protrusions 155 interact to effect the retention performance of the support structure 102. Thus the stiffness or flexural strength of the protrusion 155 and the radial force of the support structure 102 may be modified to change the interlocking capability and retention performance of the device.

The valve device 200 is also readily radially collapsible by distal and proximal drawstrings 170, 171. The distal drawstring 170 passes through eyelets 172 mounted to the support structure 102 at the distal end of the valve device 200. The distal drawstring 170 has an accessible pull string 173 which, on pulling, pulls the drawstring 171 inwardly and thus reduces the diameter of the distal end of the support structure 102. Similarly the proximal drawstring 171 passes through eyelets 175 mounted the support structure 102 at the proximal end of valve device 200. The proximal drawstring 171 has an accessible pull string 177 which, on pulling, pulls the drawstring 171 inwardly and thus reduces the diameter of the proximal end of the support structure 102. The pull strings 173, 177 can be readily gripped using a suitable instrument such as a grasper to draw the proximal and distal ends of the support structure 102 inwardly for ease of removal of the valve device 200.

It will be noted that in the case of this device 200 the diameter of the support scaffold is relatively uniform and the proximal and distal ends 201, 202 of the device 200 are not tapered. We have found that the interengagement of the rounded protrusions 155 in interstices defined in the mesh structure of the stent 140 is sufficient to retain the device 200 in position in the stent 140. Typically, the diameter of the expanded support structure 102 will be slightly larger, for example 1 to 5% larger than that of the host stent 140 at the desired deployment location to assist in maintaining the scaffold 102 in situ.

In some cases, as illustrated in Fig. 57 the devices such as the device 200 may be a radially collapsed state if it is described to re-position the valve device 200 with the stent 140 or to withdraw the device 200, for example for replacement and/or for replacement of the host stent 140.

Thus, the collapsibility of the valves enables its optional removal by disengagement of the protrusions 155 from the host stent 140, thus eliminating any axial friction associated with the host stent 140.

The valve of Figs. 1 to 57 may be relatively short and is typically less than 30 mm, less than 25 mm, less than 20 mm, less than 15 mm and is typically about 10.6mm long with an outer rim diameter of 18mm or about 11mm long for an outer rim diameter of 20mm.

The valve may have any desired number of leaflets, for example the valve 300 illustrated in Figs. 58 to 65 has six valve leaflets 333. These leaflets 333 are oriented perpendicular to direction of food flow to additionally allow greater distensibility of the valve aperture.

Referring to Figs. 58 to 65 there is illustrated another valve device. The device 300 comprises a valve 301 which can open automatically in one direction.

The valve 300 comprises a polymeric valve body having a proximal outer support region with a rim 302, six valve leaflets 303, and a main body region 306 extending between the support rim 302 and the valve leaflets 303. The valve leaflets 303 extend inwardly and distally and terminate at distal end faces 303 respectively. The leaflets each 303 have legs which extend at an included angle of 60° to each other. The adjacent pairs of legs co - apt to close the gap between the valve leaflets 303 when the valve is in the normally closed configuration.

The valve 300 has two configurations. The first configuration is a normally closed configuration in which the valve leaflets 303 co-apt to close the valve. The second configuration is an open configuration in which the valve leaflets 303 are opened such that the leaflet leg pairs are opened and spaced-apart in response to a force F1 to allow flow through the valve 300.

The various configurations of the valve 1 are illustrated in Figs. 58 to 65. In the first or normally closed configuration the valve leaflets 303 co-apt. When a force F1 is applied to the valve leaflets 303 the leaflet legs pairs open to allow flow to pass. When the force F1 is removed the leaflets 303 return to the closed position under the inherent biasing of the polymeric material of the valve body.

The valve leaflets 303 are reinforced in the region of co - aption. In this case, this is achieved by a local thickening of the polymeric material in this region. Similarly the support rim 302 is reinforced by a local thickening of the polymeric material.

The region of co-aption of the valve leaflets 303 has an axial extent which is typically from 1 to 5mm. This ensures positive co-aption of the leaflets across a significant interfacial area when the valve is in the normally closed configuration. The thickness of the leaflets at the region of co-aption is typically between 0.1 mm and 10mm.

The valve body 306 has a generally concave outer face and a generally convex inner face.

The valve 300 returns to its original working position after being fully opened. This is accomplished without damaging the working valve.

An important characteristic influencing the functioning of the valve 300 is the leaflet legs that impinge on one another. By varying the geometry and length of the leaflets 303 the valve 300 can be made to open at different pressures. Opening is also dependant on the elasticity and density of the material the device is made from. Additionally, the overall diameter and the diameter to which the leaflets open influence the opening force.

The valve may be of any suitable biocompatible polymeric material. It may be of a biocompatible polymeric material having properties which allow the valve to function as described.

The materials used for the production of this valve have a % elongation between 50% and 3000%. The material also has a tensile strength of between 0.01 and 5 MPa. Addionally the material could have an antimicrobial action to prevent colonisation when in-vivo. Additionally the material can be elastic or viscoelastic and can optionally be an open cell foam. The density of the material should be between 0.1 g/cm3 to 1.5 g/cm3.

The valve 300 may be mounted to any suitable luminal prosthesis. The rim 302 of the valve provides a mounting ring for mounting within the prosthesis, for example, the valve 300 may be mounted to the stent by suturing the rim 2 to the stent mesh using sutures.

Many emerging obesity treatments involve the placement of a tube into the duodenum, which restricts the absorption of certain nutrients at this point in the body. The resulting calorific deficit then results in weight loss. Some of these devices can cause the pyloric valve to be opened for prolonged periods thus causing rapid stomach emptying. During episodes of rapid stomach emptying the feeling of fullness is shortened and thus the patient eats more.

We have found that by placing a valve device at or near the pylorus that can controllably restrict the rate of stomach emptying then a feeling of fullness or satiety can be gained.

Referring to Figs. 66 and 67 there is illustrated a valve device 500 that can be retrospectively placed into an existing obesity treatment device such as a sleeve 501 which extends from a stomach 502 into the duodenum 503. One such sleeve device is described in US2005/0125075A. The valve 500 functions to restrict the rate of stomach emptying. The positioning of the valve 500 within a pre-positioned sleeve 501 is illustrated in Figs. 66 and 67. The valve 500 may be of the type described above and may be attached to a scaffold 505 as described above.

Referring to Figs. 68 and 69 there is illustrated a valve 550 which in this case is placed in a pyloric sphincter 551 in order to control the rate of stomach emptying and thereby provide an enhanced feeling of satiety. This approach may be used, if example in association with gastic banding or other obesity treatment system. The valve 550 may be retained in situ by any suitable means such as anchors 552.

Alternatively, as illustrated in Figs. 70 and 71 the valve 550 may be located distal of the pyloric sphincter 551 to provide a further valve acting in series with the pyloric valve or sphincter.

Referring to Figs. 72 to 77 there is illustrated a gastrointestinal implant device 600 according to the invention which comprises a sleeve 601 for extending into the duodenum and an artificial valve 602 for placement at the pylorus 603 to control flow from the stomach 604 into the duodenum which is lined by duodenal sleeve 601. The device 601 also comprises a support structure for the valve. In this case the support structure comprises a scaffold 605 to which the valve 602 is mounted. The support structure also comprises a luminal prosthesis 606 to which the scaffold is mounted. In this instance, the scaffold 605 is releasably mountable to the luminal prosthesis 606. The sleeve 601 is mounted to the support structure and in this case to the valve and/or the scaffold 605.

In this case the support structure comprises a stent-like scaffold 605 and the luminal prosthesis 606. The prosthesis 606 is for deployment at the pylorus and the scaffold 605 to which the valve 602 is mounted is releasably mountable to the pre-deployed luminal prosthesis 606. The scaffold comprises engagement elements which are releasably engagable with the luminal prosthesis 606. The engagement elements may comprise protrusions 607 which are releasably engagable with the luminal prosthesis. The luminal prosthesis 606 in this case comprises a mesh which may have a coating thereon. The protrusions 609 may engage with and in some cases penetrate the mesh. In the case of a coating on the mesh the protrusions 607 may penetrate the coating.

In this embodiment at least a part of the implant device is removable for complete removal, repositioning, or replacement. There is a release means for releasing the scaffold 605 from engagement with the prosthesis 606. The release means in this case comprises means for reducing the diameter of at least portion of the scaffold. The release means may comprise a drawstring 611 extending around the scaffold 605. In this case there is a first drawstring 611 a extending around a proximal end of the support structure and a second drawstring 611b extending around a distal end

of the support structure. For removal, the drawstrings are tightened by pulling on the loops 612 using a suitable instrument such as a grasper.

Both the prosthesis 606 and the scaffold 605 may be of a shape memory material such as Nitinol and have a reduced diameter delivery configuration and an expanded deployed configuration.

The prosthesis 606 in this case comprises a proximal flare 620 for location, in the expanded configuration at the antrum of the pylorus. The flare 620 assists in anchoring the prosthesis in position. The prosthesis 606 in this case also has a distal bulbous region 621 which assists in anchoring the prosthesis in position. The prosthesis 606 has a scaffold receiving region 622 which in this case is intermediate the proximal and distal ends of the prosthesis 606.

The scaffold 605 has a proximal region 630 to accommodate the valve 602 and a distal region 631 to accommodate the sleeve 601 in a retracted delivery configuration. The valve 602 may be attached to the scaffold 605 by sutures 632 and/or may be bonded, for example by adhesive bonding to the scaffold 605.

The sleeve 601 in this case is also attached to the scaffold 605 and/or to the valve 602, for example by bonding and/or sutures.

The valve 602 has a normally closed configuration and an open configuration in which the valve is opened for stomach emptying. The valve 602 is adapted to open automatically for stomach emptying and to return automatically to the closed configuration. The valve may be of a viscoelastic foam material such as the foam materials described in detail in this specification. The valve 602 is in this case similar to the valves described earlier and comprises an outer support region 640, at least three valve leaflets 641, and a main body region 642 extending between the support region and the valve leaflets 641. The valve 602 has a region 643 of co-aption of the valve leaflets in the closed configuration to maintain the valve in the normally closed configuration. The region 643 of co-aption may extend for an axial length of at least 1 mm.

Fig. 72 shows the luminal prosthesis 606 in a relaxed, pre-loading configuration. Fig. 73 shows the scaffold 605, valve 602 and sleeve 601. The sleeve 601 is in a retracted configuration. Fig. 74 shows the prosthesis 606 deployed at the pylorus and the scaffold 605 / valve 602 / sleeve 601 being inserted into the prosthesis 606. Fig. 75 shows the scaffold 605 / valve 602 / sleeve 601 deployed in the prosthesis 606. Fig. 76 is a view similar to Fig. 75 with the sleeve 601 expanded into a deployed configuration extending through the duodenum. Fig. 77 is a cross sectional view showing the valve 602, support structure and sleeve 601 fully deployed.

It will be appreciated that the sleeve may be configured in different ways in a retracted delivery configuration. Some examples are shown in Figs. 78 to 80. In Fig. 78 the sleeve 601 is folded somewhat like an accordion. In Fig. 79 the sleeve 601 may be folded longitudinally and may subsequently be spirally wound. In Fig. 80 the sleeve 601 has longitudinal pleats or folds and is also folded over transversely.

The sleeve 601 may be of constant diameter along the length thereof or may be tapered (Figs. 81 / 83) or may have a narrowed proximal section and a constant diameter distal section (Fig. 82).

The sleeve 601 may have a retaining means to assist in retaining the sleeve at a desired location. For example, as illustrated in Fig. 81 the sleeve 601 may have a retaining ring 650 at or near the distal end of the sleeve. There may be a plurality of such retaining rings 650 which may be spaced-apart along the sleeve 601 as illustrated in Fig. 83. The rings 650 may be of different size and/or shape to suit the target anatomy. The retaining rings 650 may have a biasing means to bias them into an enlarged configuration. For example, the retaining ring 650 may be oversized with respect to the diameter of the sleeve 601. There may be a release means such as a drawstring or the like to release the retaining ring 650 from the expanded deployed configuration.

Referring to Figs. 84 to 89 an implant device and an associated delivery system are illustrated. The delivery system comprises a delivery catheter 660 with a distal capsule 669 which contains the scaffold 605, valve 602 and sleeve 601 in the retracted configuration. The delivery system includes a proximal expandable element provided by an inflatable proximal balloon 662 and a distal expandable element provided by a distal balloon 663. The proximal balloon 662 provides a temporary seal with the proximal end 664 of the sleeve 601 at the proximal side of the valve 602. The distal balloon 665 provides a temporary distal seal between a distal olive 666 and a distal end 667 of the sleeve 601. An inflation fluid is introduced into the sleeve 601 between the proximal and distal balloons 662, 665, the fluid causes the sleeve 601 to expand axially to the expanded deployed configuration. When the sleeve 601 is in the extended deployed configuration the distal balloon 665 is deflated, allowing the olive 666 to detach and travel distally. The rest of the delivery system can then be withdrawn proximally, leaving the implant device in situ. Fig. 84 illustrates the luminal prosthesis or stent 605 with a 30mm wide proximal flare placed across the pylorus with the proximal flare resting against the pyloric antrum. An endoscope with a delivery system is advanced into the stomach. The delivery device is controlled through the shaft of the endoscope and comprises a capsule that is positioned proximal to the endoscope. The capsule is advanced to the pre-placed stent. Fig. 85 shows the stent, scaffold and valve with the sleeve in the retracted configuration. The distal olive 666 of the delivery system is also shown.

Referring to Fig. 86, water is flushed through the delivery system to elongate the plastic sleeve, which passes through the duodenum past the ligament of trietz.

Referring to Fig. 87, when the implant device is deployed the delivery system is removed and the distal olive 666 passes through the intestine.

In the case of the delivery system of Figs. 84 to 89 the valve and scaffold are deployed before the sleeve is deployed. In this arrangement the proximal seal is provided by the proximal balloon which seals against the valve as illustrated in Fig. 87.

Referring to Figs. 90 to 97 there is illustrated another delivery system. In this case the valve and scaffold are deployed after deployment of the sleeve. In this arrangement the proximal seal is provided by the proximal balloon 662 which in this case seals against the inner wall of a distal capsule 669. The balloon 662 is not fully inflated in Figs. 91, 92 and 94. A delivery catheter comprises an outer shaft 680 with a retraction hub 681 and an inner shaft 682. The shaft has various lumens and at the proximal end there are various ports connected with the lumens. There is a proximal sleeve inflation port 683, a distal tip balloon inflation port 684, a proximal seal or plunger balloon inflation port 685. There is also a guidewire port 686 (which is illustrated in Fig. 96) for a guidewire 687. Fig. 97 shows the various lumens, - a water injection lumen 690 for deployment of the sleeve, - a proximal balloon inflation lumen 691, a distal tip balloon inflation lumen 692 and a guidewire lumen 693. A flexible tube 688 extends through a lumen 689 in the inner shaft 682. The flexible tube 688 also extends through the proximal balloon 662 which in this case is of doughnut shape. The tube 688 has an outlet for inflation of balloon 665.

Referring to Fig. 90 the capsule 669 is mechanically releasable from the outer sheath, for example through a screw thread connection 695. In use, the shaft of the delivery system is inserted through the proximal end of a delivery channel of an endoscope. When the distal end of the shaft of the delivery shaft exits the distal end of the endoscope delivery channel the capsule is mounted to the distal end of the delivery shaft using the mechanical attachment which in this case is a screw-in attachment.

In Fig. 90 the sleeve / valve / scaffold implant device is in the retracted delivery configuration. The flexible tube 688 extends to the tip balloon 665 and has a hole through which air is delivered for inflation of the balloon 665. The tube 688 is of a suitable flexible material such as a plastics, for example nylon.

Referring to Fig. 92, the proximal balloon 662 is inflated to seal the sleeve 601 at the proximal end and the distal balloon 665 is inflated to seal the sleeve 601 at the distal end. Water is then flushed into the retracted sleeve 601 and by virtue of the seals 662, 665 at the proximal and distal ends, the water fills the sleeve 601, causing it to extend. The sleeve 601 is shown in a partially extended configuration in Fig. 92.

When the sleeve 601 has fully extended (Fig. 93) the distal balloon 665 is deflated, allowing the tip 666 to float into the intestine for discharge. The proximal balloon 662 remains inflated and acts as a plunger to deploy the scaffold from the capsule 669. The scaffold 605 engages with the stent 606 as described above and the delivery system is withdrawn as illustrated in Fig. 94.

Fig. 95 illustrates the proximal delivery components. The retraction hub 681 is connected to the outer shaft to enable withdrawal of the outer shaft 680 over the inner shaft 682.

Fig. 98 is a graph of the pressure profile of fixed orifice restrictors with various size orifices. The restrictions were created using a 1mm thick polyethylene membrane. Each orifice was created by drilling out the desired hole size followed by verification using a Vernier calliper. The flowrate through the test fixture was controlled at 7.86g / sec with a fluid having a viscosity of 39,000 Cps. It will be noted that when a series of fixed diameter orifice restrictors are used to impede fluid flow, the resulting back-pressures generated have a distinctive pattern. The back-pressure initially rises sharply followed by a sustained gradual pressure rise until flow is stopped. This behaviour is illustrated by Fig. 98 for 4mm, 5mm and 6mm diameter restrictions. This is undesirable for use as a flow restrictor in the stomach because a constant rise in pressure as a function of flow might give rise to gastric distress and cramping.

Fig. 99 is a pressure profile of various different restrictions. The 6mm orifice is made as described above for Fig. 98. The pressure profile represented by interrupted lines is generated using a leaflet valve as described above with reference to Figs. 58 - 65. The valve is of a viscoelastic foam material. The foam material is in this case a material described in Example 5 of the Group 1 materials described below. The density of the material was 0.9g/ml. It can be seen from Fig. 99 that a coapting valve of the above description enables the generation of a constant back-pressure over the duration of fluid flow. The valve is thus adapting to fluid flow to maintain a constant restrictive force independent of fluid flow therethrough.

The performance of the valve can be tailored by adjusting the material density, this for example can be achieved by introducing more or less material into the valve forming mold, which subsequently expands to fill the cavity. Referring to Fig. 100, the valve was made using the same material as in Fig. 99 but in this case the density was changed to approximately 0.76g/ml. Through this modification it was possible to produce a valve that generated an initially high back-pressure and subsequently adapted to the fluid flow thus lowering the back-pressure. Such a valve has an initial barrier function followed by a steady state restriction. The valve impedes flow until a pre-determined set-point pressure after which the back pressure remains substantially constant thus providing a predictable stomach emptying rate.

Various materials can be used for fabrication of the sleeve portion of the device. These materials can be for example; polyethylene, PTFE or FEP due to their low friction thus not impeding fluid flow therethrough.

Referring to Figs. 101 and 102 a sleeve 750 has means to visualise the deployment of the sleeve using a radiopaque marker. A radiopague ink or paint is used. Because of the chemical nature of the sleeve materials the

adhesion of a coating is very difficult. A longitudinal pocket 751 is provided which may be created by overlapping a portion of the sleeve material. Into this pocket 751 is deposited a radiopaque material 752 such as a liquid silicon resin filled with BaSO4, which is subsequently cured. This facilitates a low profile and a fluoroscopically distinguishable marker for visualisation in the body. Referring to Fig. 103 in this case the sleeve has a plurality of pockets 760 which may be arranged in any desired manner to facilitate visualisation, for example at particular locations.

The following sections describes two group of biomaterials that are suitable for manufacturing a valve.

**Group 1**

It has been found that in addition to the low pH and digestive enzymes of the stomach, that digestive enzymes, which originate in the pancreas are particularly effective at degrading various food constituents. Many medical devices are used in this part of the anatomy for reflux control, biliary drainage catheters, biliary stents and obesity devices. The biomaterials that are currently being used for these applications include siloxanes, polyether polyurethanes and polycarbonate polyurethanes. These materials suffer from a variety of degradation mechanisms attributable to either enzymes in the stomach or intestines or the extremes of high and low pH that exists in the intestines and stomach respectively.

According to certain embodiments there is provided a triblock copolymer of formula [polybutadiene][polyalkyl ether] [polysiloxane], *e.g.*, a triblock copolymer of formula I:

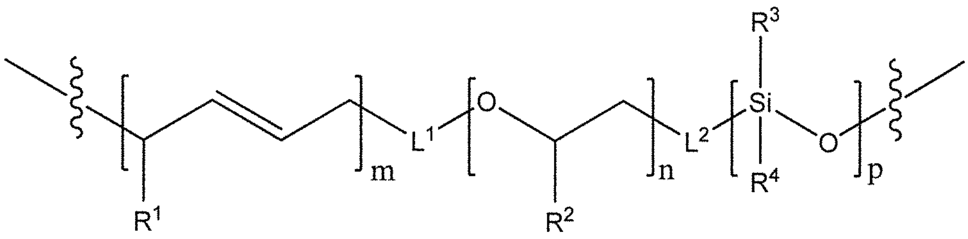

**I**

wherein the copolymers are chemically interspersed (bound) between urethane and/or urea linkages and wherein each of m, n, p, $L^1$, $L^2$, $R^1$, $R^2$, $R^3$, and $R^4$ is as defined and described herein.

A biomaterial may comprise a copolymer as defined and described herein. In certain cases, a medical device comprises a provided copolymer as defined and described herein.

Also described is a polyurethane/urea foam comprising a provided copolymer as defined and described herein. Also described isa biostable viscoelastic foam comprising a provided copolymer as defined and described herein.

Also described is a pre-formed soft segment for a polyurethane / urea foam wherein the soft segment is of formula I as defined and described herein.

Described herein are urethane/urea triblock copolymers and associated foams made from alternating blocks of butadiene, siloxane, and alkylether polymers, which are chemically and mechanically stable in the environment of the digestive system. The block copolymers may comprise siloxane, alkyl ether and alkylene polymers wherein either all three or two of the blocks are linked via urethane / urea functional groups.

Also described a multiblock copolymer that is biomimetic and hydrolytically stable in a gastric environment. Such multiblock copolymers are triblock copolymers of the formula [polybutadiene][polyalkyl ether] [polysiloxane]. In certain embodiments, provided multiblock copolymers are of formula I:

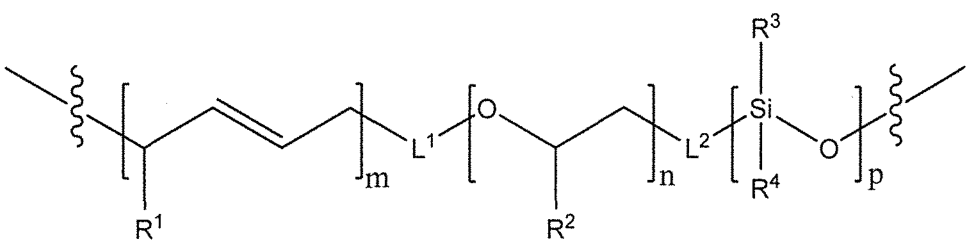

**I**

wherein:

each 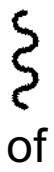 represents a point of attachment to a urethane or urea linkage;

each of $R^1$, $R^2$, $R^3$, and $R^4$ is independently selected from one or more of halogen, R, OR, $-CO_2R$, a fluorinated

hydrocarbon, a polyether, a polyester or a fluoropolymer;

each R is independently hydrogen, an optionally substituted $C_{1-20}$ aliphatic group, or an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulfur;

each of m n and p is independently 2 to 100; and

each of $L^1$ and $L^2$ is independently a bivalent $C_{1-20}$ hydrocarbon chain wherein 1-4 methylene units of the hydrocarbon chain are optionally and independently replaced by -O-, -S-, -N(R)C(O)O-, -N(R)C(O)N(R)-, -OC(O)N(R)-, -N(R)-, -C(O)-, -C(O)N(R)-, -N(R)C(O)-,$-SO_2-$, $-SO_2N(R)-$, $-N(R)SO_2-$, -OC(O)-, -C(O)O-, or a bivalent cycloalkylene, arylene, heterocyclene, or heteroarylene.

In certain examples, a provided copolymer comprises alternating blocks of butadiene (B), alkylether (A) and Siloxane (S) that are linked together with urethane/urea linkages.

In certain examples, a provided copolymer is used for a medical device to be used in the digestive system and other anatomical regions that have high concentrations of enzymes and other degrading species.

In certain examples, a provided copolymer has low water uptake thus contributing to improved chemical stability.

In certain examples, provided copolymers are biocompatible. In certain embodiments, a provided copolymer has high elongation, flexibility, chemical and mechanical stability in harsh environments.

In certain examples, a provided copolymer shows improved performance in terms of water uptake as shown in Figure 105 and chemical stability/biodurability as shown in Figure 104 and Figure 106.

### *2. Definitions:*

Compounds include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

As described herein, compounds may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted." In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable", as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40°C or less, in the absence of moisture or other chemically reactive conditions, for at least a week.

The term "aliphatic" or "aliphatic group", as used herein, denotes a hydrocarbon moiety that may be straight-chain (i.e., unbranched), branched, or cyclic (including fused, bridging, and spiro-fused polycyclic) and may be completely saturated or may contain one or more units of unsaturation, but which is not aromatic. Unless otherwise specified, aliphatic groups contain 1-20 carbon atoms. In some embodiments, aliphatic groups contain 1-10 carbon atoms. In other embodiments, aliphatic groups contain 1-8 carbon atoms. In still other embodiments, aliphatic groups contain 1-6 carbon atoms, and in yet other embodiments aliphatic groups contain 1-4 carbon atoms. Suitable aliphatic groups include, but are not limited to, linear or branched, alkyl, alkenyl, and alkynyl groups, and hybrids thereof such as (cycloalkyl)alkyl, (cycloalkenyl)alkyl or (cycloalkyl)alkenyl.

The term "lower alkyl" refers to a $C_{1-4}$ straight or branched alkyl group. Exemplary lower alkyl groups are methyl, ethyl, propyl, isopropyl, butyl, isobutyl, and tert-butyl.

The term "lower haloalkyl" refers to a $C_{1-4}$ straight or branched alkyl group that is substituted with one or more halogen atoms.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or $NR^+$ (as in N-substituted pyrrolidinyl)).

The term "unsaturated", as used herein, means that a moiety has one or more units of unsaturation.

As used herein, the term "bivalent $C_{1-8}$ [or $C_{1-6}$] saturated or unsaturated, straight or branched, hydrocarbon chain", refers to bivalent alkylene, alkenylene, and alkynylene chains that are straight or branched as defined herein.

The term "alkylene" refers to a bivalent alkyl group. An "alkylene chain" is a polymethylene group, i.e., $-(CH_2)_n-$, wherein n is a positive integer, preferably from 1 to 6, from 1 to 4, from 1 to 3, from 1 to 2, or from 2 to 3. A substituted alkylene chain is a polymethylene group in which one or more methylene hydrogen atoms are replaced with a substituent. Suitable substituents include those described below for a substituted aliphatic group.

The term "alkenylene" refers to a bivalent alkenyl group. A substituted alkenylene chain is a polymethylene group containing at least one double bond in which one or more hydrogen atoms are replaced with a substituent. Suitable substituents include those described below for a substituted aliphatic group.

The term "halogen" means F, Cl, Br, or I.

The term "aryl" used alone or as part of a larger moiety as in "aralkyl", "aralkoxy", or "aryloxyalkyl", refers to monocyclic or bicyclic ring systems having a total of five to fourteen ring members, wherein at least one ring in the system is aromatic and wherein each ring in the system contains 3 to 7 ring members. The term "aryl" may be used interchangeably with the term "aryl ring".

As described herein, compounds may contain "optionally substituted" moieties. In general, the term "substituted", whether preceded by the term "optionally" or not, means that one or more hydrogens of the designated moiety are replaced with a suitable substituent. Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group are independently halogen; $-(CH_2)_{0-4}R°$; $-(CH_2)_{0-4}OR°$; $-O-(CH_2)_{0-4}C(O)OR°$; $-(-CH_2)_{0-4}CH(OR°)_2$; $-(CH_2)_{0-4}SR°$; $-(CH_2)_{0-4}Ph$, which may be substituted with R°; $-(CH_2)_{0-4}O(CH_2)_{0-1}Ph$ which may be substituted with R°; -CH=CHPh, which may be substituted with R°; $-NO_2$; -CN; $-N_3$; $-(CH_2)_{0-4}N(R°)_2$; $-(CH_2)_{0-4}N(R°)C(O)R°$; -N(R°)C(S)R°; $-(CH_2)_{0-4}N(R°)C(O)NR°_2$ ; $-N(R°)C(S)NR°_2$ ; $-(CH_2)_{0-4}N(R°)C(O)OR°$; -N(R°)N(R°)C(O)R°; $-N(R°)N(R°)C(O)NR°_2$; -N(R°)N(R°)C(O)OR°; $-(CH_2)_{0-4}C(O)R°$; -C(S)R°; $-(CH_2)_{0-4}C(O)OR°$; $-(CH_2)_{0-4}C(O)SR°$; $-(CH_2)_{0-4}C(O)OSiR°_3$; $-(CH_2)_{0-4}OC(O)R°$; $-OC(O)(CH_2)_{0-4}SR-$, SC(S)SR°; $-(CH_2)_{0-4}SC(O)R°$; $-(CH_2)_{0-4}C(O)NR°_2$; $-C(S)NR°_2$; -C(S)SR°; -SC(S)SR°, $-(CH_2)_{0-4}OC(O)NR°_2$; -C(O)N(OR°)R°; -C(O)C(O)R°; $-C(O)CH_2C(O)R°$; -C(NOR°)R°; $-(CH_2)_{0-4}SSR°$; $-(CH_2)_{0-4}S(O)_2R°$; $-(CH_2)_{0-4}S(O)_2OR°$; $-(CH_2)_{0-4}OS(O)_2R°$; $-S(O)_2NR°_2$; $-(CH_2)_{0-4}S(O)R°$; $-N(R°)S(O)_2NR°_2$; $-N(R°)S(O)_2R°$; -N(OR°)R°; $-C(NH)NR°_2$; $-P(O)_2R°$; $-P(O)R°_2$; $-O P(O)R°_2$; $-OP(O)(OR°)_2$; $SiR°_3$; $-(C_{1-4}$ straight or branched alkylene)$O-N(R°)_2$; or $-(C_{1-4}$ straight or branched alkylene)$C(O)O-N(R°)_2$, wherein each R° may be substituted as defined below and is independently hydrogen, $C_{1-6}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, $-(CH_2)_{0-2}R^•$, $-(haloR^•)$, $-(CH_2)_{0-2}OH$, $-(CH_2)_{0-2}OR^•$, $-(CH_2)_{0-2}CH(OR^•)_2$; $-O(haloR^•)$, -CN, $-N_3$, $-(CH_2)_{0-2}C(O)R^•$, $-(CH_2)_{0-2}C(O)OH$, $-(CH_2)_{0-2}C(O)OR^•$, $-(CH_2)_{0-2}SR^•$, $-(CH_2)_{0-2}SH$, $-(CH_2)_{0-2}NH_2$, $-(CH_2)_{0-2}NHR^•$, $-(CH_2)_{0-2}NR^•_2$, $-NO_2$, $-SiR^•_3$, $-OSiR^•_3$, $-C(O)SR^•$, $-(C_{1-4}$ straight or branched alkylene)$C(O)OR^•$, or $-SSR^•$ wherein each $R^•$ is unsubstituted or where preceded by "halo" is substituted only with one or more halogens, and is independently selected from $C_{1-4}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, $=NNR^*_2$, $=NNHC(O)R^*$, $=NNHC(O)OR^*$, $=NNHS(O)_2R^*$, $=NR^*$, $=NOR^*$, $-O(C(R^*_2))_{2-3}O-$, or $-S(C(R^*_2))_{2-3}S-$, wherein each independent occurrence of $R^*$ is selected from hydrogen, $C_{1-6}$ aliphatic which may be substituted as defined below, or an unsubstituted 5-6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: $-O(CR^*_2)_{2-3}O-$, wherein each independent occurrence of $R^*$ is selected from hydrogen, $C_{1-6}$ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of $R^*$ include halogen, -R', -(halo$R^{\bullet}$), -OH, -O$R^{\bullet}$, -O(halo$R^{\bullet}$), -CN, -C(O)OH, -C(O)O$R^{\bullet}$, $-NH_2$, -NH$R^{\bullet}$, $-NR^{\bullet}_2$, or $-NO_2$, wherein each $R^{\bullet}$ is unsubstituted or where preceded by "halo" is substituted only with one or more halogens, and is independently $C_{1-4}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur.

Suitable substituents on a substitutable nitrogen of an "optionally substituted" group include $-R^{\dagger}$, $-NR^{\dagger}_2$, $-C(O)R^{\dagger}$, $-C(O)OR^{\dagger}$, $-C(O)C(O)R^{\dagger}$, $-C(O)CH_2C(O)R^{\dagger}$, $-S(O)_2R^{\dagger}$, $-S(O)_2NR^{\dagger}_2$, $-C(S)NR^{\dagger}_2$, $-C(NH)NR^{\dagger}_2$, or $-N(R^{\dagger})S(O)_2R^{\dagger}$; wherein each $R^{\dagger}$ is independently hydrogen, $C_{1-6}$ aliphatic which may be substituted as defined below, unsubstituted -OPh, or an unsubstituted 5-6-membered saturated, partially unsaturated, or aryl ring having 0-4 heteroatoms independently selected from nitrogen, oxygen, or sulfur, or, notwithstanding the definition above, two independent occurrences of $R^{\dagger}$, taken together with their intervening atom(s) form an unsubstituted 3-12-membered saturated, partially unsaturated, or aryl mono- or bicyclic ring having 0-4 heteroatoms independently selected from nitrogen, oxygen, or sulfur. Suitable substituents on the aliphatic group of $R^{\dagger}$ are independently halogen, $-R^{\bullet}$, -(halo$R^{\bullet}$), -OH, -O$R^{\bullet}$, -O(halo$R^{\bullet}$), -CN, -C(O)OH, -C(O)O$R^{\bullet}$, $-NH_2$, -NH$R^{\bullet}$, $-NR^{\bullet}_2$, or $-NO_2$, wherein each $R^{\bullet}$ is unsubstituted or where preceded by "halo" is substituted only with one or more halogens, and is independently $C_{1-4}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur.

*3. Description of Exemplary Embodiments:*

*A. Multiblock Copolymers*

As described generally above, one embodiment provides a triblock copolymer of formula [polybutadiene][polyalkyl ether] [polysiloxane]. In certain embodiments, a provided triblock copolymer is of formula I:

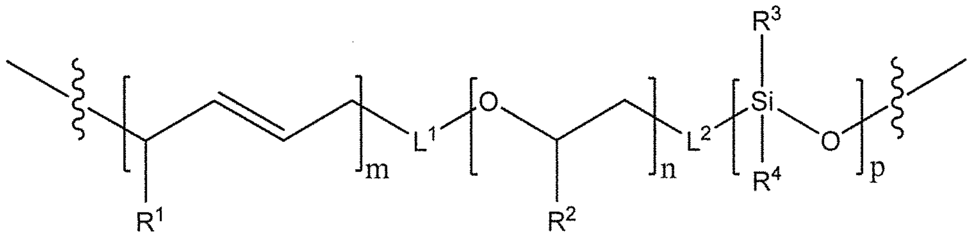

I

wherein the copolymers are chemically interspersed (bound) between urethane and/or urea linkages (i.e., at the bond designated with 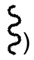) and wherein each of m, n, p, $L^1$, $L^2$, $R^1$, $R^2$, $R^3$, and $R^4$ is as defined and described herein.

In certain embodiments, m and p are each independently between 2 and 50 and n is between 2 and 20. In some embodiments, m and p are each independently between 2 and 30 and n is between 2 and 20. In certain embodiments, each of m, n, and p are independently from 8 to 16.

As defined generally above, each of $R^1$, $R^2$, $R^3$, and $R^4$ is independently selected from one or more of halogen, R, OR, $-CO_2R$, a fluorinated hydrocarbon, a polyether, a polyester or a fluoropolymer. In some embodiments, one or more of $R^1$, $R^2$, $R^3$, $R^4$, $R^5$ and $R^6$ is $-CO_2R$. In some embodiments, one or more of $R^1$, $R^2$, $R^3$, $R^4$, $R^5$ and $R^6$ is $-CO_2R$ wherein each R is independently an optionally substituted $C_{1-6}$ aliphatic group. In certain embodiments, one or more of $R^1$, $R^2$, $R^3$, and $R^4$ is $-CO_2R$ wherein each R is independently an unsubstituted $C_{1-6}$ alkyl group. Exemplary such groups include methanoic or ethanoic acid as well as methacrylic acid and other acrylic acids.

In certain embodiments, one or more of $R^1$, $R^2$, $R^3$, and $R^4$ is independently R. In some embodiments, one or more of $R^1$, $R^2$, $R^3$, and $R^4$ is an optionally substituted $C_{1-6}$ aliphatic group. In certain embodiments, one or more of $R^1$, $R^2$, $R^3$, and $R^4$ is an optionally substituted $C_{1-6}$ alkyl. In other embodiments, one or more of $R^1$, $R^2$, $R^3$, and $R^4$ is an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulphur. Exemplary such $R^1$, $R^2$, $R^3$, and $R^4$ groups include methyl, ethyl, propyl, isopropyl, cyclopropyl, butyl, isobutyl, cyclobutyl, phenyl, pyridyl, morpholinyl, pyrrolidinyl), imidazolyl, and cyclohexyl. In certain embodiments, one or more of $R^1$, $R^2$, $R^3$, and $R^4$ is methyl, ethyl, propyl, or a higher homolog. In certain embodiments, $R^2$ is methyl, ethyl, propyl, or a higher homolog. In certain embodiments, $R^2$ is methyl.

In certain embodiments, one or more of $R^1$, $R^2$, $R^3$, and $R^4$ is independently -OR. In some embodiments, one

or more of $R^1$, $R^2$, $R^3$, and $R^4$ is -OR wherein R is an optionally substituted $C_{1-6}$ aliphatic group. In certain embodiments, one or more of $R^1$, $R^2$, $R^3$, and $R^4$ is -OR wherein R is $C_{1-6}$ alkyl. In other embodiments, one or more of $R^1$, $R^2$, $R^3$, and $R^4$ is-OR wherein R is an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulphur. Exemplary such $R^1$, $R^2$, $R^3$, $R^4$, $R^5$ and $R^6$ groups include - Omethyl, -Oethyl, -Opropyl, -Oisopropyl, -Ocyclopropyl, -Obutyl, -Oisobutyl, -Ocyclobutyl, - Ophenyl, -Opyridyl, -Omorpholinyl, -Opyrrolidinyl, -Oimidazolyl, and -Ocyclohexyl.

In certain embodiments, one or more of $R^1$, $R^2$, $R^3$, and $R^4$ is independently R wherein each R is a $C_{1-6}$ aliphatic group substituted with one or more halogens. In some embodiments, each R is $C_{1-6}$ aliphatic substituted with one, two, or three halogens. In other embodiments, each R is a perfluorinated $C_{1-6}$ aliphatic group. Examples of fluorinated hydrocarbons represented by $R^1$, $R^2$, $R^3$, and $R^4$ include mono-, di-, tri, or perfluorinated methyl, ethyl, propyl, butyl, or phenyl. In some embodiments, each of $R^1$, $R^2$, $R^3$, and $R^4$ is trifluoromethyl, trifluoroethyl, or trifluoropropyl.

In certain embodiments, one or more of $R^1$, $R^2$, $R^3$, and $R^4$ is independently a polyether. Examples of polyethers represented by $R^1$, $R^2$, $R^3$, and $R^4$ include poly(ethylene oxide), poly(difluoromethyl ethylene oxide), poly(trifluoromethyl ethylene oxide), poly(propylene oxide), poly(difluoromethyl propylene oxide), poly(propylene oxide), poly(trifluoromethyl propylene oxide), poly(butylene oxide), poly(tetramethylene ether glycol), poly(tetrahydrofuran), poly(oxymethylene), poly(ether ketone), poly(etherether ketone) and copolymers thereof.

In certain embodiments, one or more of $R^1$, $R^2$, $R^3$, $R^4$, $R^5$ and $R^6$ is independently a polyester. Examples of polyesters represented by $R^1$, $R^2$, $R^3$, $R^4$, $R^5$ and $R^6$ include poly(ethylene terephthalate) (PET), poly(ethylene terephthalate ionomer) (PETI), poly(ethylene naphthalate) (PEN), poly(methylene naphthalate) (PTN), poly(butylene teraphalate) (PBT), poly(butylene naphthalate) (PBN), polycarbonate.

In certain embodiments, one or more of $R^1$, $R^2$, $R^3$, $R^4$, $R^5$ and $R^6$ is independently a fluoropolymer. Examples of fluoropolymers represented by $R^1$, $R^2$, $R^3$, $R^4$, $R^5$ and $R^6$ include poly(tetrafluoroethylene), poly(methyl di-fluoroethyl siloxane), poly(methyl tri-fluoroethyl siloxane), poly(phenyl di-fluoroethyl siloxane).

In some embodiments, $R^1$, $R^2$, $R^3$, and $R^4$ is independently hydrogen, hydroxyl, carboxylic acids such as methanoic or ethanoic acid as well as methacrylic acid and other acrylic acids. Alkyl or aryl hydrocarbons such as methyl, ethyl, propyl, butyl, phenyl and ethers thereof. Fluorinated hydrocarbons such as mono-, di-, tri, or perfluorinated methyl, ethyl, propyl, butyl, phenyl. Polyether such as Poly(ethylene oxide), poly(difluoromethyl ethylene oxide), poly(trifluoromethyl ethylene oxide), poly(propylene oxide), poly(difluoromethyl propylene oxide), poly(propylene oxide), poly(trifluoromethyl propylene oxide), poly(butylene oxide), poly(tetramethylene ether glycol), poly(tetrahydrofuran), poly(oxymethylene), poly(ether ketone), poly(etherether ketone) and copolymers thereof. Polyesters such as Poly(ethylene terephthalate) (PET), poly(ethylene terephthalate ionomer) (PETI), poly(ethylene naphthalate) (PEN), poly(methylene naphthalate) (PTN), Poly(Butylene Teraphalate) (PBT), poly(butylene naphthalate) (PBN), polycarbonate and .fluoropolymer such as Poly(tetrafluoroethylene), poly(methyl di-fluoroethyl siloxane), poly(methyl tri-fluoroethyl siloxane), poly(phenyl di-fluoroethyl siloxane).

In some embodiments, $R^1$ is hydrogen. In some embodiments, $R^1$ is halogen. In certain embodiments, $R^1$ is chloro or fluoro. In some embodiments, $R^1$ is an optionally substituted $C_{1-6}$ alkyl. In certain embodiments, $R^1$ is methyl.

In some embodiments, $R^2$ is hydrogen. In some embodiments, $R^2$ is an optionally substituted $C_{1-6}$ alkyl. In certain embodiments, $R^2$ is methyl, ethyl, propyl, or a higher homolog. In certain embodiments, $R^2$ is methyl.

In some embodiments, $R^3$ is an optionally substituted $C_{1-6}$ alkyl. In certain embodiments, $R^3$ is methyl. In certain embodiments, $R^3$ is $-CH_2CH_2F$. In some embodiments, $R^3$ is halogen. In certain embodiments, $R^3$ is fluoro. In some embodiments, $R^3$ is phenyl.

In some embodiments, $R^4$ is an optionally substituted $C_{1-6}$ alkyl. In certain embodiments, $R^4$ is methyl. In certain embodiments, $R^4$ is $-CH_2CH_2F$. In some embodiments, $R^4$ is halogen. In certain embodiments, $R^4$ is fluoro. In some embodiments, $R^4$ is phenyl

As defined generally above, each of $L^1$ and $L^2$ is independently a bivalent $C_{1-20}$ hydrocarbon chain wherein 1-4 methylene units of the hydrocarbon chain are optionally and independently replaced by -O-, -S-, -NHC(O)O-, -NHC(O)NH-, -N(R)-, -C(O)-, -C(O)N(R)-, -N(R)C(O)-,-SO2-, -SO2N(R)-, -N(R)SO2-, -OC(O)-, -C(O)O-, or a bivalent cycloalkylene, arylene, heterocyclene, or heteroarylene. In some embodiments, each of $L^1$ and $L^2$ is independently a bivalent $C_{1-20}$ alkylene chain. In certain embodiments, each of $L^1$ and $L^2$ is independently a bivalent $C_{1-10}$ alkylene chain. In certain embodiments, each of $L^1$ and $L^2$ is independently a bivalent $C_{1-6}$ alkylene chain. In certain embodiments, each of $L^1$ and $L^2$ is independently a bivalent $C_{1-4}$ alkylene chain. Exemplary such $L^1$ and $L^2$ groups include methylene, ethylene, propylene, butylene or higher bivalent alkanes.

In some embodiments, each of $L^1$ and $L^2$ is independently a bivalent $C_{1-20}$ alkylene chain wherein one methylene unit of the chain is replaced by -O-. In some embodiments, each of $L^1$ and $L^2$ is independently a bivalent $C_{1-10}$ alkylene chain wherein one methylene unit of the chain is replaced by -O-. In some embodiments, each of $L^1$ and $L^2$ is independently a bivalent $C_{1-6}$ alkylene chain wherein one methylene unit of the chain is replaced by -O-. In some embodiments, each

of $L^1$ and $L^2$ is independently a bivalent $C_{1-4}$ alkylene chain wherein one methylene unit of the chain is replaced by -O-. Exemplary such $L^1$ and $L^2$ groups include $-OCH_2-$, $-OCH_2CH_2-$, $-OCH_2CH_2CH_2-$, $-OCH_2CH_2CH_2CH_2-$, or higher bivalent alkylene ethers.

In some embodiments, each of $L^1$ and $L^2$ is independently a bivalent $C_{1-20}$ alkylene chain wherein at least one methylene unit of the chain is replaced by -O- and at least one methylene unit of the chain is replaced by a bivalent arylene. In some embodiments, each of $L^1$ and $L^2$ is independently a bivalent $C_{1-10}$ alkylene chain wherein at least one methylene unit of the chain is replaced by -O- and at least one methylene unit of the chain is replaced by a bivalent arylene. In some embodiments, each of $L^1$ and $L^2$ is independently a bivalent $C_{1-6}$ alkylene chain wherein at least one methylene unit of the chain is replaced by -O- and at least one methylene unit of the chain is replaced by a bivalent arylene. In some embodiments, each of $L^1$ and $L^2$ is independently a bivalent $C_{1-4}$ alkylene chain wherein at least one methylene unit of the chain is replaced by -O- and at least one methylene unit of the chain is replaced by a bivalent arylene. Exemplary such $L^1$ and $L^2$ groups include $-OCH_2$-phenylene-, $-OCH_2CH_2$-phenylene-, $-OCH_2CH_2$-phenylene-$CH_2$-, $-OCH_2CH_2CH_2CH_2$--phenylene-, and the like.

In some embodiments, each of $L^1$ and $L^2$ is independently a bivalent $C_{1-20}$ alkylene chain wherein three methylene units of the chain are replaced by -N(R)C(O)N(R)-, -N(R)C(O)O-, or - OC(O)N(R)-. In some embodiments, each of $L^1$ and $L^2$ is independently a bivalent $C_{1-10}$ alkylene chain wherein one methylene unit of the chain is replaced by -O-. In some embodiments, each of $L^1$ and $L^2$ is independently a bivalent $C_{1-6}$ alkylene chain wherein one methylene unit of the chain are replaced by -N(R)C(O)N(R)-, -N(R)C(O)O-, or -OC(O)N(R)-. In some embodiments, each of $L^1$ and $L^2$ is independently a bivalent $C_{1-4}$ alkylene chain wherein one methylene unit of the chain are replaced by -N(R)C(O)N(R)-, -N(R)C(O)O-, or - OC(O)N(R)-. Exemplary such $L^1$ and $L^2$ groups include -N(H)C(O)N(H)-, -N(H)C(O)O-, and - OC(O)N(H)-.

In some embodiments, $L^1$ is a urethane. In some embodiments, $L^2$ is $-CH_2CH_2-$.

In some embodiments, $R^1$ and $R^2$ is methyl. In some embodiments, one or both of $R^3$ and $R^4$ is independently a $C_{1-20}$ hydrocarbon chain wherein 1-4 methylene units of the hydrocarbon chain are optionally and independently substituted by halogen. In some embodiments, $R^3$ is a $C_{1-20}$ hydrocarbon chain wherein 1-4 methylene units of the hydrocarbon chain are optionally and independently substituted by halogen. In some embodiments, $R^4$ is a $C_{1-20}$ hydrocarbon chain wherein 1-4 methylene units of the hydrocarbon chain are optionally and independently substituted by halogen. In some embodiments, $R^3$ and $R^4$ are independently a $C_{1-20}$ hydrocarbon chain wherein 1-4 methylene units of the hydrocarbon chain are optionally and independently substituted by halogen. In some embodiments, one or both of $R^3$ and $R^4$ is independently a $C_{1-20}$ hydrocarbon chain. In some embodiments, $R^3$ is a $C_{1-20}$ hydrocarbon chain. In some embodiments, $R^4$ is a $C_{1-20}$ hydrocarbon chain. In some embodiments, $R^3$ and $R^4$ are independently a $C_{1-20}$ hydrocarbon chain. In some embodiments, $L^1$ and $L^2$ are independently a bivalent $C_{1-20}$ hydrocarbon chain wherein 1-4 methylene units of the hydrocarbon chain are optionally and independently replaced by -NHC(O)O- or -NHC(O)NH-. In some embodiments, $L^1$ is -NHC(O)O- or -NHC(O)NH-. In some embodiments, $L^2$ is a $C_{1-20}$ hydrocarbon chain.

In some examples, $R^1$ and $R^2$ are methyl, one or both of $R^3$ and $R^4$ is independently a $C_{1-20}$ hydrocarbon chain wherein 1-4 methylene units of the hydrocarbon chain are optionally and independently substituted by halogen, and $L^1$ and $L^2$ are independently a bivalent $C_{1-20}$ hydrocarbon chain wherein 1-4 methylene units of the hydrocarbon chain are optionally and independently replaced by -NHC(O)O- or -NHC(O)NH-.

In some examples, $R^1$ and $R^2$ are methyl, one or both of $R^3$ and $R^4$ is independently a $C_{1-20}$ hydrocarbon chain wherein 1-4 methylene units of the hydrocarbon chain are optionally and independently substituted by halogen, $L^1$ is -NHC(O)O- or NHC(O)NH-, and $L^2$ is a $C_{1-20}$ hydrocarbon chain.

In some examples, $R^1$ and $R^2$ are methyl, one or both of $R^3$ and $R^4$ is independently a $C_{1-20}$ hydrocarbon chain, $L^1$ is -NHC(O)O- or NHC(O)NH-, and $L^2$ is a $C_{1-20}$ hydrocarbon chain. One of ordinary skill in the art would understand that a polyurethane results from the reaction of an isocyanate and a hydroxyl group. Similarly, a polyurea results from the reaction of an isocyanate and an amine. Each of these reactions is depicted below.

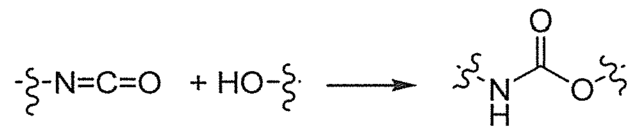

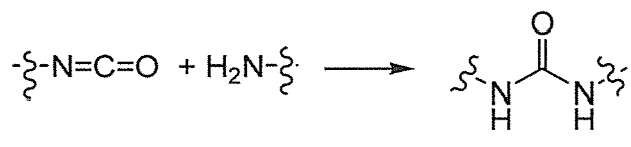

Thus, it is readily apparent that provided compounds of formula I can be functionalized with end groups suitable for forming urethane and/or urea linkages. Also a compound of formula II:

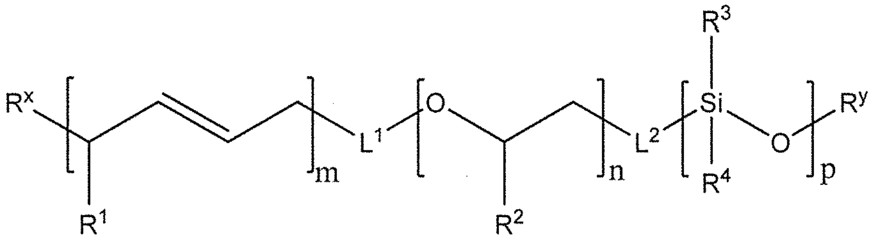

II

wherein:

each of $R^x$ and $R^y$ is independently -OH, $-NH_2$, a protected hydroxyl or a protected amine;
each of $R^1$, $R^2$, $R^3$, and $R^4$ is independently selected from one or more of halogen, R, OR, $-CO_2R$, a fluorinated hydrocarbon, a polyether, a polyester or a fluoropolymer;
each R is independently hydrogen, an optionally substituted $C_{1-20}$ aliphatic group, or an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulfur;
each of m, n, and p is independently 2 to 100; and
each of $L^1$ and $L^2$ is independently a bivalent $C_{1-20}$ hydrocarbon chain wherein 1-4 methylene units of the hydrocarbon chain are optionally and independently replaced by -O-, -S-, - N(R)C(O)O-, -N(R)C(O)N(R)-, -OC(O)N(R)-, -N(R)-, -C(O)-, -C(O)N(R)-, -N(R)C(O)-, - SO2-, -SO2N(R)-, -N(R)SO2-, -OC(O)-, -C(O)O-, or a bivalent cycloalkylene, arylene, heterocyclene, or heteroarylene.

In some examples, each of m, n, p, $L^1$, $L^2$, $R^1$, $R^2$, $R^3$, and $R^4$ is as defined and described herein.

As defined generally above, each of $R^x$ and $R^y$ is independently -OH, $-NH_2$, a protected hydroxyl or a protected amine. In some embodiments, both of $R^x$ and $R^y$ are -OH. In other embodiments, both of $R^x$ and $R^y$ are $-NH_2$. In some embodiments one of $R^x$ and $R^y$ is -OH and the other is $-NH_2$.

In some examples, each of $R^x$ and $R^y$ is independently a protected hydroxyl or a protected amine. Such protected hydroxyl and protected amine groups are well known to one of skill in the art and include those described in detail in Protecting Groups in Organic Synthesis, T. W. Greene and P. G. M. Wuts, 3rd edition, John Wiley & Sons, 1999. Exemplary protected amines include methyl carbamate, ethyl carbamante, 9-fluorenylmethyl carbamate (Fmoc), 9-(2-sulfo)fluorenylmethyl carbamate, 9-(2,7-dibromo)fluoroenylmethyl carbamate, 2,7-di-t-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-t-BOC), 1,1-dimethyl-2,2,2-trichloroethyl carbamate (TCBOC), 1-methyl-1-(4-biphenylyl)ethyl carbamate (Bpoc), 1-(3,5-di-t-butylphenyl)-1-methylethyl carbamate (t-Bumeoc), 2-(2'- and 4'-pyridyl)ethyl carbamate (Pyoc), 2-(N,N-dicyclohexylcarboxamido)ethyl carbamate, t-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, N-hydroxypiperidinyl carbamate, alkyldithio carbamate, benzyl carbamate (Cbz), p-methoxybenzyl carbamate (Moz), p-nitobenzyl carbamate, p-bromobenzyl carbamate, p-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(p-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, m-chloro-p-acyloxybenzyl carbamate, p-(dihydroxyboryl)benzyl carbamate, 5-benzisoxazolylmethyl carbamate, 2-(trifluoromethyl)-6-chromonylmethyl carbamate (Tcroc), m-nitrophenyl carbamate, 3,5-dimethoxybenzyl carbamate, o-nitrobenzyl carbamate, 3,4-dimethoxy-6-nitrobenzyl carbamate, phenyl(o-nitrophenyl)methyl carbamate, phenothiazinyl-(10)-carbonyl derivative, N'-p-toluenesulfonylaminocarbonyl derivative, N'-phenylaminothiocarbonyl derivative, t-amyl carbamate, S-benzyl thiocarbamate, p-cyanobenzyl carbamate, cyclobutyl carbamate, cyclohexyl carbamate, cyclopentyl carbamate, cyclopropylmethyl carbamate, p-decyloxybenzyl carbamate, 2,2-dimethoxycarbonylvinyl carbamate, o-(N,N-dimethylcarboxamido)benzyl carbamate, 1,1-dimethyl-3-(N,N-dimethylcarboxamido)propyl carbamate, 1,1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, p-(p'-methoxyphenylazo)benzyl carbamate, 1-methylcyclobutyl carbamate, 1-methylcyclohexyl carbamate, 1-methyl-1-cyclopropylmethyl carbamate, 1-methyl-1-(3,5-dimethoxyphenyl)ethyl carbamate, 1-methyl-1-(p-phenylazophenyl)ethyl carbamate, 1-methyl-1-phenylethyl carbamate, 1-methyl-1-(4-pyridyl)ethyl carbamate, phenyl carbamate, p-(phenylazo)benzyl carbamate, 2,4,6-tri-t-butylphenyl carbamate, 4-(trimethylammonium)benzyl carbamate, 2,4,6-trimethylbenzyl carbamate, formamide, acetamide, chloroacetamide, trichloroacetamide, trifluoroacetamide, phenylacetamide, 3-phenylpropanamide, picolinamide, 3-pyridylcarboxamide, N-benzoylphenylalanyl derivative, benzamide, p-phenylbenzamide, o-nitophenylacetamide, o-nitrophenoxyacetamide, acetoacetamide, (N'-dithiobenzyloxycarbonylamino)acetamide, 3-(p-hydroxyphenyl)propanamide, 3-(o-nitrophenyl)propanamide, 2-methyl-2-(o-nitrophenoxy)propanamide, 2-methyl-2-(o-phenylazophenoxy)propanamide, 4-chlorobutanamide, 3-methyl-3-nitrobutanamide, o-nitrocinnamide, N-acetylmethionine derivative, o-nitrobenzamide, o-(benzoyloxymethyl)benzamide, 4,5-diphenyl-3-oxazolin-2-one, N-phthalimide, N-dithiasuccinimide (Dts), N-2,3-diphenylmaleimide, N-2,5-dimethylpyrrole, N-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1-substituted 3,5-dinitro-4-pyridone, N-methylamine, N-allylamine, N-[2-(trimethylsilyl)ethoxy]methylamine (SEM), N-3-acetoxypropylamine, N-(1-isopropyl-4-nitro-2-oxo-3-pyroolin-3-yl)amine, quaternary ammonium salts, N-benzylamine, N-di(4-methoxyphenyl)methylamine, N-5-dibenzosuberylamine, N-triphenylmethylamine (Tr), N-[(4-methoxyphenyl)diphenylmethyl]amine (MMTr), N-9-phenylfluorenylamine (PhF), N-2,7-dichloro-9-fluorenylmethyleneamine, N-ferrocenylmethylamino (Fcm), N-2-picolylamino N'-oxide, N-1,1-dimethylthiomethyleneamine, N-benzylideneamine, N-p-methoxybenzylideneamine, N-diphenylmethyleneamine, N-[(2-pyridyl)mesityl]methyleneamine, N-(N',N'-dimethylaminomethylene)amine, N,N'-isopropylidenediamine, N-p-nitrobenzylideneamine, N-salicylideneamine, N-5-chlorosalicylideneamine, N-(5-chloro-2-hydroxyphenyl)phenylmethyleneamine, N-cyclohexylideneamine, N-(5,5-dimethyl-3-oxo-1-cyclohexenyl)amine, N-borane derivative, N-diphenylborinic acid derivative, N-[phenyl(pentacarbonylchromium- or tungsten)carbonyl]amine, N-copper chelate, N-zinc chelate, N-nitroamine, N-nitrosoamine, amine N-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o-nitrobenzenesulfenamide (Nps), 2,4-dinitrobenzenesulfenamide, pentachlorobenzenesulfenamide, 2-nitro-4-methoxybenzenesulfenamide, triphenylmethylsulfenamide, 3-nitropyridinesulfenamide (Npys), p-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide.

Exemplary hydroxyl protecting groups include methyl, methoxylmethyl (MOM), methylthiomethyl (MTM), t-butylthiomethyl, (phenyldimethylsilyl)methoxymethyl (SMOM), benzyloxymethyl (BOM), p-methoxybenzyloxymethyl (PMBM), (4-methoxyphenoxy)methyl (p-AOM), guaiacolmethyl (GUM), t-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, t-butyl, allyl, p-chlorophenyl, p-methoxyphenyl, 2,4-dinitrophenyl, benzyl, p-methoxybenzyl, 3,4-dimethoxybenzyl, o-nitrobenzyl, p-nitrobenzyl, p-halobenzyl, 2,6-dichlorobenzyl, p-cyanobenzyl, p-phenylbenzyl, 2-picolyl, 4-picolyl, 3-methyl-2-picolyl N-oxido, diphenylmethyl, p,p'-dinitrobenzhydryl, 5-dibenzosuberyl, triphenylmethyl, α-naphthyldiphenylmethyl, p-methoxyphenyldiphenylmethyl, di(p-methoxyphenyl)phenylmethyl, tri(p-methoxyphenyl)methyl, 4-(4'-bromophenacyloxyphenyl)diphenylmethyl, 4,4',4''-tris(4,5-dichlorophthalimidophenyl)methyl, 4,4',4''-tris(levulinoyloxyphenyl)methyl, 4,4',4''-tris(benzoyloxyphenyl)methyl, 3-(imidazol-1-yl)bis(4',4''-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, t-butyldimethylsilyl (TBDMS), t-butyldiphenylsilyl (TBDPS), tribenzylsilyl, tri-p-xylylsilyl, triphenylsilyl, diphenylmethylsilyl (DPMS), t-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, p-phenylbenzoate, 2,4,6-trimethylbenzoate (mesitoate), alkyl methyl carbonate, 9-fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc),

alkyl isobutyl carbonate, alkyl vinyl carbonate alkyl allyl carbonate, alkyl p-nitrophenyl carbonate, alkyl benzyl carbonate, alkyl p-methoxybenzyl carbonate, alkyl 3,4-dimethoxybenzyl carbonate, alkyl o-nitrobenzyl carbonate, alkyl p-nitrobenzyl carbonate, alkyl S-benzyl thiocarbonate, 4-ethoxy-1-napththyl carbonate, methyl dithiocarbonate, 2-iodobenzoate, 4-azidobutyrate, 4-nitro-4-methylpentanoate, o-(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate, o-(methoxycarbonyl)benzoate, $\alpha$-naphthoate, nitrate, alkyl N,N,N',N'-tetramethylphosphorodiamidate, alkyl N-phenylcarbamate, borate, dimethylphosphinothioyl, alkyl 2,4-dinitrophenylsulfenate, sulfate, methanesulfonate (mesylate), benzylsulfonate, and tosylate (Ts). For protecting 1,2- or 1,3-diols, the protecting groups include methylene acetal, ethylidene acetal, 1-t-butylethylidene ketal, 1-phenylethylidene ketal, (4-methoxyphenyl)ethylidene acetal, 2,2,2-trichloroethylidene acetal, acetonide, cyclopentylidene ketal, cyclohexylidene ketal, cycloheptylidene ketal, benzylidene acetal, p-methoxybenzylidene acetal, 2,4-dimethoxybenzylidene ketal, 3,4-dimethoxybenzylidene acetal, 2-nitrobenzylidene acetal, methoxymethylene acetal, ethoxymethylene acetal, dimethoxymethylene ortho ester, 1-methoxyethylidene ortho ester, 1-ethoxyethylidine ortho ester, 1,2-dimethoxyethylidene ortho ester, $\alpha$-methoxybenzylidene ortho ester, 1-(N,N-dimethylamino)ethylidene derivative, $\alpha$-(N,N'-dimethylamino)benzylidene derivative, 2-oxacyclopentylidene ortho ester, di-t-butylsilylene group (DTBS), 1,3-(1,1,3,3-tetraisopropyldisiloxanylidene) derivative (TIPDS), tetra-t-butoxydisiloxane-1,3-diylidene derivative (TBDS), cyclic carbonates, cyclic boronates, ethyl boronate, and phenyl boronate.

One of ordinary skill in the art will appreciate that the choice of hydroxyl and amine protecting groups can be such that these groups are removed at the same time (e.g., when both protecting groups are acid labile or base labile). Alternatively, such groups can be removed in a step-wise fashion (e.g., when one protecting group is removed first by one set of removal conditions and the other protecting group is removed second by a different set of removal conditions). Such methods are readily understood by one of ordinary skill in the art.

In certain embodiments, the present invention provides a compound of any of formulae **II-a, II-b, II-c,** and **II-d:**

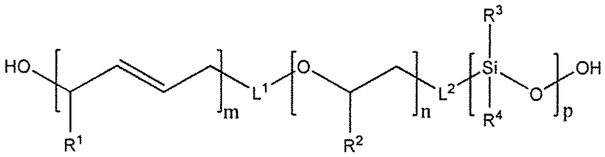

**II-a**

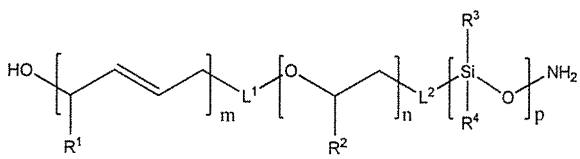

**II-b**

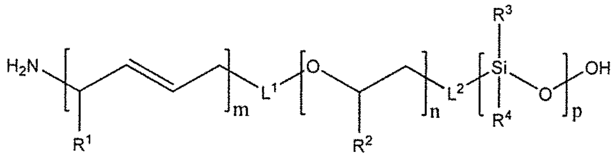

**II-c**

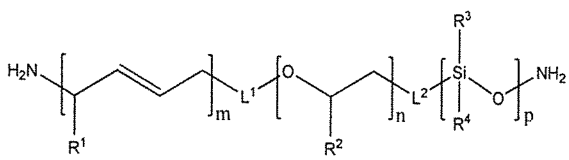

**II-d**

wherein each of m, n, p, $L^1$, $L^2$, $R^1$, $R^2$, $R^3$, and $R^4$ is as defined and described herein.

Exemplary triblock copolymers include:

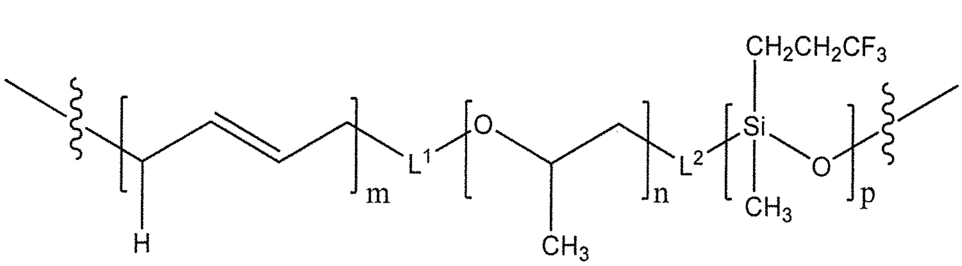

and

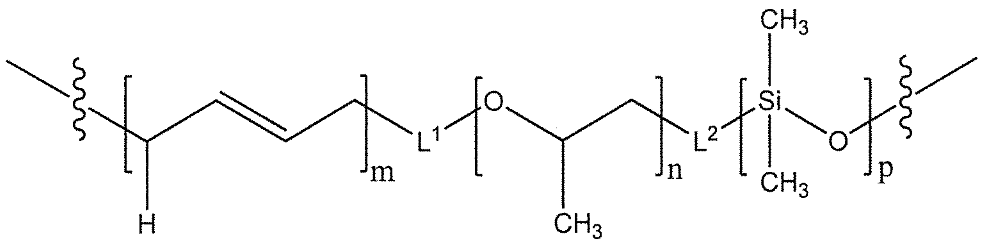

wherein each of $L^1$, $L^2$, m, n, and p is as defined and described herein.

Also described are a polymer foam, comprising:

(a) one or more triblock copolymers of formula I:

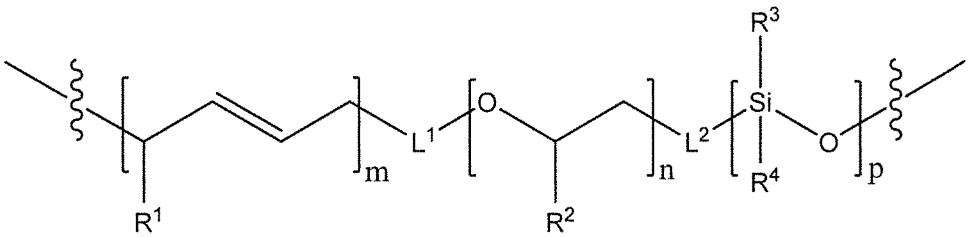

**I**

wherein each of m, n, p, $L^1$, $L^2$, $R^1$, $R^2$, $R^3$, and $R^4$ is as defined and described herein; and
(b) wherein the copolymers are chemically interspersed (bound) between urethane and/or urea linkages (i.e., at the bond designated with 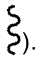).

Also described is a polyurethane/urea foam comprising a soft segment triblock copolymer of formula **I.**

Also described is a viscoelastic biostable foam, comprising:

(a) one or more triblock copolymers of formula **I:**

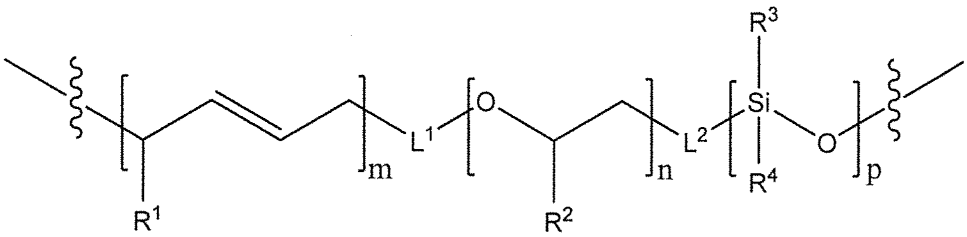

**I**

wherein each of m, n, p, $L^1$, $L^2$, $R^1$, $R^2$, $R^3$, and $R^4$ is as defined and described herein; and
(b) wherein the copolymers are chemically interspersed (bound) between urethane and/or urea linkages (i.e., at the bond designated with 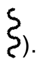).

It has been surprisingly found that polyurethanes and/or polyureas comprising a triblock copolymer are stable to gastric fluid. Such polyurethanes and polyureas prepared using triblock copolymers of the present invention are viscoelastic and stable to gastric fluid. In some embodiments, a provided viscoelastic material is a foam.

In certain examples, a provided biostable foam is stable to gastric fluid. In some embodiments, a provided biostable foam is stable to gastric fluid for at least one year. In some embodiments, a provided biostable foam is stable to gastric fluid for at least 3 months, for at least 4 months, for at least 5 months, for at least 6 months, for at least 7 months, for at least 8 months, for at least 9 months, for at least 10 months, for at least 11 months, or for at least one year. Methods for determining stability of a provided biostable foam are known in the art utilizing simulated gastric fluid and include those described in detail in the Exemplification, *infra.*

In some examples, a provided viscoelastic foam, comprising a triblock copolymer of the present invention, is characterized in that the foam takes up less than about 30% by weight of water at equilibrium. In certain embodiments, a provided viscoelastic foam takes up less than about 5%, less than about 10%, less than about 15%, less than about 20%, less than about 25%, or less than about 30% by weight of water at equilibrium. One of ordinary skill in the art will appreciate that such chemical stability (i.e., in gastric fluid and therefore at very low pH) and hyrophobicity (i.e., water uptake of less than about 30% by weight) are characterisitics that differ dramatically from known siloxane polymers that are utilized in, e.g., the manufacture of contact lenses. For example, siloxane polymer that are utilized in, e.g., the manufacture of contact lenses require a water uptake of 50-120%.

It was suprisingly found that a provided foam has a high elongation capacity and the ability to recover very slowly following elongation. Indeed, it was found that a provided viscoelastic foam has an elongation capacity of about 200-1200%. In some embodiments, a provided viscoelastic foam has an elongation capacity of about 500%.

In some examples, a provided viscoelastic foam has a tensile strength of about 0.1 to about 1.0 MPa. In certain embodiments, a provided viscoelastic foam has a tensile strength of about 0.25 to about 0.5 MPa.

In some examples, a provided viscoelastic foam has a Young's Modulus of about 0.05 to about 1.0 MPa. In certain embodiments, a provided viscoelastic foam has a Young's Modulus of about 0.05 to about 0.5 MPa.

One of ordinary skill in the art will appreciate that, depending upon the physical characteristics required for a particular use of a provided foam, a foam of varying densities can be prepared. For example, a valve having a thinner wall would require a foam having a higher density than a similar valve having a thicker wall in order to result in each valve having a similar physical characteristic (e.g., tensile strength, and the like). Thus, in certain embodiments, a provided viscoelastic foam has a density of 0.1 to 1.5 g/$cm^3$. In certain embodiments, a provided viscoelastic foam has a density of 0.3 to 1.2 g/$cm^3$. In certain embodiments, a provided viscoelastic foam has a density of 0.8 to 0.9 g/$cm^3$. In some embodiments, a provided viscoelastic foam has a density of 0.5 to 0.6 g/$cm^3$.

## EXEMPLIFICATION

### Example 1 - Synthesis of a hydroxyl terminated, triblock copolymer based on polybutadiene, polyalkylether and poly(trifluoromethyl siloxane)

Step 1 - synthesis of a fluorosiloxane based triblock copolymer pre-soft-segment:

This is a 2 stage process. In the first stage silanol terminated poly(trifluoropropyl methyl siloxane) is converted into its dihydride derivative. In the next stage, this dihydride derivative is reacted with the allyl terminated poly(propylene glycol).

The synthetic procedure is as follows:

Stage 1:

To a 4 neck separable flask fitted with mechanical stirrer, was added 40 g of Silanol terminated poly(trifluoropropyl methylsiloxane) (FMS-9922 from Gelest Inc.) and this was mixed with 50ml of toluene and fitted with a continuous flush of Nitrogen. To the reaction mixture 7.57 g of dimethyl chlorosilane (DMCS, from Sigma Aldrich) was added slowly over about 20 minutes keeping the temperature of the mixture constant at 30°C. With each addition of dimethyl chlorosilane, the mixture became hazy but cleared in a short period of time. Once the addition of dimethyl chlorosilane was complete, the mixture was heated to 90 °C for 3 hours. The reaction was then washed with excess water several times to reduce the acidity of the mixture. The resulting mixture was dried over silica gel, filtered and vacuumed to remove solvent and traces of water at 65 °C overnight. A clear fluid was then obtained with a very strong Si-H band in infra red spectroscopy (IR) at 2130 $cm^{-1}$, which confirms the reaction. GPC analysis showed the molecular weight to be 1200 g/mol.

Stage 2:

To 90 ml of reagent grade toluene in a 4 neck separable flask fitted with mechanical stirrer, 46.67g of allyl terminated poly(propylene glycol) (MW=700g/mol, Jiangsu GPRO Group Co.) was added and then heated to reflux. Then 40 g of hydride terminated FMS-9922 was dissolved in 50 mL of reagent grade toluene and the temperature raised to around 90°C. To the reaction mixture 2 drops of hexachloroplatinic (IV) acid (0.01 M $H_2PtCl_6$ from Sigma) solution in isopropanol (by Merck) was then added. After this catalyst solution had been added, the mixture was refluxed for 1 hour and the solvent distilled off in order to get the final product. The reaction was followed by H-NMR and gel permeation chromatography (GPC) confirmed the final molecular weight to be 2700 g/mol.

Step 1

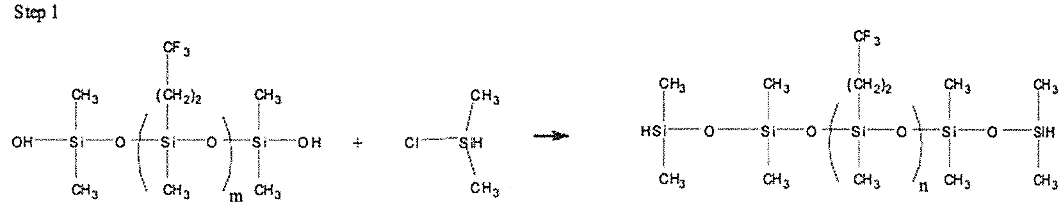

Step 2

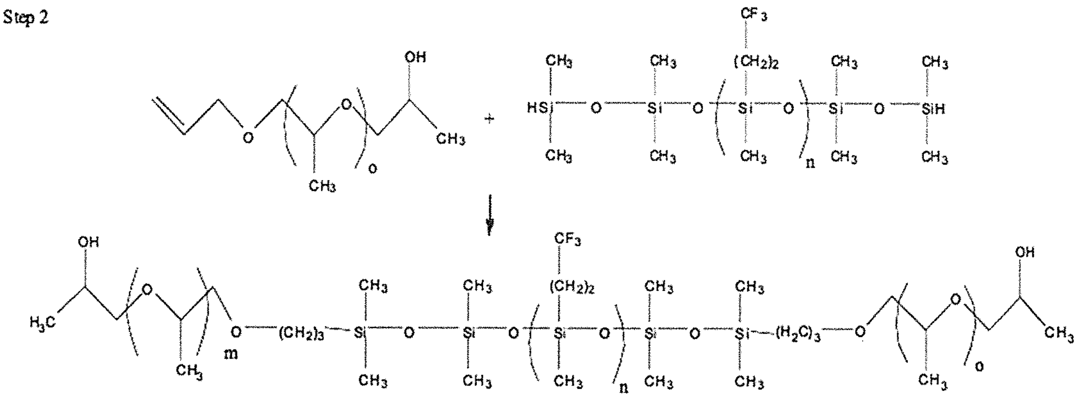

Table 1. Resulting polymer block ratios

| Stoiciometric ratios for reaction product: | | | |
|---|---|---|---|
| Polymer block | PO | F-SiO | PO |
| | m | n | o |
| Ratio | 11 | 9.7 | 11 |

**Example 2 - Preparation of polyurethane foam from the triblock copolymer of example 1:**

The process for preparing the foam was a two-step procedure:

Step 1) First, a mixture was made with 0.041 g of DABCO LV-33 (Airproducts), 0.10 g of Zinc neodecanoate (Bicat Zn from Shepherd chemicals), 0.467g of diethanol amine (DEOA, from Sigma), 5.0 g of synthesized block copolymer from Example 1, 0.250 g water and 0.05 g of surfactant (Silsurf C-208 from Siltech Corp.) in a plastic flat bottomed container. This is then thoroughly mixed for 30 sec until a homogenous mixture was obtained.

Step 2) To the above mixture, 15 g of a methylene diphenyl isocyanate (MDI) based polybutadiene pre-polymer (Krasol NN3a from Sartomer) was added. This was then thoroughly mixed by a mechanical stirrer for about 30 seconds. The material was then molded and cured at 70 °C for 2.5 hours and post cured at 50 °C for another 3 hours.

**Example 3 - Preparation of a thermoplastic polyurethane from the triblock copolymer of example 1:**

To 30g of a methylene diphenyl isocyanate (MDI) based polybutadiene pre-polymer (Krasol NN3a from Sartomer) was added 50 g of THF. This was mixed for about 5 min to dissolve the pre-polymer and was then transferred to a four neck separable flask. To this was added 0.2 g of zinc neodecanoate (BiCat Zn from Shepherd Chemicals) and 9.5 g of the triblock copolymer synthesized as described above in Example 1. A mechanical agitator was attached and the reactor was fitted with a nitrogen purge. The mixture was stirred for 24 h and the THF was then removed under vacuum. The resulting viscous polymer was then poured into a Teflon dish and the remaining solvent was removed under vacuum for 24 h at 50 °C.

The final product has the formula:

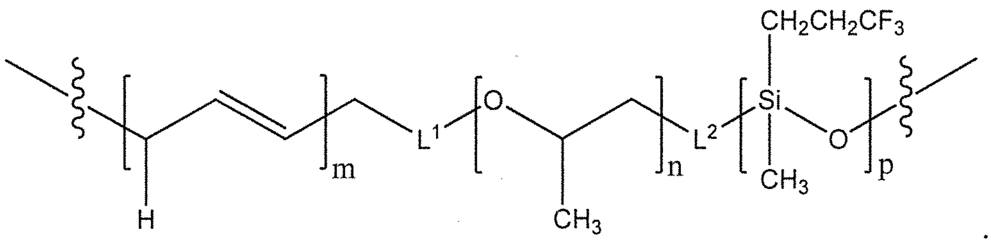

**Example 4 - Synthesis of a triblock copolymer based on polybutadiene, polyalkylether and poly(dimethyl siloxane)**

Step 1 - Synthesis of a dimethylsiloxane based triblock copolymer pre-soft-segment:

To 130 mL of reagent grade toluene in a separable flask fitted with a mechanical stirrer, was added 64 g of allyl terminated poly(propylene glycol) (MW=700g/mol, Jiangsu GPRO Co.) and both were mixed and heated to reflux. Then 40 g of hydride terminated poly(dimethyl siloxane) (Silmer H Di 10 by Siltech Corp.) was dissolved in 50 mL reagent grade toluene and the temperature raised to around 90 °C. To this reaction mixture 2 drops of hexachloroplatinic (IV) acid (0.01M $H_2PtCl_6$ from Sigma) solution in isopropanol was added. After this catalyst solution was added, the mixture was refluxed for 1 hour and then the solvent was distilled off in order to get the final product. The reaction was followed with H-NMR and gel permeation chromatography (GPC) confirmed the final molecular weight of the product to be 2300g/mol.

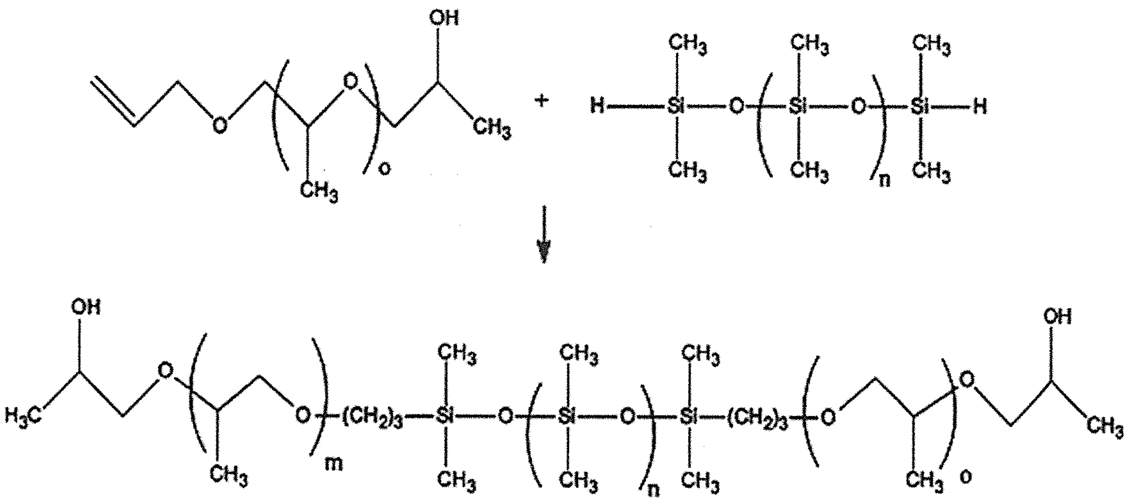

Table 2. Polymer block ratios

| Stoiciometric ratios for reaction product: | | | |
|---|---|---|---|
| Polymer block | PO | SiO | PO |
| | m | n | o |
| Ratio | 11 | 11 | 11 |

**Example 5 Preparation of a crosslinked water blown foam from the triblock copolymer of example 4:**

The process for preparing the foam was a two-step procedure:

Step 1) First, a mixture was made with 0.041 g of DABCO LV-33 (Airproducts), 0.10 g of zinc neodecanoate (Bicat Zn from Shepherd chemicals), 0.467 g of diethanolamine (DEOA, from Sigma), 5.0 g of triblock copolymer synthesised as described above in example 4, 0.250 g water and 0.05 g of surfactant (Silsurf C-208 from Siltech Corp.) in a plastic flat bottomed container. This is then thoroughly mixed for 30 sec until a homogenous mixture was obtained.

Step 2) To the above mixture, 15 g of a methylene diphenyl isocyanate (MDI) based polybutadiene pre-polymer (Krasol NN3a from Sartomer) was added. This was then thoroughly mixed by a mechanical stirrer for about 30 seconds. The material was then molded and cured at 70 °C for 2.5 h and post cured at 50 °C for another 3 h.

**Example 6 - Preparation of a thermoplastic polyurethane from the triblock copolymer of example 4**

To 30 g of a methylene diphenyl isocyanate (MDI) based polybutadiene pre-polymer (Krasol NN3a from Sartomer) was added 50 g of THF. This was mixed for about 5 min to dissolve the pre-polymer and was then transferred to a four neck separable flask. To this was added 0.2g of zinc neodecanoate (BiCat Zn from Shepherd Chemicals) and 9.5 g of the triblock copolymer synthesised as described above in example 4. A mechanical agitator was attached and the reactor was fitted with a nitrogen purge. The mixture was stirred for 24 h and the THF was then removed under vacuum. The resulting viscous polymer was then poured into a Teflon dish and the remaining solvent was removed under vacuum for 24 h at 50 °C.

The final product has the formula:

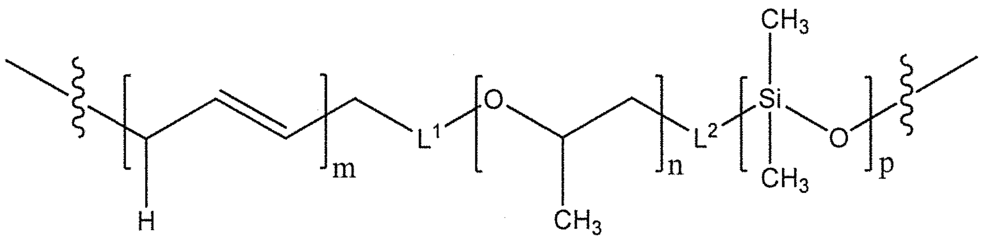

**Example 7 - Use**

Devices for use in the gastrointestinal system have historically not been made from specifically designed materials. Off the shelf materials used for application in the corrosive environment of the stomach have limited biostability and generally lose their functionality after a short time.

In certain embodiments, the foam of the invention can be used for production of a valve of the type described in our US 2007/0198048. In certain embodiments, the foam of the invention can be used for production of a valve of the type described in our US 2010/0137998. The valve has an open position and a closed position. The valve will have a proximal end and a distal end. The valve material can open from the proximal direction when the action of swallowing (liquid or solid) stretches an oriface by between 100% and 3000% in circumference. The open orifice optionally closes non-elastically over a prolonged period of time, thus mimicing the body's natural response. The duration taken to close may be between 2 and 15 sec. The material can stretch to between 100% - 300% from the distal direction when gas, liquid or solids exceeds a pre-determined force of between 25cm$H_2O$ and 60cm$H_2O$. In some embodiments, the material absorbs less than 15% of its own mass of water at equilibrium. In some embodiments, the material loses (leaches) less than 3% of it's own mass at equilibrium in water or alcohol. In some embodiments, the material loses less than 10% of its tensile strength when immersed in a simulated gastric fluid at pH 1.2 for 30 days. In some embodiments, the valve material loses less than 25% of its % elongation when immersed in a simulated gastric fluid at pH 1.2 for 30 days.

**Example 8 - Use**

The thermoplastic material may be applied to a medical device as a coating or additionally may be extruded into a specific shape. A solution of the thermoplastic polyurethane may be applied to a stent or other device, which is held on a PTFE mandrel. A continuous coating will be formed through the evaporation of the carrier solvent. This will in turn provide a protective coating to a stent ot other medical device.

By evaporation of the solvent used in the production of the thermoplastic polyurethane a solid polymer suitable for extrusion can be formed. For example the polymer produced in example 2 may be extruded at 190°C with 5Kg of force resulting in a Melt Flow Index (ISO 1133) of 0.475g/10mins. Such an extrusion could be used to build a catheter or other tubular device.

**Example 9 - Valve functional testing**

The healthy lower esophageal sphincter (LES) remains closed until an individual induces relaxation of the muscle by swallowing and thus allowing food to pass in the antegrade direction. Additionally when an individual belches or vomits they generate enough pressure in the stomach in the retrograde direction to overcome the valve. An anti-reflux

valve must enable this functionality when placed in the body, thus a simple functional test is carried out to asses performance.

It has been reported that post fundoplication patients have yield pressures between 22 - 45 mmHg and that most of the patients with gastric yield pressure above 40 mmHg experienced problems belching. *See* Yield pressure, anatomy of the cardia and gastro-oesophageal reflux. Ismail, J. Bancewicz, J. Barow British Journal of Surgery. Vol: 82, 1995, pages: 943-947. Thus, in order to facilitate belching but prevent reflux, an absolute upper GYP value of 40 mmHg (550 $mmH_2O$) is reasonable. It was also reported that patients with visible esophagitis all have gastric yield pressure values under 15 mmHg, therefore, there is good reason to selectively target a minimum gastric yield pressure value that exceeds 15 mmHg. *See* Id. An appropriate minimum gastric yield pressure value would be 15mmHg + 25% margin of error thus resulting in a minimum effective valve yield pressure value of 18.75 mmHg or 255 $mmH_2O$.

The test apparatus consists of a 1m high vertical tube to which is connected a peristaltic pump and a fitting that is designed to house the valve to be tested.

The valve to be tested is placed in a water bath at 37 °C for 30 minutes to allow its temperature to equilibrate. Once the temperature of the valve has equilibrated it is then installed into the housing such that the distal closed end of the valve faces the inside of the test apparatus. The pump is then switched on at a rate of 800 ml/min to begin filling the vertical tube. The rising column of water exerts a pressure that forces the valve shut initially. As the pressure in the column rises the valve reaches a point where it everts and allows the water to flow through. This point, known as the yield pressure, is then recorded and the test repeated four times.

**Example 10 -Rationale for accelerated aging of material**

***Clinical Condition being simulated***

The lower oesophagus of a normal patient can be exposed to the acidic contents of the stomach periodically without any adverse side effects. However, patients with gastro esophageal reflux disease experience damage to the mucosa of the lower oesophagus due to increased exposure to the gastric contents. Exposure of the lower oesophagus to acidic gastric contents is routinely measured in the clinic using dedicated pH measurement equipment. A typical procedure involves measuring pH over a 24-hour period. The levels of acid exposure in pathological reflux disease patients is summarised in Table 3 from six clinical references. *See* DeMeester TR, Johnson LF, Joseph GJ, et al. Patterns of Gastroesophageal Reflux in Health and Disease Ann. Surg. Oct 1976 459-469; Pandolfino JE, Richter JE, Ours T, et al. Ambulatory Esophageal pH Monitoring Using a Wireless System Am. J. Gastro 2003; 98:4; Mahmood Z, McMahon BP, Arfin Q, et al. Results of endoscopic gastroplasty for gastroesophageal reflux disease: a one year prospective follow-up Gut 2003; 52:34-9; Park PO, Kjellin T, Appeyard MN, et al. Results of endoscopic gastroplasty suturing for treatment of GERD: a multicentre trial Gastrointest endosc 2001; 53:AB115; Filipi CJ, Lehman GA, Rothstein RI, et al. Transoral flexible endoscopic suturing for treatment of GERD: a multicenter trial Gastrointest endosc 2001; 53 416-22; and Arts J, Slootmaekers S Sifrim D, et al. Endoluminal gastroplication (Endocinch) in GERD patient's refractory to PPI therapy Gastroenterology 2002; 122:A47.

Table 3. Summary of acid exposure in patients with reflux disease

| **Investigator** | **Number of patients** | **Details** | **% 24h <pH4** |
|---|---|---|---|
| DeMeester | 54 | Combined refluxers | 13.5 |
| Pandolfino | 41 | Gerd | 6.5 |
| Mahmood | 21 | Gerd | 11.11 |
| Park | 142 | Gerd | 8.5 |
| Filipi | 64 | Gerd | 9.6 |
| Arts | 20 | Gerd | 17 |
| **Average** | | | 11.035 |

Key Clinical Parameters

Considering that the lower oesophagus is exposed to the acidic pH exposure time for an average of 11 % of the measurement period, an accelerated aging methodology can easily be conceived. Constant exposure of a test material to the gastric contents (or USP Simulated Gastric Fluid - Reference USP Pharmacopeia) would represent an almost 10-fold increase in the rate of aging. Thus the time required to simulate one year of exposure of the lower

oesophagus to the gastric contents is described by equation 1.

$$\left(\frac{11.035}{100}\right) \times 365\ days = 40.28\ days \qquad \textit{Equation 1}$$

***Clinical Rationale***

Immersion of test specimens in USP Simulated gastric fluid for 40.28 days at 37 °C will approximate one year's exposure of the lower oesophagus to acidic gastric contents in a GERD patient's scenario as illustrated by Table 4.

Table 4. Correlation of simulated and realtime gastric fluid exposure in a GERD patient.

| Simulated Exposure | Real Time |
|---|---|
| 1 year | 40.28 days |
| 2 years | 80.56 days |
| 3 years | 120.84 days |

Results of accelerated stability of a valve prepared from a viscoelastic foam of the present invention as described in example 5 are depicted in Figure 104. The valve is in this case of the type illustrated and described with reference to Figures 59 to 65.

The performance of these valves was determined by measurement of the hydrostatic yield pressure or eversion pressure. The results of mass uptake testing of dogbone shaped coupons of the material described in example 5 are shown in Figure 105. Results of accelerated stability of dogbone shaped coupons prepared from a viscoelastic foam of the present invention as described in example 2 are depicted in Figure 106. The performance of these samples was determined using tensile testing.

**Group 2**

Use of polyethers as soft segments in polyurethane foams is know to result in soft elastic and viscoelastic materials due to the dynamic reinforcing effect of hydrogen bonding. Conversely, use of non-hydrogen bonding hydrophobic soft segments results in harder, less elastic material. Blending of such hydrophobic and hydrophilic homopolymer soft segments as shown in Figure 85 via urethane/urea linkages is known in the art to achieve mechanical properties appropriate to specific applications.

Acid catalysed hydrolytic degradation occurs at urethane linkages within polyurethane materials. These urethane/urea linkages are therefore the 'weak-links' of the polyurethane material. It follows that the intrinsic hydrophilicity of the polyurethane material will affect the rate of hydrolysis through modulation of water uptake. Thus, such materials are incompatible with use in a gastric environment (i.e., a highly acidic aqueous environment).

Also described isa multiblock copolymer that is biomimetic and hydrolytically stable in a gastric environment. Such multiblock copolymers are of formula I:

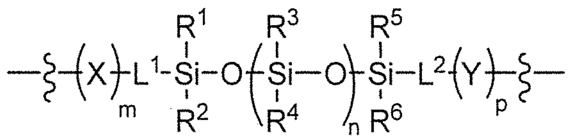

**I**

wherein:

each

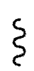

represents a point of attachment to a urethane or urea linkage;

each of X and Y is independently a polymer or co-polymer chain formed from one or more of a polyether, a polyester, a polycarbonate, or a fluoropolymer;

each of $R^1$, $R^2$, $R^3$, $R^4$, $R^5$ and $R^6$ is independently selected from one or more of R, OR, $-CO_2R$, a fluorinated hydrocarbon, a polyether, a polyester or a fluoropolymer;

each R is independently hydrogen, an optionally substituted $C_{1-20}$ aliphatic group, or an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulfur;

each of m n and p is independently 2 to 100; and

each of $L^1$ and $L^2$ is independently a bivalent $C_{1-20}$ hydrocarbon chain wherein 1-4 methylene units of the hydrocarbon chain are optionally and independently replaced by -O-, -S-, -N(R)-, - C(O)-, -C(O)N(R)-, -N(R)C(O)-, $-SO_2-$, $-SO_2N(R)-$, $-N(R)SO_2-$, -OC(O)-, -C(O)O-, or a bivalent cycloalkylene, arylene, heterocyclene, or heteroarylene, provided that neither of $L^1$ nor $L^2$ comprises a urea or urethane moiety.

***2. Definitions:***

Compounds include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

As described herein, compounds may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted." In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned by are preferably those that result in the formation of stable or chemically feasible compounds. The term "stable", as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40°C or less, in the absence of moisture or other chemically reactive conditions, for at least a week.

The term "aliphatic" or "aliphatic group", as used herein, denotes a hydrocarbon moiety that may be straight-chain (i.e., unbranched), branched, or cyclic (including fused, bridging, and spirofused polycyclic) and may be completely saturated or may contain one or more units of unsaturation, but which is not aromatic. Unless otherwise specified, aliphatic groups contain 1-20 carbon atoms. In some embodiments, aliphatic groups contain 1-10 carbon atoms. In other embodiments, aliphatic groups contain 1-8 carbon atoms. In still other embodiments, aliphatic groups contain 1-6 carbon atoms, and in yet other embodiments aliphatic groups contain 1-4 carbon atoms. Suitable aliphatic groups include, but are not limited to, linear or branched, alkyl, alkenyl, and alkynyl groups, and hybrids thereof such as (cycloalkyl)alkyl, (cycloalkenyl)alkyl or (cycloalkyl)alkenyl.

The term "lower alkyl" refers to a $C_{1-4}$ straight or branched alkyl group. Exemplary lower alkyl groups are methyl, ethyl, propyl, isopropyl, butyl, isobutyl, and tert-butyl.

The term "lower haloalkyl" refers to a $C_{1-4}$ straight or branched alkyl group that is substituted with one or more halogen atoms.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or $NR^+$ (as in N-substituted pyrrolidinyl)).

The term "unsaturated", as used herein, means that a moiety has one or more units of unsaturation.

As used herein, the term "bivalent $C_{1-8}$ [or $C_{1-6}$] saturated or unsaturated, straight or branched, hydrocarbon chain", refers to bivalent alkylene, alkenylene, and alkynylene chains that are straight or branched as defined herein.

The term "alkylene" refers to a bivalent alkyl group. An "alkylene chain" is a polymethylene group, i.e., $-(CH_2)_n-$, wherein n is a positive integer, preferably from 1 to 6, from 1 to 4, from 1 to 3, from 1 to 2, or from 2 to 3. A substituted

alkylene chain is a polymethylene group in which one or more methylene hydrogen atoms are replaced with a substituent. Suitable substituents include those described below for a substituted aliphatic group.

The term "alkenylene" refers to a bivalent alkenyl group. A substituted alkenylene chain is a polymethylene group containing at least one double bond in which one or more hydrogen atoms are replaced with a substituent. Suitable substituents include those described below for a substituted aliphatic group.

The term "halogen" means F, Cl, Br, or I.

The term "aryl" used alone or as part of a larger moiety as in "aralkyl", "aralkoxy", or "aryloxyalkyl", refers to monocyclic or bicyclic ring systems having a total of five to fourteen ring members, wherein at least one ring in the system is aromatic and wherein each ring in the system contains 3 to 7 ring members. The term "aryl" may be used interchangeably with the term "aryl ring".

As described herein, compounds may contain "optionally substituted" moieties. In general, the term "substituted", whether preceded by the term "optionally" or not, means that one or more hydrogens of the designated moiety are replaced with a suitable substituent. Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group are independently halogen; $-(CH_2)_{0-4}R°$: $-(CH_2)_{0-4}OR°$; $-O-(CH_2)_{0-4}C(O)OR°$; $-(CH_2)_{0-4}CH(OR°)_2$; $-(CH_2)_{0-4}SR°$; $-(CH_2)_{0-4}Ph$, which may be substituted with R°; $-(CH_2)_{0-4}O(CH_2)_{0-1}Ph$ which may be substituted with R°; -CH=CHPh, which may be substituted with R°; $-NO_2$; -CN; $-N_3$; $-(CH_2)_{0-4}N(R°)_2$; $-(CH_2)_{0-4}N(R°)C(O)R°$; -N(R°)C(S)R°; $-(CH_2)_{0-4}N(R°)C(O)NR°_2$; $-N(R°)C(S)NR°_2$; $-(CH_2)_{0-4}N(R°)C(O)OR°$; -N(R°)N(R°)C(O)R°; $-N(R°)N(R°)C(O)NR°_2$; -N(R°)N(R°)C(O)OR°; $-(CH_2)_{0-4}C(O)R°$; -C(S)R°; $-(CH_2)_{0-4}C(O)OR°$, $-(CH_2)_{0-4}C(O)SR°$; $-(CH_2)_{0-4}C(O)OSiR°_3$; $-(CH_2)_{0-4}OC(O)R°$; $-OC(O)(CH_2)_{0-4}SR-$, SC(S)SR°; $-(CH_2)_{0-4}SC(O)R°$; $-(CH_2)_{0-4}C(O)NR°_2$; $-C(S)NR°_2$; -C(S)SR°; -SC(S)SR°, $-(CH_2)_{0-4}OC(O)NR°_2$; -C(O)N(OR°)R°; -C(O)C(O)R°; $C(O)CH_2C(O)R°$; -C(NOR°)R°; $-(CH_2)_{0-4}SSR°$; $-(CH_2)_{0-4}S(O)_2R°$; $-(CH_2)_{0-4}S(O)_2OR°$; $-(CH_2)_{0-4}OS(O)_2R°$; $-S(O)_2NR°_2$; $-(CH_2)_{0-4}S(O)R°$; $-N(R°)S(O)_2NR°_2$; $-N(R°)S(O)_2R°$; -N(OR°)R°; $-C(NH)NR°_2$; $-P(O)_2R°$; $-P(O)R°_2$; $-O P(O)R°_2$; $-OP(O)(OR°)_2$; $SiR°_3$; $-(C_{1-4}$ straight or branched alkylene)O-N(R°)$_2$; or $-(C_{1-4}$ straight or branched alkylene)C(O)O-N(R°)$_2$, wherein each R° may be substituted as defined below and is independently hydrogen, $C_{1-6}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, $-(CH_2)_{0-2}R^●$, -(haloR●), $-(CH_2)_{0-2}OH$, $-(CH_2)_{0-2}OR^●$, $-(CH_2)_{0-2}CH(OR^●)_2$; -O(haloR●), -CN, $-N_3$, $-(CH_2)_{0-2}C(O)R^●$, $-(CH_2)_{0-2}C(O)OH$, $-(CH_2)_{0-2}C(O)OR^●$, $-(CH_2)_{0-2}SR^●$, $-(CH_2)_{0-2}SH$, $-(CH_2)_{0-2}NH_2$, $-(CH_2)_{0-2}NHR^●$, $-(CH_2)_{0-2}NR^●_2$, $-NO_2$, $-SiR^●_3$, $-OSiR^●_3$, -C(O)SR●, $-(C_{1-4}$ straight or branched alkylene)C(O)OR●, or -SSR● wherein each R● is unsubstituted or where preceded by "halo" is substituted only with one or more halogens, and is independently selected from $C_{1-4}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, $=NNR^*_2$, $=NNHC(O)R^*$, $=NNHC(O)OR^*$, $=NNHS(O)_2R^*$, $=NR^*$, $=NOR^*$, $-O(C(R^*_2))_{2-3}O-$, or $-S(C(R^*_2))_{2-3}S-$, wherein each independent occurrence of $R^*$ is selected from hydrogen, $C_{1-6}$ aliphatic which may be substituted as defined below, or an unsubstituted 5-6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: $-O(CR^*_2)_{2-3}O-$, wherein each independent occurrence of $R^*$ is selected from hydrogen, $C_{1-6}$ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of $R^*$ include halogen, -R●, -(haloR●), -OH, -OR●, -O(haloR●), -CN, -C(O)OH, -C(O)OR●, $-NH_2$, -NHR', $-NR^●_2$, or $-NO_2$, wherein each R● is unsubstituted or where preceded by "halo" is substituted only with one or more halogens, and is independently $C_{1-4}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur.

Suitable substituents on a substitutable nitrogen of an "optionally substituted" group include - R†, $-NR^†_2$, -C(O)R†,

$-C(O)OR^\dagger$, $-C(O)C(O)R^\dagger$, $-C(O)CH_2C(O)R^\dagger$, $-S(O)_2R^\dagger$, $-S(O)_2NR^\dagger_2$, $-C(S)NR^\dagger_2$, $-C(NH)NR^\dagger_2$, or $-N(R^\dagger)S(O)_2R^\dagger$; wherein each $R^\dagger$ is independently hydrogen, $C_{1-6}$ aliphatic which may be substituted as defined below, unsubstituted -OPh, or an unsubstituted 5-6-membered saturated, partially unsaturated, or aryl ring having 0-4 heteroatoms independently selected from nitrogen, oxygen, or sulfur, or, notwithstanding the definition above, two independent occurrences of $R^\dagger$, taken together with their intervening atom(s) form an unsubstituted 3-12-membered saturated, partially unsaturated, or aryl mono- or bicyclic ring having 0-4 heteroatoms independently selected from nitrogen, oxygen, or sulfur.

Suitable substituents on the aliphatic group of $R^\dagger$ are independently halogen, $-R^\bullet$, $-(haloR^\bullet)$, -OH, $-OR^\bullet$, $-O(haloR^\bullet)$, -CN, -C(O)OH, $-C(O)OR^\bullet$, $-NH_2$, $-NHR^\bullet$, $-NR^\bullet_2$, or $-NO_2$, wherein each $R^\bullet$ is unsubstituted or where preceded by "halo" is substituted only with one or more halogens, and is independently $C_{1-4}$ aliphatic, $-CH_2Ph$, $-O(CH_2)_{0-1}Ph$, or a 5-6-membered saturated, partially unsaturated, or aryl ring having 0-4 heteroatoms independently selected from nitrogen, oxygen, or sulfur.

*3. Description of Exemplary Embodiments:*

*A. Multiblock Copolymers*

As described generally above, one embodiment provides a triblock copolymer of formula I:

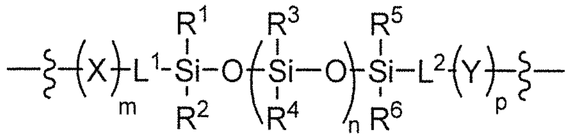

**I**

wherein the copolymers are chemically interspersed (bound) between urethane and/or urea linkages (i.e., at the bond designated with 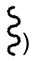) and wherein each of X, Y, m, n, p, $L^1$, $L^2$, $R^1$, $R^2$, $R^3$, $R^4$, $R^5$, and $R^6$ is as defined and described herein.

As defined generally above, the each of X and Y groups of formula I is independently a polymer or co-polymer chain formed from one or more of a polyether, a polyester, a polycarbonate, and a fluoropolymer.

Examples of polymer or co-polymer chains represented by X and/or Y include: poly(ethylene oxide), poly(difluoromethyl ethylene oxide), poly(trifluoromethyl ethylene oxide), poly(propylene oxide), poly(difluoromethyl propylene oxide), poly(propylene oxide), poly(trifluoromethyl propylene oxide), poly(butylene oxide), poly(tetramethylene ether glycol), poly(tetrahydrofuran), poly(oxymethylene), poly(ether ketone), poly(etherether ketone) and copolymers thereof, poly(dimethylsiloxane), poly(diethylsiloxane) and higher alkyl siloxanes, poly(methyl phenyl siloxane), poly(diphenyl siloxane), poly(methyl di-fluoroethyl siloxane), poly(methyl tri-fluoroethyl siloxane), poly(phenyl di-fluoroethyl siloxane), poly(phenyl trifluoroethyl siloxane) and copolymers thereof, poly(ethylene terephthalate) (PET), poly(ethylene terephthalate ionomer) (PETI), poly(ethylene naphthalate) (PEN), poly(methylene naphthalate) (PTN), poly(butylene teraphalate) (PBT), poly(butylene naphthalate) (PBN), polycarbonate. In certain embodiments, the present invention provides a pre-formed soft segment for a polyurethane / urea foam.

In some examples X is a polyether and Y is a polyether. More specifically in one case X and Y are both poly(propylene oxide).

In certain examples, m and p are each independently between 2 and 50 and n is between 2 and 20. In some embodiments, m and p are each independently between 2 and 30 and n is between 2 and 20.

As defined generally above, each of $R^1$, $R^2$, $R^3$, $R^4$, $R^5$ and $R^6$ is independently selected from one or more of R, OR, $-CO_2R$, a fluorinated hydrocarbon, a polyether, a polyester or a fluoropolymer. In some embodiments, one or more of $R^1$, $R^2$, $R^3$, $R^4$, $R^5$ and $R^6$ is $-CO_2R$. In some embodiments, one or more of $R^1$, $R^2$, $R^3$, $R^4$, $R^5$ and $R^6$ is $-CO_2R$ wherein each R is independently an optionally substituted $C_{1-6}$ aliphatic group. In certain embodiments, one or more of $R^1$, $R^2$, $R^3$, $R^4$, $R^5$ and $R^6$ is $-CO_2R$ wherein each R is independently an unsubstituted $C_{1-6}$ alkyl group. Exemplary such groups include methanoic or ethanoic acid as well as methacrylic acid and other acrylic acids.

In certain examples, one or more of $R^1$, $R^2$, $R^3$, $R^4$, $R^3$ and $R^6$ is independently R. In some embodiments, one or more of $R^1$, $R^2$, $R^3$, $R^4$, $R^5$ and $R^6$ is an optionally substituted $C_{1-6}$ aliphatic group. In certain embodiments, one or more of $R^1$, $R^2$, $R^3$, $R^4$, $R^5$ and $R^6$ is an optionally substituted $C_{1-6}$ alkyl. In other embodiments, one or more of $R^1$, $R^2$, $R^3$, $R^4$, $R^5$ and $R^6$ is an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulphur. Exemplary such $R^1$, $R^2$, $R^3$, $R^4$, $R^5$ and $R^6$ groups include methyl, ethyl, propyl, isopropyl, cyclopropyl, butyl, isobutyl, cyclobutyl, phenyl, pyridyl, morpholinyl, pyrrolidinyl,

imidazolyl, and cyclohexyl.

In certain examples, one or more of $R^1$, $R^2$, $R^3$, $R^4$, $R^5$ and $R^6$ is independently -OR. In some embodiments, one or more of $R^1$, $R^2$, $R^3$, $R^4$, $R^5$ and $R^6$ is -OR wherein R is an optionally substituted $C_{1-6}$ aliphatic group. In certain embodiments, one or more of $R^1$, $R^2$, $R^3$, $R^4$, $R^5$ and $R^6$ is -OR wherein R is $C_{1-6}$ alkyl. In other embodiments, one or more of $R^1$, $R^2$, $R^3$, $R^4$, $R^5$ and $R^6$ is-OR wherein R is an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulphur. Exemplary such $R^1$, $R^2$, $R^3$, $R^4$, $R^5$ and $R^6$ groups include -Omethyl, -Oethyl, -Opropyl, -Oisopropyl, -Ocyclopropyl, -Obutyl, -Oisobutyl, -Ocyclobutyl, -Ophenyl, -Opyridyl, -Omorpholinyl, -Opyrrolidinyl, -Oimidazolyl, and -Ocyclohexyl.

In certain examples, one or more of $R^1$, $R^2$, $R^3$, $R^4$, $R^5$ and $R^6$ is independently R wherein each R is a $C_{1-6}$ aliphatic group substituted with one or more halogens. In some embodiments, each R is $C_{1-6}$ aliphatic substituted with one, two, or three halogens. In other embodiments, each R is a perfluorinated $C_{1-6}$ aliphatic group. Examples of fluorinated hydrocarbons represented by $R^1$, $R^2$, $R^3$, $R^4$, $R^5$ and $R^6$ include mono-, di-, tri, or perfluorinated methyl, ethyl, propyl, butyl, or phenyl. In some embodiments, each of $R^1$, $R^2$, $R^3$, $R^4$, $R^5$ and $R^6$ is trifluoromethyl, trifluoroethyl, or trifluoropropyl.

In certain examples, one or more of $R^1$, $R^2$, $R^3$, $R^4$, $R^5$ and $R^6$ is independently a polyether. Examples of polyethers represented by $R^1$, $R^2$, $R^3$, $R^4$, $R^5$ and $R^6$ include poly(ethylene oxide), poly(difluoromethyl ethylene oxide), poly(trifluoromethyl ethylene oxide), poly(propylene oxide), poly(difluoromethyl propylene oxide), poly(propylene oxide), poly(trifluoromethyl propylene oxide), poly(butylene oxide), poly(tetramethylene ether glycol), poly(tetrahydrofuran), poly(oxymethylene), poly(ether ketone), poly(etherether ketone) and copolymers thereof.

In certain examples, one or more of $R^1$, $R^2$, $R^3$, $R^4$, $R^5$ and $R^6$ is independently a polyester. Examples of polyesters represented by $R^1$, $R^2$, $R^3$, $R^4$, $R^5$ and $R^6$ include poly(ethylene terephthalate) (PET), poly(ethylene terephthalate ionomer) (PETI), poly(ethylene naphthalate) (PEN), poly(methylene naphthalate) (PTN), poly(butylene teraphalate) (PBT), poly(butylene naphthalate) (PBN), polycarbonate.

In certain examples, one or more of $R^1$, $R^2$, $R^3$, $R^4$, $R^5$ and $R^6$ is independently a fluoropolymer. Examples of fluoropolymers represented by $R^1$, $R^2$, $R^3$, $R^4$, $R^5$ and $R^6$ include poly(tetrafluoroethylene), poly(methyl di-fluoroethyl siloxane), poly(methyl tri-fluoroethyl siloxane), poly(phenyl di-fluoroethyl siloxane).

In some examples, $R^1$, $R^2$, $R^3$, $R^4$, $R^5$ and $R^6$ is independently hydrogen, hydroxyl, carboxylic acids such as methanoic or ethanoic acid as well as methacrylic acid and other acrylic acids. Alkyl or aryl hydrocarbons such as methyl, ethyl, propyl, butyl, phenyl and ethers thereof. Fluorinated hydrocarbons such as mono-, di-, tri, or perfluorinated methyl, ethyl, propyl, butyl, phenyl. Polyether such as Poly(ethylene oxide), poly(difluoromethyl ethylene oxide), poly(trifluoromethyl ethylene oxide), poly(propylene oxide), poly(difluoromethyl propylene oxide), poly(propylene oxide), poly(trifluoromethyl propylene oxide), poly(butylene oxide), poly(tetramethylene ether glycol), poly(tetrahydrofuran), poly(oxymethylene), poly(ether ketone), poly(etherether ketone) and copolymers thereof. Polyesters such as Poly(ethylene terephthalate) (PET), poly(ethylene terephthalate ionomer) (PETI), poly(ethylene naphthalate) (PEN), poly(methylene naphthalate) (PTN), Poly(Butylene Teraphalate) (PBT), poly(butylene naphthalate) (PBN), polycarbonate and .fluoropolymer such as Poly(tetrafluoroethylene), poly(methyl di-fluoroethyl siloxane), poly(methyl tri-fluoroethyl siloxane), poly(phenyl di-fluoroethyl siloxane).

In some examples, m and p are between 2 and 50 and n is between 2 and 20. In certain embodiments, m and o are between 2 and 30 and n is between 2 and 20.

As defined generally above, each of $L^1$ and $L^2$ is independently a bivalent $C_{1-20}$ hydrocarbon chain wherein 1-4 methylene units of the hydrocarbon chain are optionally and independently replaced by -O-, -S-, -N(R)-, -C(O)-, -C(O)N(R)-, -N(R)C(O)-, $-SO_2-$, $-SO_2N(R)-$, $-N(R)SO_2-$, - OC(O)-, -C(O)O-, or a bivalent cycloalkylene, arylene, heterocyclene, or heteroarylene, provided that neither of $L^1$ nor $L^2$ comprises a urea or urethane moiety. In some embodiments, each of $L^1$ and $L^2$ is independently a bivalent $C_{1-20}$ alkylene chain. In certain embodiments, each of $L^1$ and $L^2$ is independently a bivalent $C_{1-10}$ alkylene chain. In certain embodiments, each of $L^1$ and $L^2$ is independently a bivalent $C_{1-6}$ alkylene chain. In certain embodiments, each of $L^1$ and $L^2$ is independently a bivalent $C_{1-4}$ alkylene chain. Exemplary such $L^1$ and $L^2$ groups include methylene, ethylene, propylene, butylene or higher bivalent alkanes.

In some examples, each of $L^1$ and $L^2$ is independently a bivalent $C_{1-20}$ alkylene chain wherein one methylene unit of the chain is replaced by -O-. In some embodiments, each of $L^1$ and $L^2$ is independently a bivalent $C_{1-10}$ alkylene chain wherein one methylene unit of the chain is replaced by -O-. In some embodiments, each of $L^1$ and $L^2$ is independently a bivalent $C_{1-6}$ alkylene chain wherein one methylene unit of the chain is replaced by -O-. In some embodiments, each of $L^1$ and $L^2$ is independently a bivalent $C_{1-4}$ alkylene chain wherein one methylene unit of the chain is replaced by -O-. Exemplary such $L^1$ and $L^2$ groups include $-OCH_2-$, $-OCH_2CH_2-$, $-OCH_2CH_2CH_2-$, $-OCH_2CH_2CH_2CH_2-$, or higher bivalent alkylene ethers.

In some examples, each of $L^1$ and $L^2$ is independently a bivalent $C_{1-20}$ alkylene chain wherein at least one methylene unit of the chain is replaced by -O- and at least one methylene unit of the chain is replaced by a bivalent arylene. In some embodiments, each of $L^1$ and $L^2$ is independently a bivalent $C_{1-10}$ alkylene chain wherein at least one

methylene unit of the chain is replaced by -O- and at least one methylene unit of the chain is replaced by a bivalent arylene. In some embodiments, each of $L^1$ and $L^2$ is independently a bivalent $C_{1-6}$ alkylene chain wherein at least one methylene unit of the chain is replaced by -O- and at least one methylene unit of the chain is replaced by a bivalent arylene. In some embodiments, each of $L^1$ and $L^2$ is independently a bivalent $C_{1-4}$ alkylene chain wherein at least one methylene unit of the chain is replaced by -O- and at least one methylene unit of the chain is replaced by a bivalent arylene. Exemplary such $L^1$ and $L^2$ groups include $-OCH_2$-phenylene-, $-OCH_2CH_2$--phenylene-, $-OCH_2CH_2$-phenylene-$CH_2$-, $-OCH_2CH_2CH_2CH_2$--phenylene-, and the like.

One of ordinary skill in the art would understand that a polyurethane results from the reaction of a diisocyanate and a hydroxyl group. Similarly, a polyurea results from the reaction of a diisocyanate and an amine. Each of these reactions is depicted below.

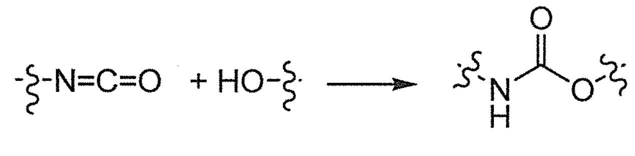

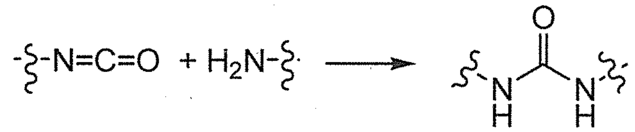

Thus, it is readily apparent that provided compounds of formula I can be functionalized with end groups suitable for forming urethane and/or urea linkages. In certain embodiments, the present invention provides a compound of formula **II**:

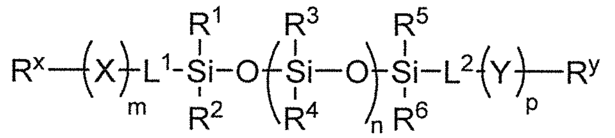

**II**

wherein:

each of $R^x$ and $R^y$ is independently -OH, $-NH_2$, a protected hydroxyl or a protected amine;

each of X and Y is independently a polymer or co-polymer chain formed from one or more of a polyether, a polyester, a polycarbonate, and a fluoropolymer;

each of $R^1$, $R^2$, $R^3$, $R^4$, $R^5$ and $R^6$ is independently selected from one or more of R, OR, $-CO_2R$, a fluorinated hydrocarbon, a polyether, a polyester or a fluoropolymer;

each R is independently hydrogen, an optionally substituted $C_{1-20}$ aliphatic group, or an optionally substituted group selected from phenyl, 8-10 membered bicyclic aryl, a 4-8 membered monocyclic saturated or partially unsaturated heterocyclic ring having 1-2 heteroatoms independently selected from nitrogen, oxygen, or sulphur, or 5-6 membered monocyclic or 8-10 membered bicyclic heteroaryl group having 1-4 heteroatoms independently selected from nitrogen, oxygen, or sulfur;

each of m n and p is independently 2 to 100; and

each of $L^1$ and $L^2$ is independently a bivalent $C_{1-20}$ hydrocarbon chain wherein 1-4 methylene units of the hydrocarbon chain are optionally and independently replaced by -O-, -S-, -N(R)-, - C(O)-, -C(O)N(R)-, -N(R)C(O)-, $-SO_2$-, $-SO_2N(R)$-, $-N(R)SO_2$-, -OC(O)-, -C(O)O-, or a bivalent cycloalkylene, arylene, heterocyclene, or heteroarylene, provided that neither of $L^1$ nor $L^2$ comprises a urea or urethane moiety.

In some embodiments, each of X, Y, m, n, p, $L^1$, $L^2$, $R^1$, $R^2$, $R^3$, $R^4$, $R^5$, and $R^6$ is as defined and described herein.

As defined generally above, each of $R^x$ and $R^y$ is independently -OH, $-NH_2$, a protected hydroxyl or a protected amine. In some embodiments, both of $R^x$ and $R^y$ are -OH. In other embodiments, both of $R^x$ and $R^y$ are $NH_2$. In some embodiments one of $R^x$ and $R^y$ is -OH and the other is $-NH_2$.

In some examples, each of $R^x$ and $R^y$ is independently a protected hydroxyl or a protected amine. Such protected

hydroxyl and protected amine groups are well known to one of skill in the art and include those described in detail in Protecting Groups in Organic Synthesis, T. W. Greene and P. G. M. Wuts, 3rd edition, John Wiley & Sons, 1999. Exemplary protected amines include methyl carbamate, ethyl carbamante, 9-fluorenylmethyl carbamate (Fmoc), 9-(2-sulfo)fluorenylmethyl carbamate, 9-(2,7-dibromo)fluoroenylmethyl carbamate, 2,7-di-t-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-t-BOC), 1,1-dimethyl-2,2,2-trichloroethyl carbamate (TCBOC), 1-methyl-1-(4-biphenylyl)ethyl carbamate (Bpoc), 1-(3,5-di-t-butylphenyl)-1-methylethyl carbamate (t-Bumeoc), 2-(2'- and 4'-pyridyl)ethyl carbamate (Pyoc), 2-(N,N-dicyclohexylcarboxamido)ethyl carbamate, t-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, N-hydroxypiperidinyl carbamate, alkyldithio carbamate, benzyl carbamate (Cbz), p-methoxybenzyl carbamate (Moz), p-nitobenzyl carbamate, p-bromobenzyl carbamate, p-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(p-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, m-chloro-p-acyloxybenzyl carbamate, p-(dihydroxyboryl)benzyl carbamate, 5-benzisoxazolylmethyl carbamate, 2-(trifluoromethyl)-6-chromonylmethyl carbamate (Tcroc), m-nitrophenyl carbamate, 3,5-dimethoxybenzyl carbamate, o-nitrobenzyl carbamate, 3,4-dimethoxy-6-nitrobenzyl carbamate, phenyl(o-nitrophenyl)methyl carbamate, phenothiazinyl-(10)-carbonyl derivative, N'-p-toluenesulfonylaminocarbonyl derivative, N'-phenylaminothiocarbonyl derivative, t-amyl carbamate, S-benzyl thiocarbamate, p-cyanobenzyl carbamate, cyclobutyl carbamate, cyclohexyl carbamate, cyclopentyl carbamate, cyclopropylmethyl carbamate, p-decyloxybenzyl carbamate, 2,2-dimethoxycarbonylvinyl carbamate, o-(N,N-dimethylcarboxamido)benzyl carbamate, 1,1-dimethyl-3-(N,N-dimethylcarboxamido)propyl carbamate, 1,1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, p-(p'-methoxyphenylazo)benzyl carbamate, 1-methylcyclobutyl carbamate, 1-methylcyclohexyl carbamate, 1-methyl-1-cyclopropylmethyl carbamate, 1-methyl-1-(3,5-dimethoxyphenyl)ethyl carbamate, 1-methyl-1-(p-phenylazophenyl)ethyl carbamate, 1-methyl-1-phenylethyl carbamate, 1-methyl-1-(4-pyridyl)ethyl carbamate, phenyl carbamate, p-(phenylazo)benzyl carbamate, 2,4,6-tri-t-butylphenyl carbamate, 4-(trimethylammonium)benzyl carbamate, 2,4,6-trimethylbenzyl carbamate, formamide, acetamide, chloroacetamide, trichloroacetamide, trifluoroacetamide, phenylacetamide, 3-phenylpropanamide, picolinamide, 3-pyridylcarboxamide, N-benzoylphenylalanyl derivative, benzamide, p-phenylbenzamide, o-nitophenylacetamide, o-nitrophenoxyacetamide, acetoacetamide, (N'-dithiobenzyloxycarbonylamino)acetamide, 3-(p-hydroxyphenyl)propanamide, 3-(o-nitrophenyl)propanamide, 2-methyl-2-(o-nitrophenoxy)propanamide, 2-methyl-2-(o-phenylazophenoxy)propanamide, 4-chlorobutanamide, 3-methyl-3-nitrobutanamide, o-nitrocinnamide, N-acetylmethionine derivative, o-nitrobenzamide, o-(benzoyloxymethyl)benzamide, 4,5-diphenyl-3-oxazolin-2-one, N-phthalimide, N-dithiasuccinimide (Dts), N-2,3-diphenylmaleimide, N-2,5-dimethylpyrrole, N-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1-substituted 3,5-dinitro-4-pyridone, N-methylamine, N-allylamine, N-[2-(trimethylsilyl)ethoxy]methylamine (SEM), N-3-acetoxypropylamine, N-(1-isopropyl-4-nitro-2-oxo-3-pyroolin-3-yl)amine, quaternary ammonium salts, N-benzylamine, N-di(4-methoxyphenyl)methylamine, N-5-dibenzosuberylamine, N-triphenylmethylamine (Tr), N-[(4-methoxyphenyl)diphenylmethyl]amine (MMTr), N-9-phenylfluorenylamine (PhF), N-2,7-dichloro-9-fluorenylmethyleneamine, N-ferrocenylmethylamino (Fcm), N-2-picolylamino N'-oxide, N-1,1-dimethylthiomethyleneamine, N-benzylideneamine, N-p-methoxybenzylideneamine, N-diphenylmethyleneamine, N-[(2-pyridyl)mesityl]methyleneamine, N-(N',N'-dimethylaminomethylene)amine, N,N'-isopropylidenediamine, N-p-nitrobenzylideneamine, N-salicylideneamine, N-5-chlorosalicylideneamine, N-(5-chloro-2-hydroxyphenyl)phenylmethyleneamine, N-cyclohexylideneamine, N-(5,5-dimethyl-3-oxo-1-cyclohexenyl)amine, N-borane derivative, N-diphenylborinic acid derivative, N-[phenyl(pentacarbonylchromium- or tungsten)carbonyl]amine, N-copper chelate, N-zinc chelate, N-nitroamine, N-nitrosoamine, amine N-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o-nitrobenzenesulfenamide (Nps), 2,4-dinitrobenzenesulfenamide, pentachlorobenzenesulfenamide, 2-nitro-4-methoxybenzenesulfenamide, triphenylmethylsulfenamide, 3-nitropyridinesulfenamide (Npys), p-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide.

Exemplary hydroxyl protecting groups include methyl, methoxylmethyl (MOM), methylthiomethyl (MTM), t-butylthiomethyl, (phenyldimethylsilyl)methoxymethyl (SMOM), benzyloxymethyl (BOM), p-methoxybenzyloxymethyl (PMBM), (4-methoxyphenoxy)methyl (p-AOM), guaiacolmethyl (GUM), t-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, t-butyl, allyl, p-chlorophenyl, p-methoxyphenyl, 2,4-dinitrophenyl, benzyl, p-methoxybenzyl, 3,4-dimethoxybenzyl, o-nitrobenzyl, p-nitrobenzyl, p-halobenzyl, 2,6-dichlorobenzyl, p-cyanobenzyl, p-phenylbenzyl, 2-picolyl, 4-picolyl, 3-methyl-2-picolyl N-oxido, diphenylmethyl, p,p'-dinitrobenzhydryl, 5-dibenzosuberyl, triphenylmethyl, α-naphthyldiphenylmethyl, p-methoxyphenyldiphenylmethyl, di(p-methoxyphenyl)phenylmethyl, tri(p-methoxyphenyl)methyl, 4-(4'-bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4''-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, t-butyldimethylsilyl (TBDMS), t-butyldiphenylsilyl (TBDPS), tribenzylsilyl, tri-p-xylylsilyl, triphenylsilyl, diphenylmethylsilyl (DPMS), t-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, p-phenylbenzoate, 2,4,6-trimethylbenzoate (mesitoate), alkyl methyl carbonate, 9-fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), alkyl isobutyl carbonate, alkyl vinyl carbonate alkyl allyl carbonate, alkyl p-nitrophenyl carbonate, alkyl benzyl carbonate, alkyl p-methoxybenzyl carbonate, alkyl 3,4-dimethoxybenzyl carbonate, alkyl o-nitrobenzyl carbonate, alkyl p-nitrobenzyl carbonate, alkyl S-benzyl thiocarbonate, 4-ethoxy-1-napththyl carbonate, methyl dithiocarbonate, 2-iodobenzoate, 4-azidobutyrate, 4-nitro-4-methylpentanoate, o-(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate, o-(methoxycarbonyl)benzoate, α-naphthoate, nitrate, alkyl N,N,N',N'-tetramethylphosphorodiamidate, alkyl N-phenylcarbamate, borate, dimethylphosphinothioyl, alkyl 2,4-dinitrophenylsulfenate, sulfate, methanesulfonate (mesylate), benzylsulfonate, and tosylate (Ts). For protecting 1,2- or 1,3-diols, the protecting groups include methylene acetal, ethylidene acetal, 1-t-butylethylidene ketal, 1-phenylethylidene ketal, (4-methoxyphenyl)ethylidene acetal, 2,2,2-trichloroethylidene acetal, acetonide, cyclopentylidene ketal, cyclohexylidene ketal, cycloheptylidene ketal, benzylidene acetal, p-methoxybenzylidene acetal, 2,4-dimethoxybenzylidene ketal, 3,4-dimethoxybenzylidene acetal, 2-nitrobenzylidene acetal, methoxymethylene acetal, ethoxymethylene acetal, dimethoxymethylene ortho ester, 1-methoxyethylidene ortho ester, 1-ethoxyethylidine ortho ester, 1,2-dimethoxyethylidene ortho ester, α-methoxybenzylidene ortho ester, 1-(N,N-dimethylamino)ethylidene derivative, α-(N,N'-dimethylamino)benzylidene derivative, 2-oxacyclopentylidene ortho ester, di-t-butylsilylene group (DTBS), 1,3-(1,1,3,3-tetraisopropyldisiloxanylidene) derivative (TIPDS), tetra-t-butoxydisiloxane-1,3-diylidene derivative (TBDS), cyclic carbonates, cyclic boronates, ethyl boronate, and phenyl boronate.

One of ordinary skill in the art will appreciate that the choice of hydroxyl and amine protecting groups can be such that these groups are removed at the same time (e.g., when both protecting groups are acid labile or base labile). Alternatively, such groups can be removed in a step-wise fashion (e.g., when one protecting group is removed first by one set of removal conditions and the other protecting group is removed second by a different set of removal conditions). Such methods are readily understood by one of ordinary skill in the art.

Also described are a compound of any of formulae **II-a, II-b, II-c,** and **II-d:**

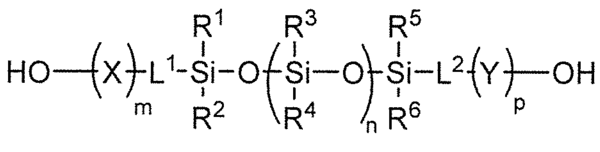

**II-a**

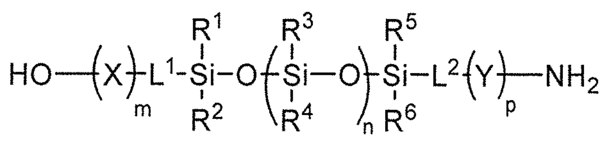

**II-b**

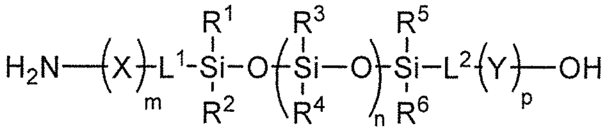

**II-c**

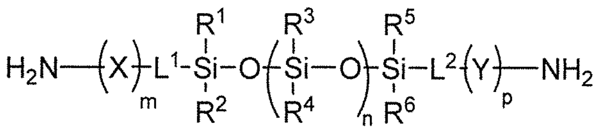

**II-d**

wherein each of X, Y, m, n, p, $L^1$, $L^2$, $R^1$, $R^2$, $R^3$, $R^4$, $R^5$, and $R^6$ is as defined and described herein.

Exemplary triblock copolymers are set forth below:

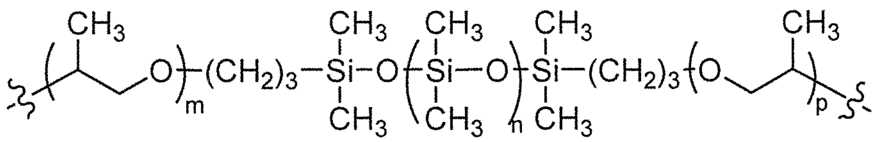

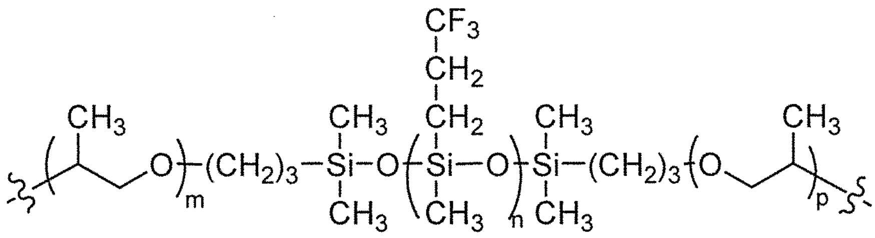

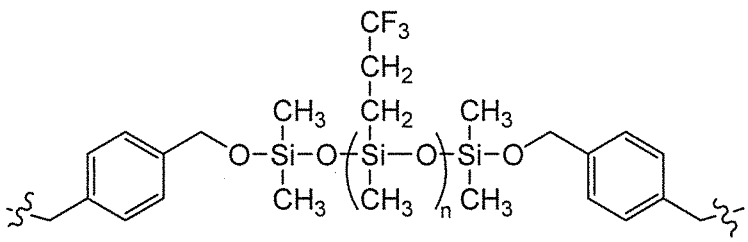

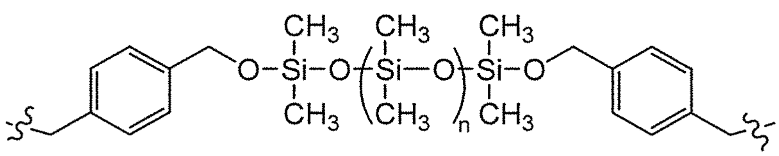

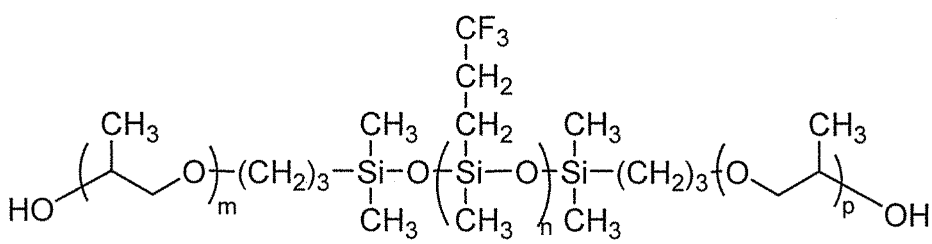

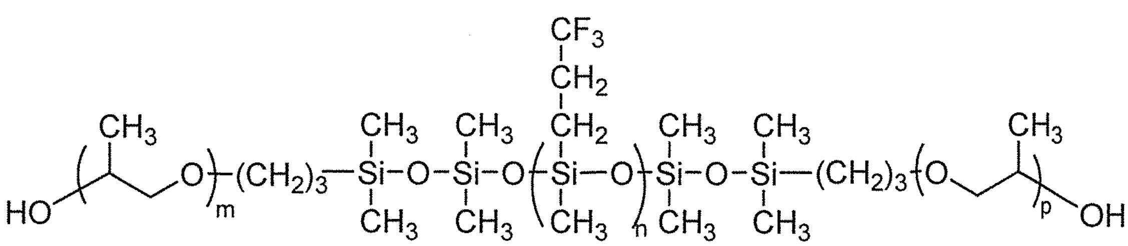

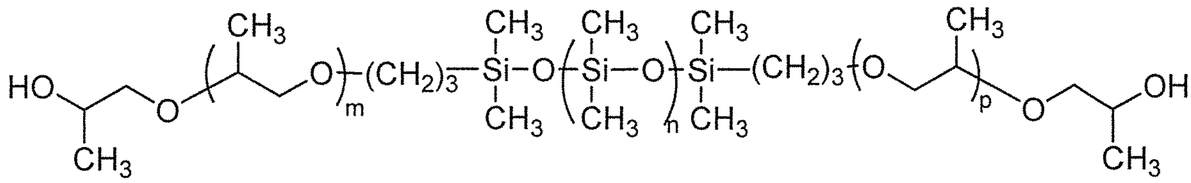

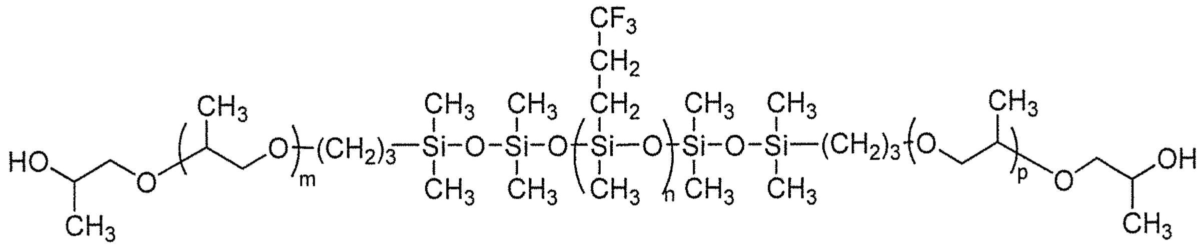

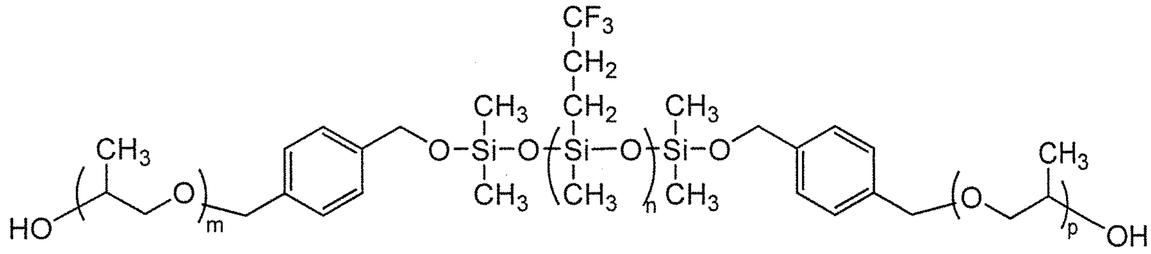

wherein each of m, n, and p is as defined and described herein.

In some casesa polymer foam. comprises:

(a) one or more triblock copolymers of formula I:

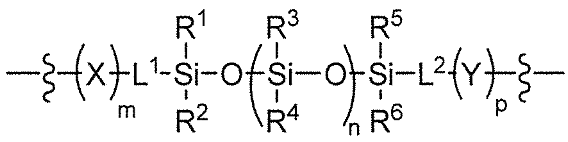

**I**

wherein each of X, Y, m, n, p, $L^1$, $L^2$, $R^1$, $R^2$, $R^3$, $R^4$, $R^5$, and $R^6$ is as defined and described herein; and

(b) wherein the copolymers are chemically interspersed (bound) between urethane and/or urea linkages (i.e., at the bond designated with 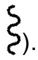).

Also described is a pre-formed soft segment of the formula I as defined above. In some casesa polyurethane/urea foam comprising a soft segment triblock copolymer of formula I is described.

Also described is a viscoelastic biostable water blown foam, comprising:

(a) one or more triblock copolymers of formula I:

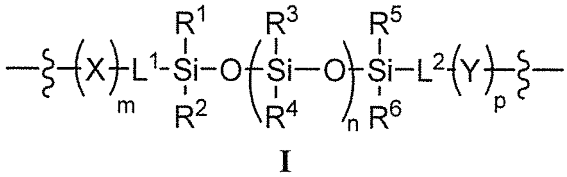

**I**

wherein each of X, Y, m, n, p, $L^1$, $L^2$, $R^1$, $R^2$, $R^3$, $R^4$, $R^5$, and $R^6$ is as defined and described herein; and

(b) wherein the copolymers are chemically interspersed (bound) between urethane and/or urea linkages (i.e., at the bond designated with 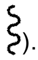).

It has been surprisingly found that polyurethanes and/or polyureas comprising a triblock copolymer are stable to gastric fluid. Such polyurethanes and polyureas prepared using triblock copolymers of the present invention are viscoelastic and stable to gastric fluid. In some embodiments, a provided viscoelastic material is a foam.

In certain examples, a provided biostable foam is stable to gastric fluid. In some embodiments, a provided biostable foam is stable to gastric fluid for at least one year. In some embodiments, a provided biostable foam is stable to gastric fluid for at least 3 months, for at least 4 months, for at least 5 months, for at least 6 months, for at least 7 months, for at least 8 months, for at least 9 months, for at least 10 months, for at least 11 months, or for at least one year. Methods for determining stability of a provided biostable foam are known in the art utilizing simulated gastric fluid and include those described in detail in the Exemplification, *infra.*

In some examples, a provided viscoelastic foam, comprising a triblock copolymer is characterized in that the foam takes up less than about 30% by weight of water at equilibrium. In certain embodiments, a provided viscoelastic foam takes up less than about 5%, less than about 10%, less than about 15%, less than about 20%, less than about 25%, or less than about 30% by weight of water at equilibrium. One of ordinary skill in the art will appreciate that such chemical stability (i.e., in gastric fluid and therefore at very low pH) and hyrophobicity (i.e., water uptake of less than about 30% by weight) are characterisitics that differ dramatically from known siloxane polymers that are utilized in, e.g., the manufacture of contact lenses. For example, siloxane polymer that are utilized in, e.g., the manufacture of contact lenses require a water uptake of 50-120%.

Also described isa viscoelastic foam comprising a triblock copolymer. It was suprisingly found that a provided foam has a high elongation capacity and the ability to recover very slowly following elongation. Indeed, it was found that a provided viscoelastic foam has an elongation capacity of about 200-1200%. In some embodiments, a provided viscoelastic foam has an elongation capacity of about 500%.

In some embodiments, a provided viscoelastic foam has a tensile strength of about 0.1 to about 1.0 MPa. In certain embodiments, a provided viscoelastic foam has a tensile strength of about 0.25 to about 0.5 MPa.

In some examples, a provided viscoelastic foam has a Young's Modulus of about 0.1 to about 0.6 MPa. In certain embodiments, a provided viscoelastic foam has a Young's Modulus of about 0.1 to about 0.5 MPa.

One of ordinary skill in the art will appreciate that, depending upon the physical characteristics required for a particular use of a provided foam, a foam of varying densities can be prepared. For example, a valve having a thinner wall would require a foam having a higher density than a similar valve having a thicker wall in order to result in each valve having a similar physical characteristic (e.g., tensile strength, and the like). Thus, in certain embodiments, a provided viscoelastic foam has a density of 0.1 to 1.5 g/cm$^3$. In certain embodiments, a provided viscoelastic foam has a density of 0.3 to 1.2 g/cm$^3$. In certain embodiments, a provided viscoelastic foam has a density of 0.8 to 0.9 g/cm$^3$. In some embodiments, a provided viscoelastic foam has a density of 0.5 to 0.6 g/cm$^3$.

Described are polyether-siloxane and polyether-fluorosiloxane polyurethane materials with a greatly reduced number of weak-links as illustrated by Figure 108 and Figure 109. This was achieved by preforming the soft segment prior to the polyurethane reaction. In the examples below a triblock copolymer based on polydimethyl siloxane and polypropylene oxide was used but it will be appreciated that other triblock copolymers such as those formed from polysiloxanes and poly(ethylene oxide), poly(difluoromethyl ethylene oxide), poly(trifluoromethyl ethylene oxide), poly(propylene oxide), poly(difluoromethyl propylene oxide), poly(propylene oxide), poly(trifluoromethyl propylene oxide), poly(butylene oxide), poly(tetramethylene ether glycol), poly(tetrahydrofuran), poly(oxymethylene), poly(ether ketone), poly(etherether ketone) and copolymers thereof, poly(dimethylsiloxane), poly(diethylsiloxane) and higher alkyl siloxanes, poly(methyl phenyl siloxane), poly(diphenyl siloxane), poly(methyl di-fluoroethyl siloxane), poly(methyl tri-fluoroethyl siloxane), poly(phenyl di-fluoroethyl siloxane), poly(phenyl tri-fluoroethyl siloxane) and copolymers thereof, poly(ethylene terephthalate) (PET), poly(ethylene terephthalate ionomer) (PETI), poly(ethylene naphthalate) (PEN), poly(methylene naphthalate) (PTN), poly(butylene teraphalate) (PBT), poly(butylene naphthalate) (PBN) and polycarbonate could be used.

Referring to Figure 108, copolymers of the form ABA, ABC and BAB were produced from homopolymers of polysiloxane and polypropylene oxide which were covalently linked using bonds less labile than urethane/urea. The molecular weight and chemical charateristics of such homopolymers were tailored to achieve a pre-soft-segment with the appropriate balance of hydrophilicity/hydrophobicity. Without wishing to be bound by any particular theory, it is believe that by using a non-urethane linked tri-block copolymer instead of the constiuent homopolymers as soft segments that the mechanical characteristics and hydrolytic stability of the resulting material is substantially improved.

Also described is a foam comprising a copolymer. as described herein Such foams offer specific advantages over solid elastomers, especially for gastrointestinal device applications. These advantages include enhanced biostability in the gastric environment, compressibility, viscoelasticity and high 'surface area to volume ratio'. The foam formulations

can mimic mechanical characteristics of the native gastrointestinal tissue.

A biostable water blown foam was prepared from heterogenous reagents.

The prior art describes polyurethane foams that are prepared by the sequential reaction of polymer chains to one another resulting in a high molecular weight solid material. In all cases the polymeric precursors described in the art are linked together by urethane/urea linkages as illustrated in Figure 107. However, each urethane/urea linkage is a possible site for degradation.

We have prepared a biostable polyurethane/urea foam with much fewer 'weak links' by using co-polymer precursors as shown in Figure 108.

Polyurethane reactions have historically been carried out in a single phase due to ease of processing. However, we have made novel materials by combining physically heterogenous reaction pre-cursors together to form a stable two-phase dispersion ('water-in-oil') which was then reacted to form a foam.

## EXEMPLIFICATION

In two specific examples X and Y are both polyethers namely poly(propylene oxide) (PPO). These were formulated into copolymers with poly(dimethylsiloxane) (PDMS) and poly(trifluoropropyl methylsiloxane) respectively in varying ratios as described by the following formulae:

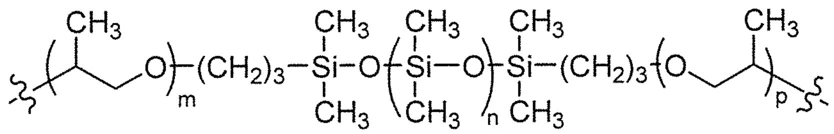

and

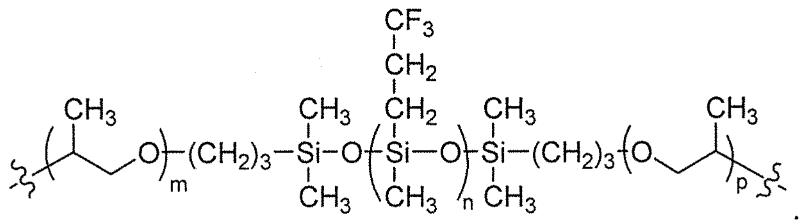

The formulations contained a number of other components including:

### Branching agent - DEOA

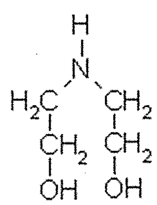

Diethanolamine (DEOA) is used as a branching agent although it is sometimes known as a crosslinking agent. The molecular weight of DEOA is 105.14 g/mol. The effect of the DEOA is to influence softness and elasticity of the end polymer.

### Gelling catalyst - Bismuth Neodecanoate (BICAT)

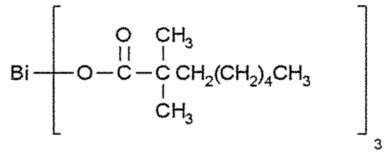

Bismuth neodecanoate is supplied as BiCat 8108M from Shepherd. It has a molecular weight of 722.75 g/mol. This catalyst is used to facilitate the complete reaction between isocyanate and hydroyl or amine functional groups.

**Blowing Catalyst -** DABCO **33-lv**

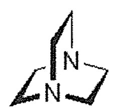

DABCO is a common blowing catalyst for reaction between NCO and $H_2O$. It has a molecular weight of 112.17 g/mol. This catalyst has the effect, in combination with $H_2O$, of manipulating the foam rise characteristics.

**Example 1**

**Synthesis of aliphatic linked fluorosiloxane based triblock copolymer pre-soft-segment:**

This is a 2 step process. In the first step silanol terminated poly(trifluoropropyl methyl siloxane) is converted into its dihydride derivative. In the next step, this dihydride derivative is reacted with the allyl terminated poly(propylene glycol).
The synthetic procedure is as follows:

Step 1:

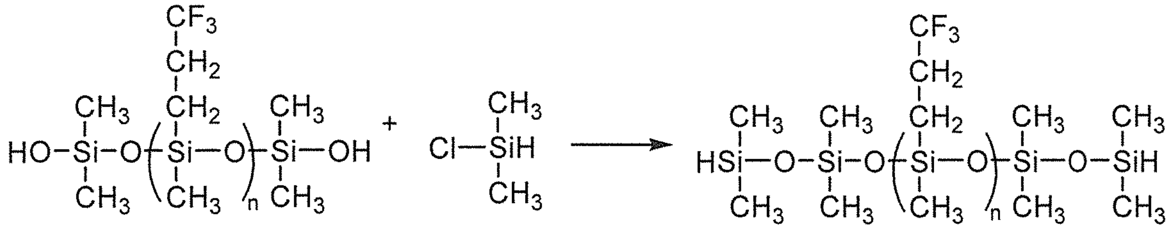

To a 4 neck separable flask fitted with mechanical stirrer, was added 40 g of Silanol terminated poly(trifluoropropyl methylsiloxane) (FMS-9922 from Gelest Inc.) and this was mixed with 50ml of toluene and fitted with a continuous flush of Nitrogen. To the reaction mixture 7.57 g of dimethyl chlorosilane (DMCS, from Sigma Aldrich) was added slowly over about 20 minutes keeping the temperature of the mixture constant at 30°C. With each addition of dimethyl chlorosilane, the mixture became hazy but cleared in a short period of time. Once the addition of dimethyl chlorosilane was complete, the mixture was heated to 90°C for 3 hours. The reaction was then washed with excess water several times to reduce the acidity of the mixture. The resulting mixture was dried over silica gel, filtered and vacuumed to remove solvent and traces of water at 65°C overnight. A clear fluid was then obtained with a very strong Si-H band in infra red spectroscopy (IR) at 2130 $cm^{-1}$ , which confirms the reaction. GPC analysis showed the molecular weight to be 1200g/mol.

Step 2:

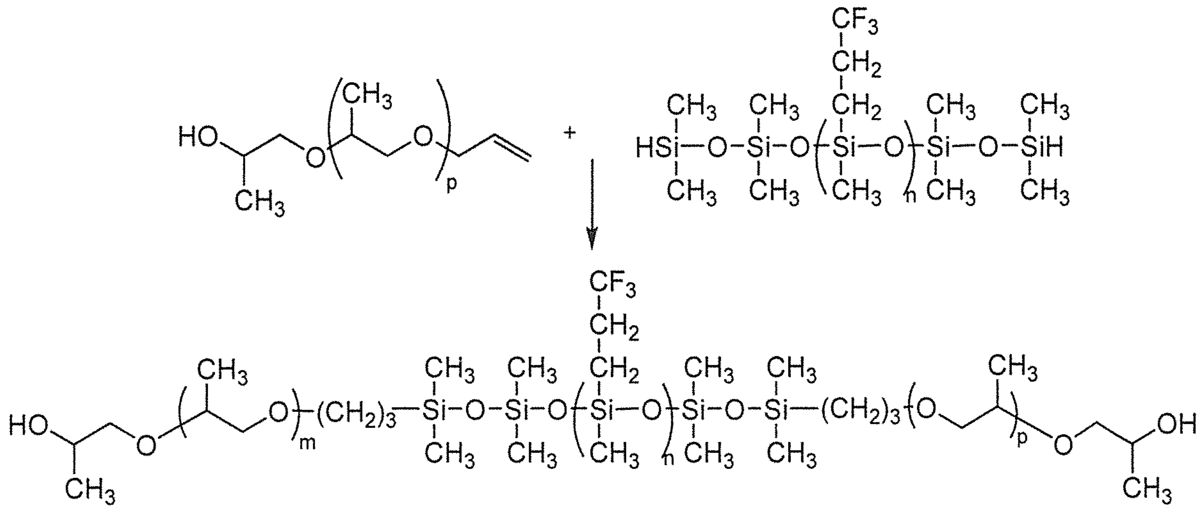

To 90 ml of reagent grade toluene in a 4 neck separable flask fitted with mechanical stirrer, 46.67g of Allyl terminated poly(propylene glycol) (MW=700g/mol, Jiangsu GPRO Group Co.) was added and then heated to reflux. Then 40g of Hydride terminated FMS-9922 was dissolved in 50ml of reagent grade toluene and the temperature raised to around 90°C. To the reaction mixture 2 drops of hexachloroplatinic(IV) acid (0.01M $H_2PtCl_6$ from Sigma) solution in isopropanol (by Merck) was then added. After this catalyst solution had been added, the mixture was refluxed for 1 hour and the solvent distilled off in order to get the final product. The reaction was followed by H-NMR and gel permeation chromatography (GPC) confirmed the final molecular weight to be 2700g/mol.

**Table 1. Resulting polymer block ratios**

| Stoiciometric ratios for reaction product: | | | |
|---|---|---|---|
| Polymer block | PO | F-SiO | PO |
| | m | n | p |
| Ratio | 11 | 9.7 | 11 |

**Example 2**

**Synthesis of aliphatic linked dimethylsiloxane based triblock copolymer pre-soft-segment:**

To 130ml of reagent grade toluene in a separable flask fitted with a mechanical stirrer, was added 64g of allyl terminated poly(propylene glycol) (MW=700g/mol, Jiangsu GPRO Co.) and both were mixed and heated to reflux. Then 40g of hydride terminated poly(dimethyl siloxane) (Silmer H Di 10 by Siltech Corp.) was dissolved in 50ml reagent grade toluene and the temperature raised to around 90°C. To this reaction mixture 2 drops of hexachloroplatinic(IV) acid (0.01M $H_2PtCl_6$ from Sigma) solution in isopropanol was added. After this catalyst solution was added, the mixture was refluxed for 1 hour and then the solvent was distilled off in order to get the final product. The reaction was followed with H-NMR and gel permeation chromatography (GPC) confirmed the final molecular weight of the product to be 2300g/mol.

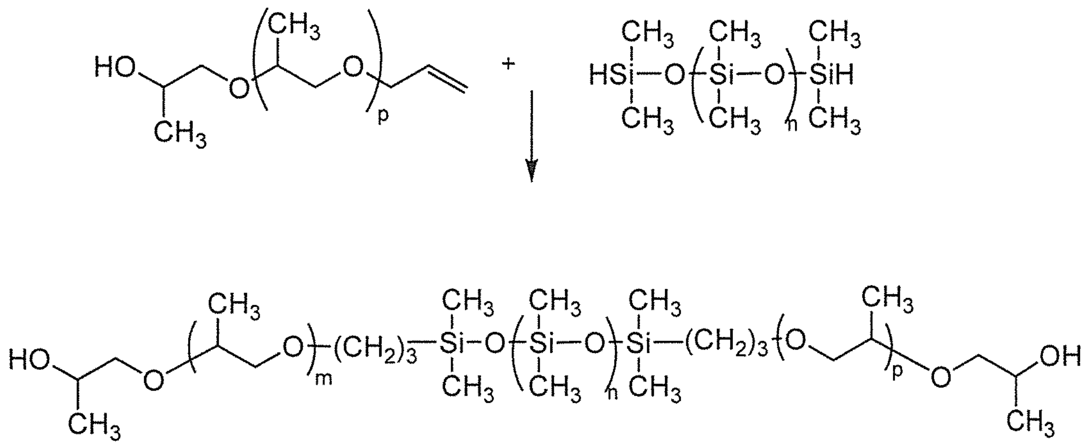

**Table 2. Polymer block ratios**

| Stoiciometric ratios for reaction product: | | | |
|---|---|---|---|
| Polymer block | PO | SiO | PO |
| | m | n | p |
| Ratio | 11 | 11 | 11 |

**Example 3**

**Synthesis of aromatic linked siloxane based triblock copolymer pre-soft-segment:**

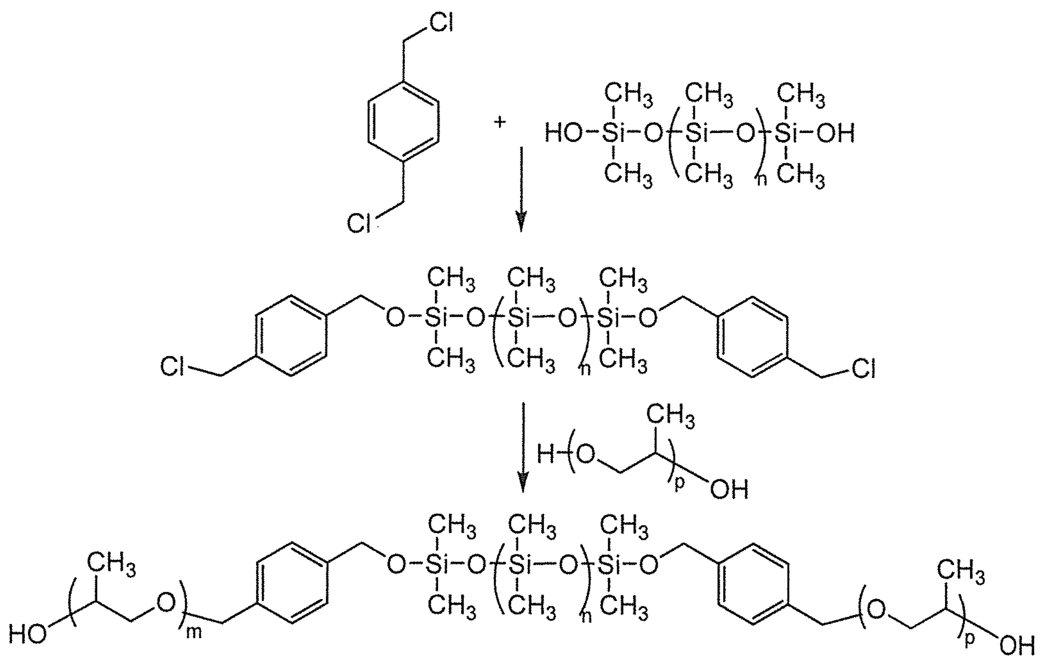

To a 100 ml separable flask fitted with a mechanical stirrer, 15g of hydroxy terminated polydimethyl siloxane (DMS-S14 from Gelest Inc.) was added along with 5.36g of di-chloro p-xylene (from Sigma) and 0.0089g of Copper(II) acetylacetonate ($Cu(Acac)_2$ from Sigma). The reaction mixture was refluxed at 110°C for 5 hrs. At this point, 19.77g of hydroxy terminated poly(propylene glycol) (from Sigma) was added dropwise and the reaction mixture was then refluxed

for another 15hr. The progress of reaction was followed by $^1$H-NMR and the final molecular weight, determined by gel permeation chromatography (GPC), was 3000 g/mol.
H-NMR analysis: Solvent used for $^1$H-NMR analysis is $CDCl_3$.
Aromatic H = 7.25-7.45 ppm, $-CH_2$ = 4.5-4.6 ppm, $-CH_3$ (of PPO)= 1-1.4 ppm, $-CH_2$ (of PPO)= 3.2-3.8 ppm, ---OH (of PPO)= 3.8-4 ppm, $-CH_3$(silanol)= 0.5-0.8 ppm.

**Table 3. Resulting polymer block ratios**

| Stoiciometric ratios for reaction product: | | | |
|---|---|---|---|
| Polymer block | PO | SiO | PO |
| | m | n | p |
| Ratio | 14 | 15.5 | 14 |

**Example 4**

**Synthesis of aromatic linked fluorosiloxane based triblock copolymer pre-soft-segment:**

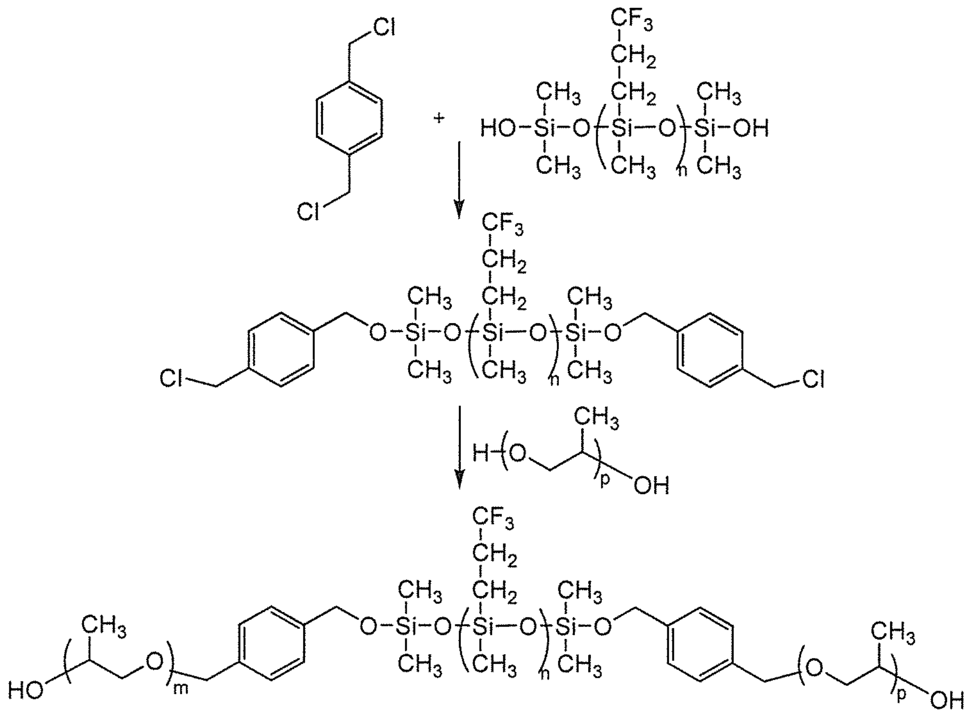

To a 100 ml separable flask fitted with a mechanical stirrer, 15g of hydroxy terminated polytrifluoromethyl siloxane (FMS-9922 , Gelest inc.) was added along with 5.9g of di-chloro p-xylene and 0.0098g of copper(II) acetylacetonate ($Cu(Acac)_2$ from Sigma). The reaction mixture was refluxed at 110 °C for 5 hrs. At this point, 21.75g of hydroxy terminated poly(propylene glycol) (from Sigma) was added dropwise to the reaction mixture. The reaction was refluxed for another 15hr. The progress of reaction was followed by $^1$H-NMR analysis and the molecular weight, determined by gel permeation chromatography (GPC), was 3100 g/mol.
$^1$H-NMR analysis: Solvent used for H-NMR analysis is $CDCl_3$.
Aromatic $^1$H = 7.25-7.45 ppm, $-CH_2$ = 4.5-4.6 ppm, $-CH_3$ (of PPO)= 1-1.4 ppm, $-CH_2$ (of PPO)= 3.2-3.8 ppm, ---OH (of PPO)= 3.8-4 ppm, $-CH_3$(silanol)= 0.5-0.8 ppm.

**Table 4. Polymer block ratios**

| Stoiciometric ratios for reaction product: | | | |
|---|---|---|---|
| Polymer block | PO | FSiO | PO |
| | m | n | p |
| Ratio | 14 | 9.2 | 14 |

**Example 5**

**Preparation of water blown foam:**

The pre-soft segments prepared can be described as having polymer block ratios which are numerically represented by the letters m, n and o for the constituents PO/SiO/PO respectively. The triblock copolymers prepared in Examples 1 and 2 with specific m, n, o ratios were formulated into polyurethane/urea foams as illustrated by Table 7.

The process for preparing the foam was a two-step procedure. The following describes the method of manufacture of the first product in Table 7. The same procedure was used to prepare other foams as described by Table 8.

Step 1) Firstly a mixture was made with 0.041g of DABCO LV-33 (Airproducts), 0.120g of bismuth neodecanoate (Bicat 8108M from Shepherd chemicals), 0.467g of diethanol amine (DEOA, from Sigma), 7.917 g of synthesized block copolymer, 0.200g water and 0.1 g of surfactant (Niax L-618 from Airproducts) in a plastic flat bottomed container. This is then thoroughly mixed manually for 30 sec until a homogenous mixture was obtained.

Step 2) To the above mixture, 15g of a diisocyanate prepolymer (PPT 95A Airproducts) was added. This was then thoroughly mixed by a mechanical stirrer for about 5 seconds. The material was then molded and cured at 70°C for 2.5 hours and post cured at 50°C for another 3 hours.

**Table 5. Formulation details for foam**

| Formulation Identification | Polymer block (PO/SiO/PO) Ratio m:n:p | DABCO | BICAT | DEOA | $H_2O$ |
|---|---|---|---|---|---|
| VF230209A | 11:11:11 | 0.0325 | 0.015 | 0.40 | 1.0 |
| VF090309B | 11:9:11 | 0.0325 | 0.015 | 0.40 | 1.0 |

**Example 6**

**Comparative example of formulation of water blown foam from triblock copolymer pre-soft segment and individual homopolymers:**

Polyurethane/urea polymer foams from Example 5 were compared to foams made from the stoiciometric equivalent homopolymer soft segments. The foams with homopolymer based soft segments (VF130309 and VF190309) shown in Figure 109 were produced as follows (VF130309):

Step 1) Firstly a mixture was made with 0.041g of DABCO LV-33 (Airproducts), 0.120g of bismuth neodecanoate (Bicat 8108M from Shepherd chemicals), 0.467g of diethanol amine (DEOA, from Sigma), 3.056g of poly(dimetyl siloxane) diol (DMS-s14 Gelest Inc.), 1.633 g of polypropylene oxide (Mw = 700g/mol), 0.200g water and 0.1 g of surfactant (Niax L-618 from Airproducts). These were added to a plastic flat bottomed container and were thoroughly mixed manually for 30 sec until a homogenous mixture was obtained.

Step 2) To the above mixture, 15g of a diisocyanate prepolymer (PPT 95A Airproducts) was added. This was then thoroughly mixed by a mechanical stirrer for 5 seconds. The material was then molded and cured at 70°C for 2.5 hours and post cured at 50°C for another 3 hours.

The foams in this example were made into dumbell shapes for tensile testing. Figures 110 and 111 illustrate the difference in mechanical behaviour between the comparitive materials indicating a favourable lowering in modulus for the triblock copolymer pre-soft-segments.

**Example 7**

**Comparitive stability of triblock copolymer soft segment versus homopolymer soft segment**

Tensile test specimens were prepared in the same manner to the materials used in Example 4 and were subjected to accelerated aging in simulated gastric fluid (as per United States Pharmacopeia, "USP"). The materials produced with the pre-synthesised triblock copolymer soft segments resulted in substantially improved mechanical stability in gastric fluid as compared to the urethane/urea linked homopolymer equivalent as illustrated in Figure 112. This facilitates the use of such materials for prolonged periods in digestive and more specifically gastric environments.

Example 8

Preparation of water blown foams

Several water blown polyurethane/urea foams were also produced with varying PO/EO/SiO polymer block ratios. The process for preparing the foam as described above was used.

**Table 6. Water blown formulations incorporating siloxane containing copolymer pre-soft-segments.**

| Polymer block ratio (PO/EO/SiO) m:n:p | DABCO | BICAT | DEOA | $H_2O$ |
|---|---|---|---|---|
| 41.5:8.3:0.5 | 0.114 | 0.022 | 0.22 | 2.72 |
| 40.2:7.8:0.5 | 0.114 | 0.022 | 0.22 | 2.72 |
| 37.5:7:0.5 | 0.114 | 0.022 | 0.22 | 2.72 |
| 33.5:5.7:0.5 | 0.114 | 0.022 | 0.22 | 2.72 |
| 29.6:4.4:0.5 | 0.114 | 0.022 | 0.22 | 2.72 |
| 21.6:1.8:0.5 | 0.114 | 0.022 | 0.22 | 2.72 |
| 19:1:0.5 | 0.114 | 0.022 | 0.22 | 2.72 |
| 29.6:4.5:1.1 | 0.114 | 0.022 | 0.22 | 2.72 |

The results from the formulations described in Table 6 are shown in Table 7.

**Table 7. Results from mechanical testing of foams from Table 5**

| Polymer block ratio (PO/EO/SiO) m:n:p | % Elongation | Tensile Strength (N) |
|---|---|---|
| 41.5:8.3:0.5 | 233 | 0.46 |
| 40.2:7.8:0.5 | 243 | 0.31 |
| 37.5:7:0.5 | 237 | 0.3 |
| 33.5:5.7:0.5 | 260 | 0.23 |
| 29.6:4.4:0.5 | 320 | 0.23 |
| 21.6:1.8:0.5 | 497 | 0.23 |
| 19:1:0.5 | 462 | 0.22 |
| 29.6:4.5:1.1 | 437 | 0.29 |

**Example 9**

**Use Example**

Devices for use in the gastrointestinal system have historically not been made from specifically designed materials. Off the shelf materials used for application in the corrosive environment of the stomach have limited biostability and generally lose their functionality after a short time.

The foam described can be used for production of a valve of the type described in our US2007-0198048A..

The valve has an open position and a closed position. The valve will have a proximal end and a distal end. The valve material can open from the proximal direction when the action of swallowing (liquid or solid) stretches an oriface by between 100% and 3000% in circumference. The open orifice optionally closes non-elastically over a prolonged period of time, thus mimicing the body's natural response. The duration taken to close may be between 2 and 15 sec. The material can stretch to between 100% - 300% from the distal direction when gas, liquid or solids exceeds a pre-determined force of between 25cm$H_2O$ and 60cm$H_2O$. In some embodiments, the material absorbs less than 15% of its own mass of water at equilibrium. In some embodiments, the material loses (leaches) less than 3% of it's own mass at equilibrium in water or alcohol. In some embodiments, the material loses less than 10% of its tensile strength when immersed in a simulated gastric fluid at pH 1.2 for 30 days. In some embodiments, the valve material loses less than 25% of its % elongation when immersed in a simulated gastric fluid at pH 1.2 for 30 days.

**Example 10**

**Valve functional testing**

The healthy lower esophageal sphincter (LES) remains closed until an individual induces relaxation of the muscle by swallowing and thus allowing food to pass in the antegrade direction. Additionally when an individual belches or vomits they generate enough pressure in the stomach in the retrograde direction to overcome the valve. An anti-reflux valve must enable this functionality when placed in the body, thus a simple functional test is carried out to asses performance.

It has been reported that post fundoplication patients have yield pressures between 22 - 45 mmHg and that most of the patients with gastric yield pressure above 40 mmHg experienced problems belching. *See* Yield pressure, anatomy of the cardia and gastro-oesophageal reflux. Ismail, J. Bancewicz, J. Barow British Journal of Surgery. Vol: 82, 1995, pages: 943-947. Thus, in order to facilitate belching but prevent reflux, an absolute upper GYP value of 40 mmHg (550 mm$H_2O$) is reasonable. It was also reported that patients with visible esophagitis all have gastric yield pressure values under 15 mmHg, therefore, there is good reason to selectively target a minimum gastric yield pressure value that exceeds 15 mmHg. *See* Id. An appropriate minimum gastric yield pressure value would be 15mmHg + 25% margin of error thus resulting in a minimum effective valve yield pressure value of 18.75 mmHg or 255 mm$H_2O$.

The test apparatus consists of a 1m high vertical tube as shown in Figure 113, to which is connected a peristaltic pump and a fitting that is designed to house the valve to be tested.

The valve to be tested is placed in a water bath at 37 °C for 30 minutes to allow its temperature to equilibrate. Once the temperature of the valve has equilibrated it is then installed into the housing such that the distal closed end of the valve faces the inside of the test apparatus. The pump is then switched on at a rate of 800 ml/min to begin filling the vertical tube. The rising column of water exerts a pressure that forces the valve shut initially. As the pressure in the column rises the valve reaches a point where it everts and allows the water to flow through. This point, known as the yield pressure, is then recorded and the test repeated four times.

**Example 11**

**Rationale for accelerated aging of material**

Clinical Condition being simulated

The lower oesophagus of a normal patient can be exposed to the acidic contents of the stomach periodically without any adverse side effects. However, patients with gastro esophageal reflux disease experience damage to the mucosa of the lower oesophagus due to increased exposure to the gastric contents. Exposure of the lower oesophagus to acidic gastric contents is routinely measured in the clinic using dedicated pH measurement equipment. A typical procedure involves measuring pH over a 24-hour period. The levels of acid exposure in pathological reflux disease patients is summarised in Table 8 from six clinical references. *See* DeMeester TR, Johnson LF, Joseph GJ, et al. Patterns of Gastroesophageal Reflux in Health and Disease Ann. Surg. Oct 1976 459-469; Pandolfino JE, Richter JE, Ours T, et al. Ambulatory Esophageal pH Monitoring Using a Wireless System Am. J. Gastro 2003; 98:4; Mahmood Z, McMahon BP, Arfin Q, et al. Results of endoscopic gastroplasty for gastroesophageal reflux disease: a one year prospective follow-up Gut 2003; 52:34-9; Park PO, Kjellin T, Appeyard MN, et al. Results of endoscopic gastroplasty suturing for treatment of GERD: a multicentre trial Gastrointest endosc 2001; 53:AB115; Filipi CJ, Lehman GA, Rothstein RI, et al. Transoral flexible endoscopic suturing for treatment of GERD: a multicenter trial Gastrointest endosc 2001; 53 416-22; and Arts J, Slootmaekers S Sifrim D, et al. Endoluminal gastroplication (Endocinch) in GERD patient's refractory to PPI therapy Gastroenterology 2002; 122:A47.

**Table 8. Summary of acid exposure in patients with reflux disease**

| Investigator | Number of patients | Details | % 24h <pH4 |
|---|---|---|---|
| DeMeester | 54 | Combined refluxers | 13.5 |
| Pandolfino | 41 | Gerd | 6.5 |
| Mahmood | 21 | Gerd | 11.11 |
| Park | 142 | Gerd | 8.5 |
| Filipi | 64 | Gerd | 9.6 |
| Arts | 20 | Gerd | 17 |
| **Average** | | | 11.035 |

Key Clinical Parameters

Considering that the lower oesophagus is exposed to the acidic pH exposure time for an average of 11% of the measurement period, an accelerated aging methodology can easily be conceived. Constant exposure of a test material to the gastric contents (or USP Simulated Gastric Fluid - Reference USP Pharmacopeia) would represent an almost 10-fold increase in the rate of aging. Thus the time required to simulate one year of exposure of the lower oesophagus to the gastric contents is described by equation 1.

$$\left(\frac{11.035}{100}\right) \times 365 days = 40.28 days \qquad \textit{Equation 1}$$

Clinical Rationale

Immersion of test specimens in USP Simulated gastric fluid for 40.27 days at 37 °C will approximate one year's exposure of the lower oesophagus to acidic gastric contents in a GERD patient's scenario.

| Simulated Exposure | Real Time |
|---|---|
| 1 year | 40.28 days |
| 2 years | 80.56 days |
| 3 years | 120.84 days |

Results of accelerated stability of a valve prepared from a viscoelastic foam of the present invention are depicted in Figures 114 and 115.

This invention is to be defined by the appended claims rather than by the specific embodiments that have been represented by way of example.

**Claims**

1. A gastrointestinal implant device comprising a sleeve (501, 601, 750) configured to extend into the duodenum comprising:-

   an artificial valve (1, 100, 110, 140, 301, 500, 552, 602) configured for placement at the pylorus to control flow from the stomach into the duodenal sleeve (501, 601, 750); and
   a support structure (20, 606) configured for the valve, wherein the support structure comprises a luminal prosthesis (20, 606),
   **characterised in that** the luminal prosthesis comprises a proximal flare (620).

2. An implant device as claimed in claim 1 wherein the luminal prosthesis (606) comprises a distal bulbous region (612).

3. An implant device as claimed in claim 1 or 2 wherein the luminal prosthesis (606) comprises a scaffold receiving region (622).

4. An implant device as claimed in claim 3 wherein the scaffold receiving region (622) is intermediate the proximal and distal ends of the luminal prosthesis (606).

5. A gastrointestinal implant device as claimed in any of claims 1 to 4 wherein the sleeve comprises a retaining means (650) to assist in retaining the sleeve at a desired location.

6. An implant device as claimed in claim 5 wherein the retaining means comprises a retaining ring (650) which may be located at or adjacent to a distal end of the sleeve.

7. An implant device as claimed in claim 5 or 6 comprising a plurality of retaining rings (650) which are axially spaced-apart along the sleeve.

8. A gastrointestinal implant device as claimed in any of claims 1 to 7 wherein the valve (1, 100, 110, 140, 301, 500, 552, 602) is of a viscoelastic foam and comprising at least three valve leaflets (3, 4, 5, 333), the valve (1, 100, 110, 140, 301, 500, 552, 602) having a normally closed configuration and an open configuration, the valve leaflets (3, 4, 5, 333) being movable from the closed configuration to the open configuration for flow from the stomach.

9. An implant device as claimed in claim 8 wherein the valve (1, 100, 110, 140, 301, 500, 552, 602) is adapted to open automatically for stomach emptying and to return automatically to the closed configuration.

10. An implant device as claimed in claim 8 or 9 wherein the valve (1, 100, 110, 140, 301, 500, 552, 602) comprises an outer support region (2, 302) and a main body region (6, 306) extending between the support region (2, 302) and the valve leaflets (3, 4,5, 333).

11. An implant device as claimed in any of claims 8 to 10 wherein the valve (1, 100, 110, 140, 301, 500, 552, 602) has a region (643) of co-aption of the valve leaflets (3, 4, 5) in the closed configuration.

12. An implant device as claimed in any of claims 8 to 11 comprising an anchor for anchoring the valve at the pylorus.

13. An implant device as claimed in any of claims 1 to 12 wherein the sleeve is mounted to the valve or to an anchor for the valve.

14. An implant device as claimed in any of claims 1 to 13 wherein the device is adapted for placement in the pyloric sphincter.

15. An implant device as claimed in any of claims 1 to 14 wherein the device is adapted for placement distal of the pyloric sphincter.

## Patentansprüche

1. Gastrointestinale Implantatvorrichtung, umfassend eine Hülse (501, 501, 750), die dazu konfiguriert ist, sich in das Duodenum zu erstrecken, umfassend:

   eine künstliche Klappe (1, 100, 110, 140, 301, 500, 552, 602), die zur Platzierung an dem Pylorus konfiguriert ist, um die Strömung vom Magen in die Duodenalhülse (501, 601, 750) zu steuern, und
   eine Stützstruktur (20, 606), die für die Klappe konfiguriert ist, wobei die Stützstruktur eine Luminalprothese (20, 606) umfasst, **dadurch gekennzeichnet, dass** die Luminalprothese eine proximale Schürze (620) umfasst.

2. Implantatvorrichtung nach Anspruch 1, wobei die Luminalprothese (606) einen distalen knollenförmigen Bereich (612) umfasst.

3. Implantatvorrichtung nach Anspruch 1 oder 2, wobei die Luminalprothese (606) einen Gerüstaufnahmebereich (622) umfasst.

4. Implantatvorrichtung nach Anspruch 3, wobei der Gerüstaufnahmebereich (622) zwischen dem proximalen und dem distalen Ende der Luminalprothese (606) liegt.

5. Implantatvorrichtung nach einem der Ansprüche 1 bis 4, wobei die Hülse ein Haltemittel (650) umfasst, um zum Halten der Hülse an einem gewünschten Ort beizutragen.

6. Implantatvorrichtung nach Anspruch 5, wobei das Haltemittel einen Haltering (650) umfasst, der an einem distalen Ende der Hülse oder benachbart dazu angeordnet sein kann.

7. Implantatvorrichtung nach Anspruch 5 oder 6, umfassend eine Vielzahl von Halteringen (650), die entlang der Hülse axial beabstandet sind.

8. Gastrointestinale Implantatvorrichtung nach einem der Ansprüche 1 bis 7, wobei die Klappe (1, 100, 110, 140, 301, 500, 552, 602) aus einem viskoelastischen Schaumstoff ist und mindestens drei Klappensegel (3, 4, 5, 333) umfasst, wobei die Klappe (1, 100, 110, 140, 301, 500, 552, 602) eine normalerweise geschlossene Konfiguration und eine offene Konfiguration hat, wobei die Klappensegel (3, 4, 5, 333) für Strömung aus dem Magen aus der geschlossenen Konfiguration in die offene Konfiguration bewegbar sind.

9. Implantatvorrichtung nach Anspruch 8, wobei die Klappe (1, 100, 110, 140, 301, 500, 552, 602) geeignet ist, sich automatisch zum Leeren des Magens zu öffnen und automatisch in die geschlossene Konfiguration zurückzukehren.

10. Implantatvorrichtung nach Anspruch 8 oder 9, wobei die Klappe (1, 100, 110, 140, 301, 500, 552, 602) einen äußeren Stützbereich (2, 302) und einen Hauptkörperbereich (6, 306) umfasst, der sich zwischen dem Stützbereich (2, 302) und den Klappensegeln (3, 4, 5, 333) erstreckt.

11. Implantatvorrichtung nach einem der Ansprüche 8 bis 10, wobei die Klappe (1, 100, 110, 140, 301, 500, 552, 602) einen Bereich (643) hat, in dem die Klappensegel (3, 4, 5) in der geschlossenen Konfiguration geschlossen sind.

12. Implantatvorrichtung nach einem der Ansprüche 8 bis 11, umfassend einen Anker zur Verankerung der Klappe am Pylorus.

13. Implantatvorrichtung nach einem der Ansprüche 1 bis 12, wobei die Hülse an der Klappe oder an einem Anker für die Klappe montiert ist.

14. Implantatvorrichtung nach einem der Ansprüche 1 bis 13, wobei die Vorrichtung für eine Platzierung im Pylorussphinkter geeignet ist.

15. Implantatvorrichtung nach einem der Ansprüche 1 bis 14, wobei die Vorrichtung für eine Platzierung distal vom Pylorussphinkter geeignet ist.

## Revendications

1. Dispositif d'implant gastro-intestinal comprenant un manchon (501, 601, 750) configuré pour pénétrer dans le duodénum comprenant :

   une valvule artificielle (1, 100, 110, 140, 301, 500, 552, 602) configurée pour être placée au niveau du pylore afin de commander le flux passant de l'estomac dans le manchon duodénal (501, 601, 750) ; et
   une structure de support (20, 606) configurée pour la valvule,
   dans lequel la structure d'appui comprend une prothèse luminale (20, 606),
   **caractérisé en ce que** la prothèse luminale comprend un évasement proximal (620).

2. Dispositif d'implant selon la revendication 1, dans lequel la prothèse luminale (606) comprend une zone distale (612) bulbeuse.

3. Dispositif d'implant selon la revendication 1 ou 2, dans lequel la prothèse luminale (606) comprend une zone (622) de réception d'échafaudage.

**4.** Dispositif d'implant selon la revendication 3, dans lequel la zone (622) de réception d'échafaudage est située entre les extrémités proximale et distale de la prothèse luminale (606).

**5.** Dispositif d'implant gastro-intestinal selon l'une quelconque des revendications 1 à 4, dans lequel le manchon comprend un moyen de retenue (650) pour aider à retenir le manchon à un endroit souhaité.

**6.** Dispositif d'implant selon la revendication 5, dans lequel le moyen de retenue comprend un anneau de retenue (650) qui peut être situé au niveau de l'extrémité distale du manchon ou adjacent à celle-ci.

**7.** Dispositif d'implant selon la revendication 5 ou 6, comprenant une pluralité d'anneaux de retenue (650) qui sont espacés axialement le long du manchon.

**8.** Dispositif d'implant gastro-intestinal selon l'une quelconque des revendications 1 à 7, dans lequel la valvule (1, 100, 110, 140, 301, 500, 552, 602) est en mousse viscoélastique et comprend au moins trois lames valvulaires (3, 4, 5, 333), la valvule (1, 100, 110, 140, 301, 500, 552, 602) ayant une configuration normalement fermée et une configuration ouverte, les lames valvulaires (3, 4, 5, 333) étant mobiles de la configuration fermée à la configuration ouverte pour permettre un flux depuis l'estomac.

**9.** Dispositif d'implant selon la revendication 8, dans lequel la valvule (1, 100, 110, 140, 301, 500, 552, 602) est apte à s'ouvrir automatiquement pour que l'estomac se vide et à revenir automatiquement à la configuration fermée.

**10.** Dispositif d'implant selon la revendication 8 ou 9, dans lequel la valvule (1, 100, 110, 140, 301, 500, 552, 602) comprend une zone extérieure (2, 302) de soutien et une zone principale (6, 306) de corps qui s'étend entre la zone (2, 302) de soutien et les lames valvulaires (3, 4, 5, 333).

**11.** Dispositif d'implant selon l'une quelconque des revendications 8 à 10, dans lequel la valvule (1, 100, 110, 140, 301, 500, 552, 602) comporte une zone (643) de coaptation des lames valvulaires (3, 4, 5) dans la configuration fermée.

**12.** Dispositif d'implant selon l'une quelconque des revendications 8 à 11, comprenant un ancrage pour ancrer la valvule au pylore.

**13.** Dispositif d'implant selon l'une quelconque des revendications 1 à 12, dans lequel le manchon est monté sur la valvule ou sur un ancrage pour la valvule.

**14.** Dispositif d'implant selon l'une quelconque des revendications 1 à 13, dans lequel le dispositif est apte à être placé dans le sphincter pylorique.

**15.** Dispositif d'implant selon l'une quelconque des revendications 1 à 14, dans lequel le dispositif est apte à être placé en position distale par rapport au sphincter pylorique.

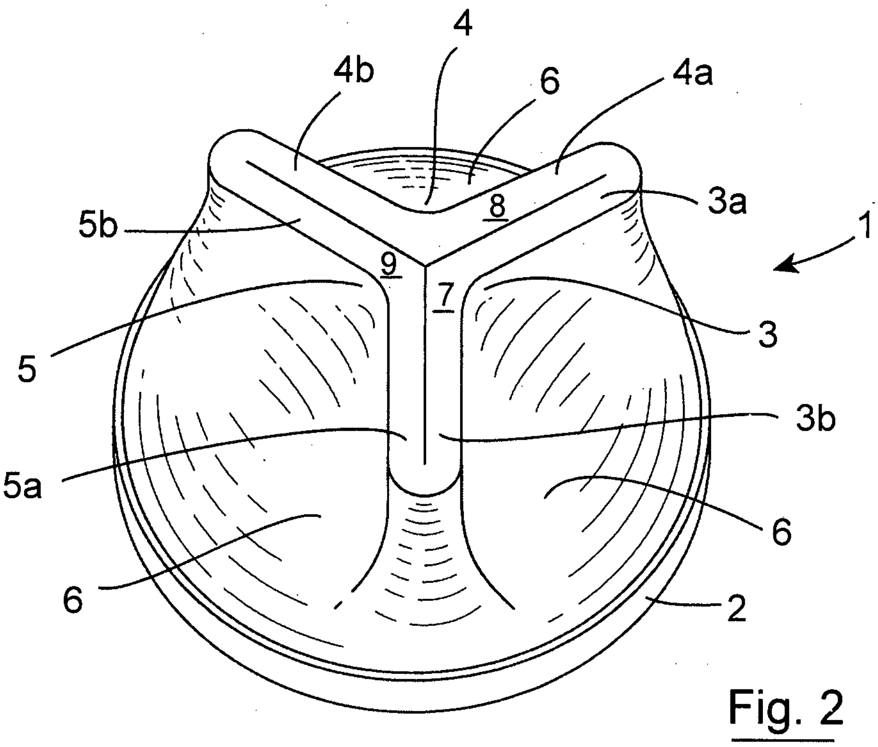


Fig. 2

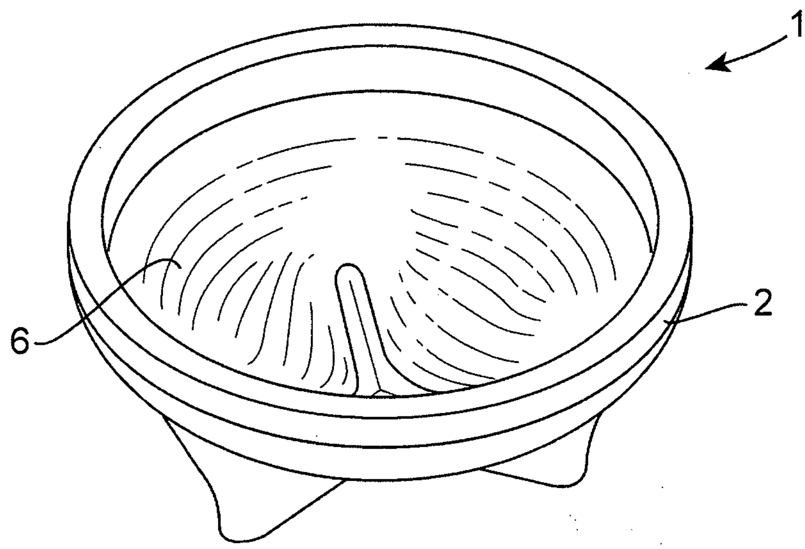


Fig. 1

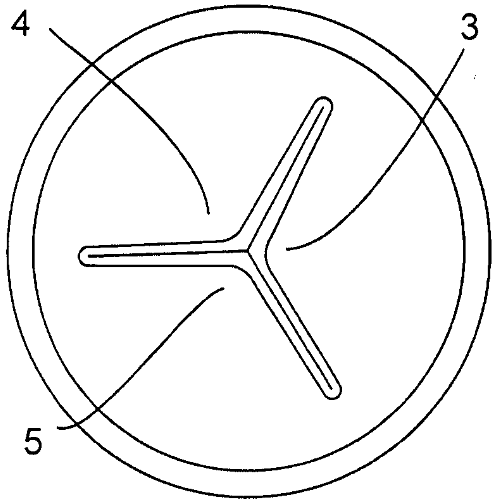


Fig. 3

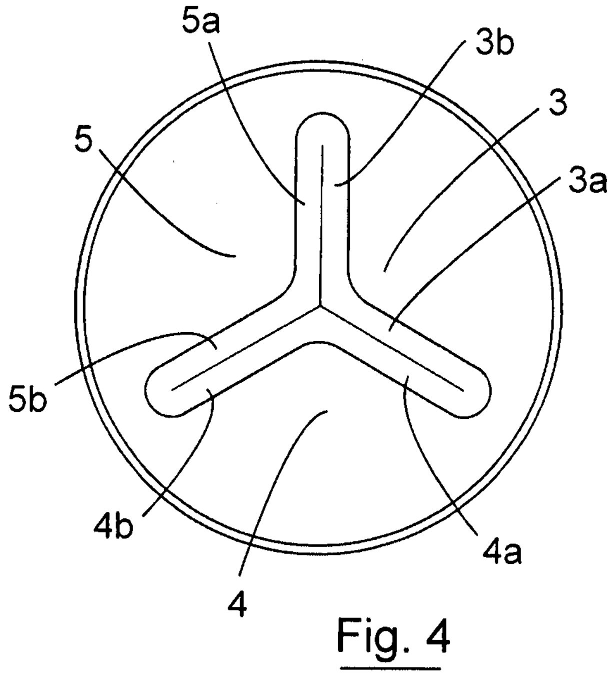


Fig. 4

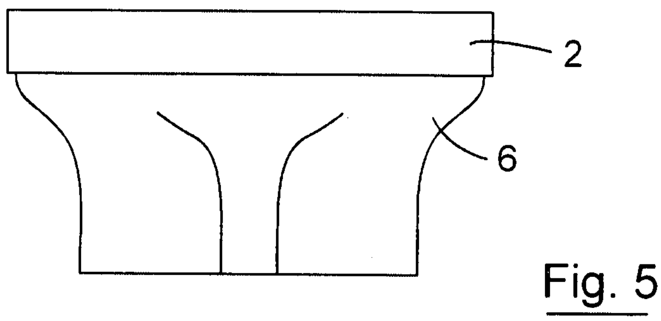


Fig. 5

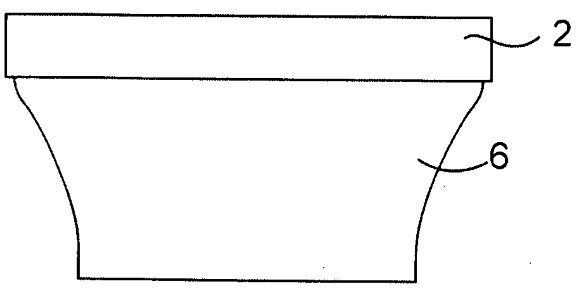


Fig. 6

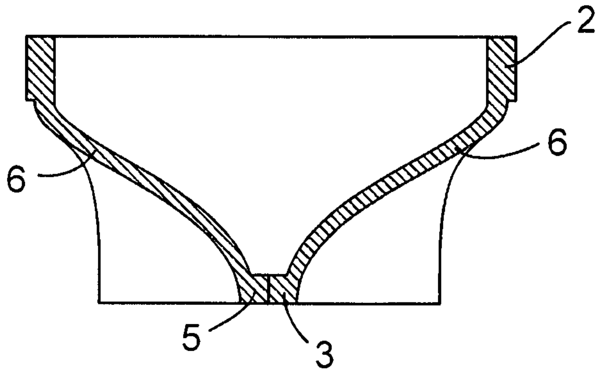


Fig. 9

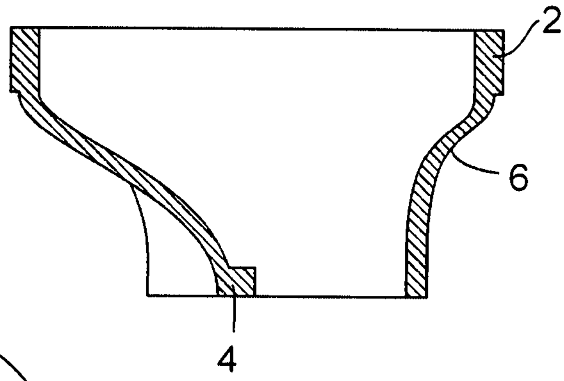


Fig. 10

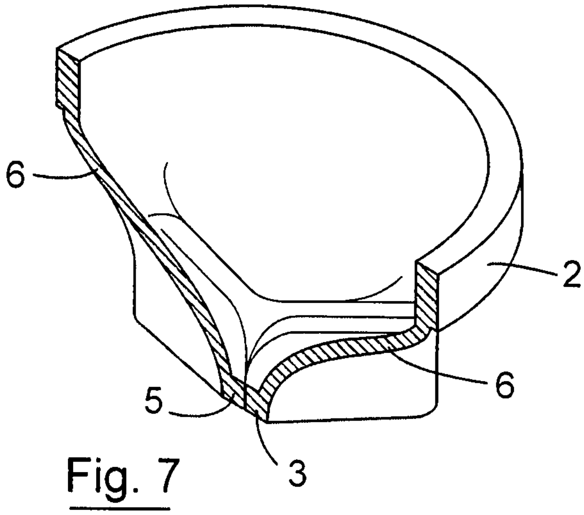


Fig. 7

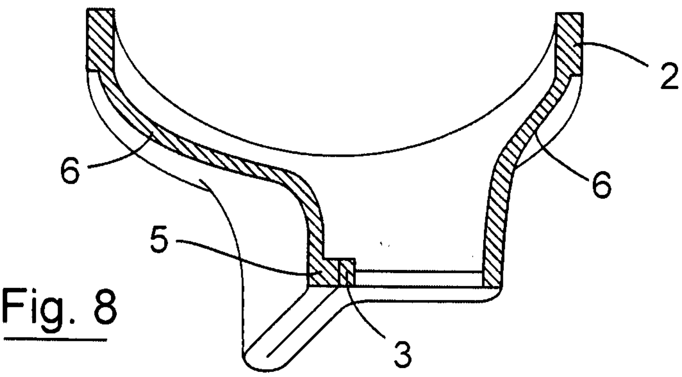


Fig. 8

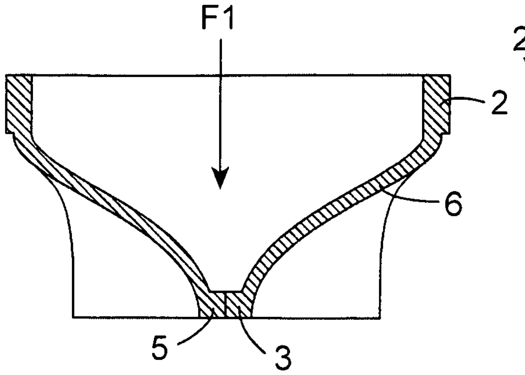


Fig. 11

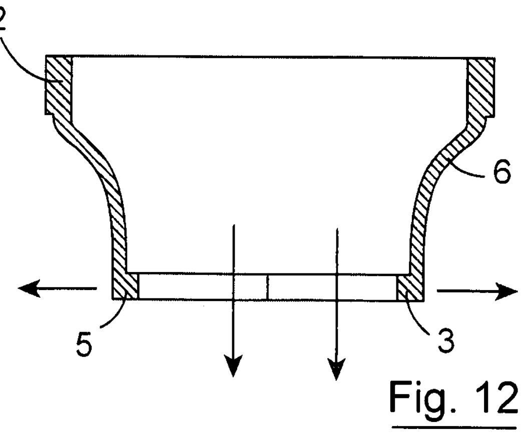


Fig. 12

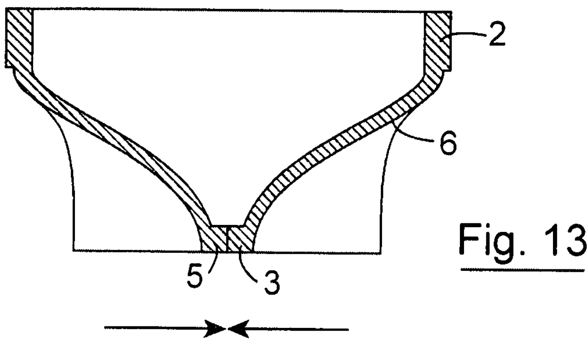


Fig. 13

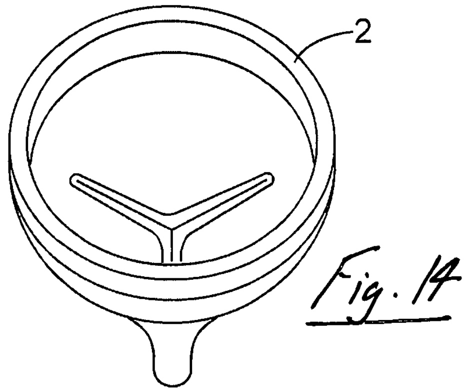


Fig. 14

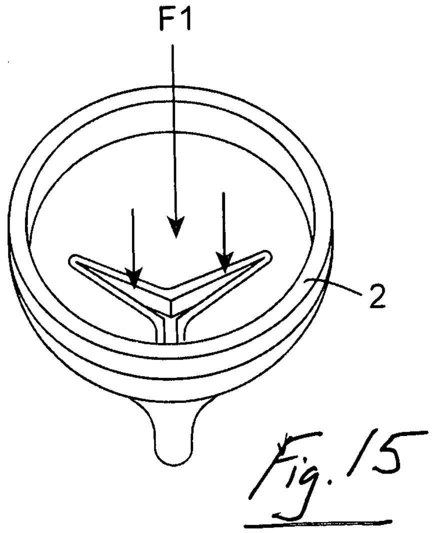


Fig. 15

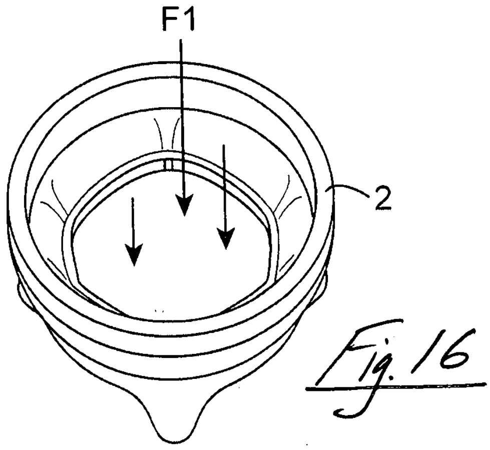


Fig. 16

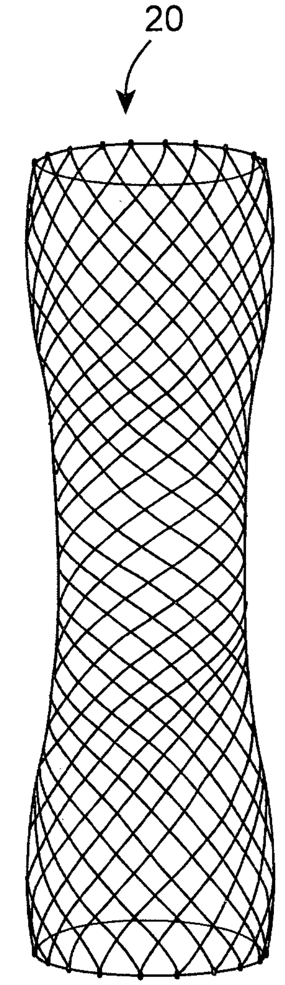


Fig. 17

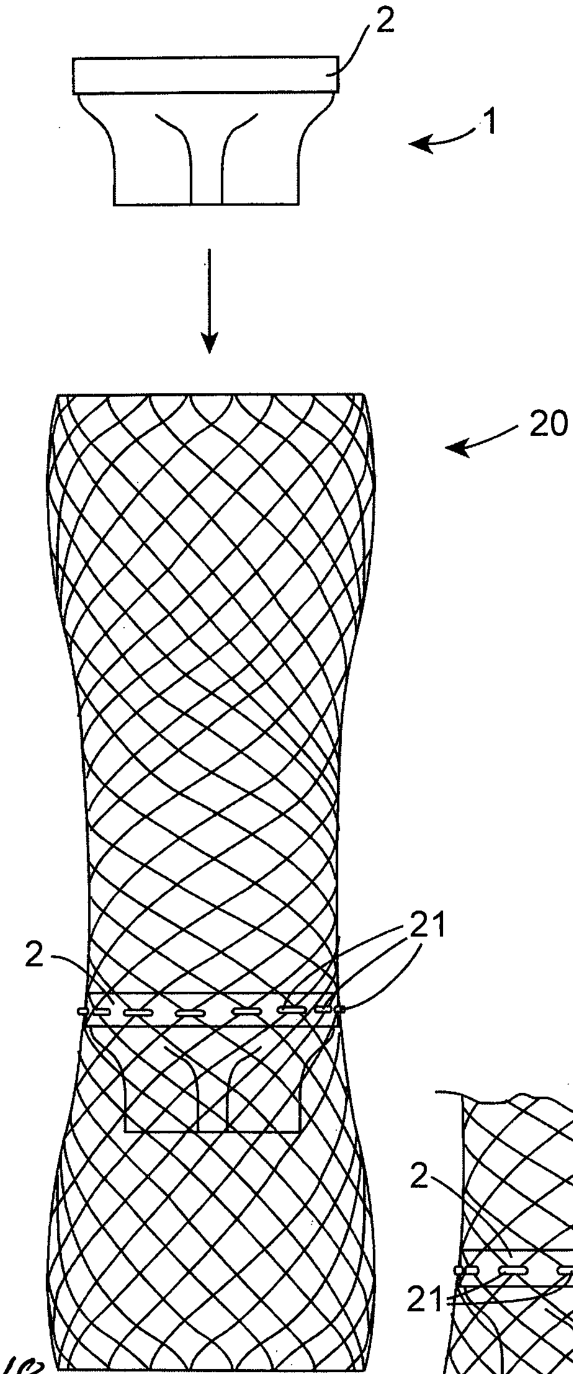


Fig. 18

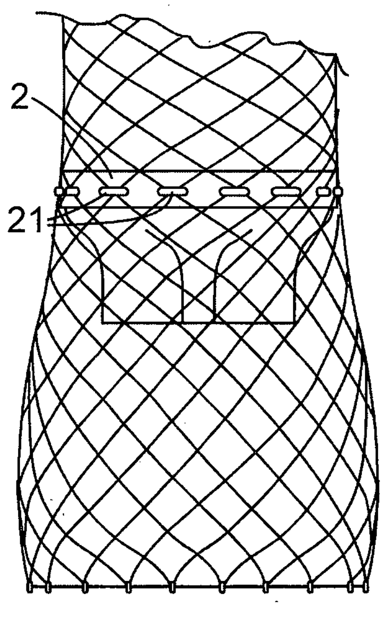


Fig. 19

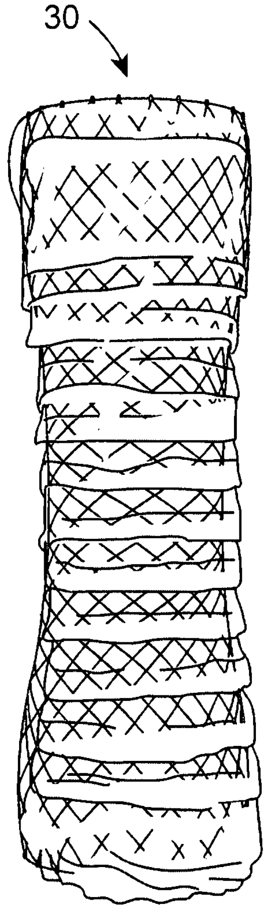


Fig. 20

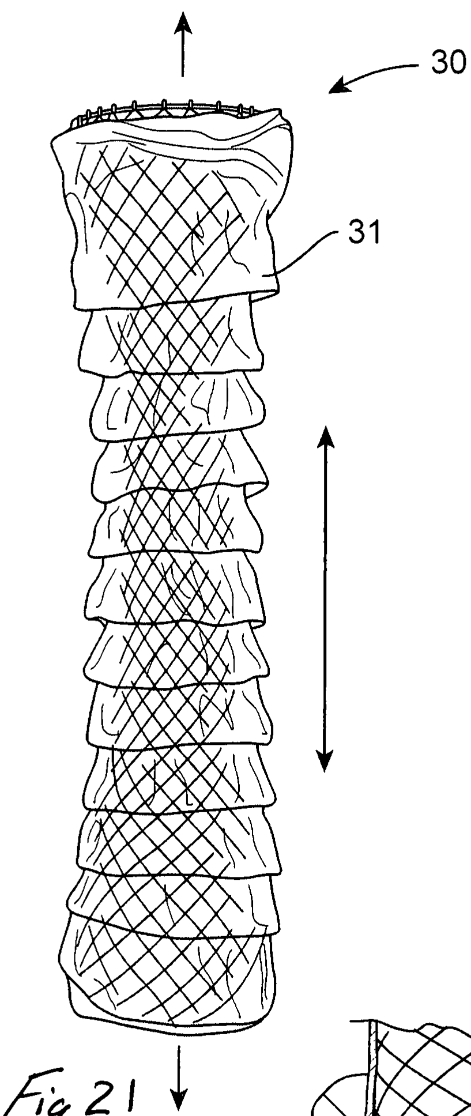


Fig. 21

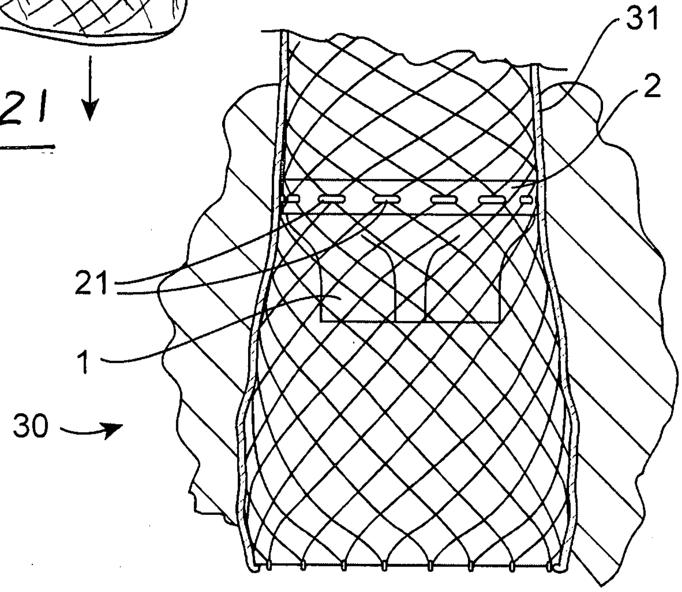


Fig. 23

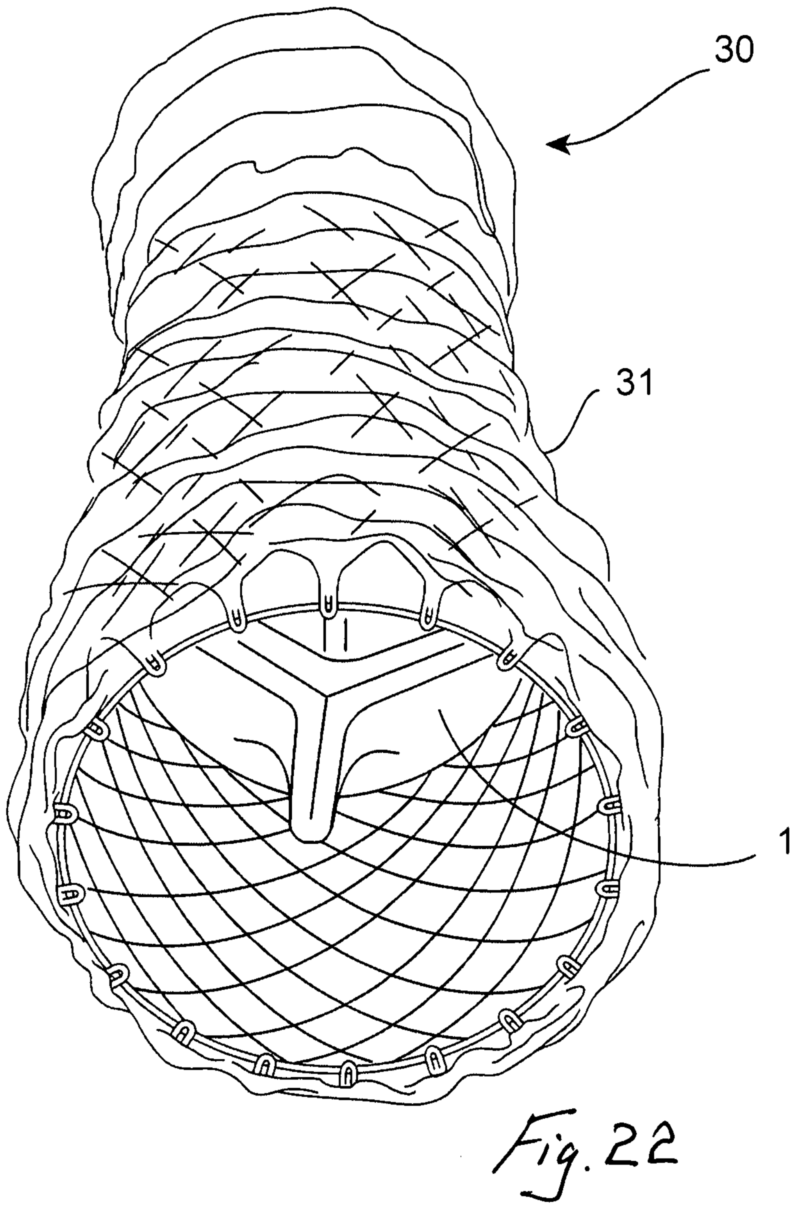


Fig. 22

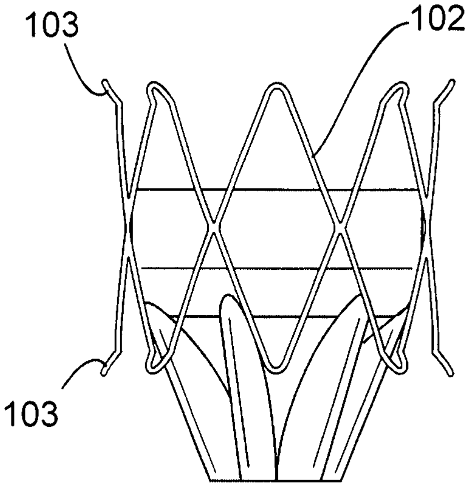


Fig.25

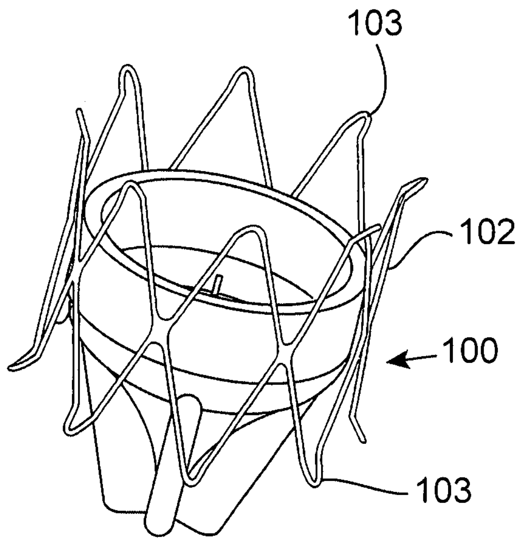


Fig. 24

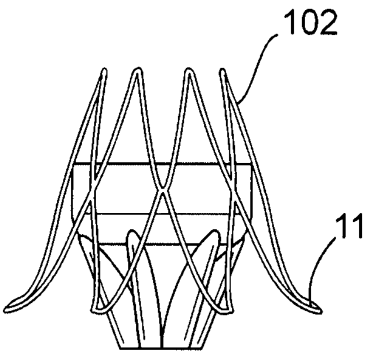


Fig. 27

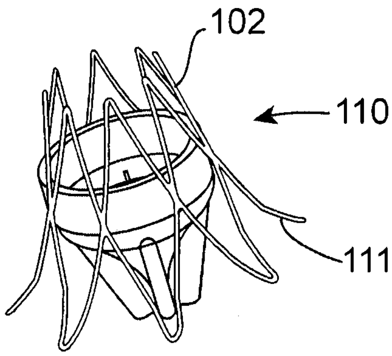


Fig.26

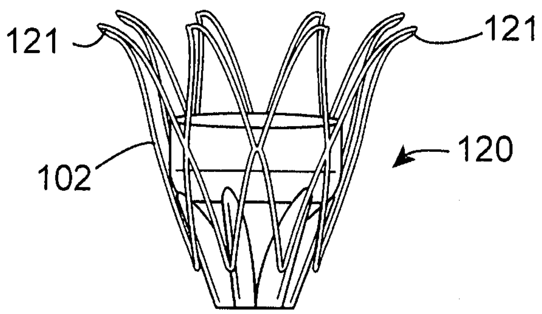


Fig. 28

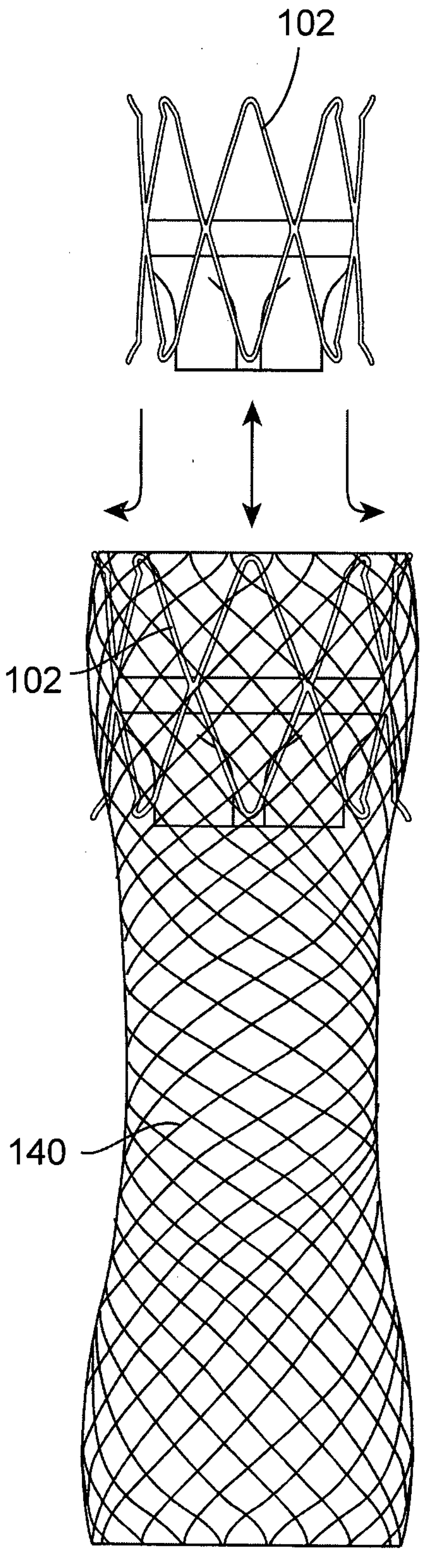


Fig. 29

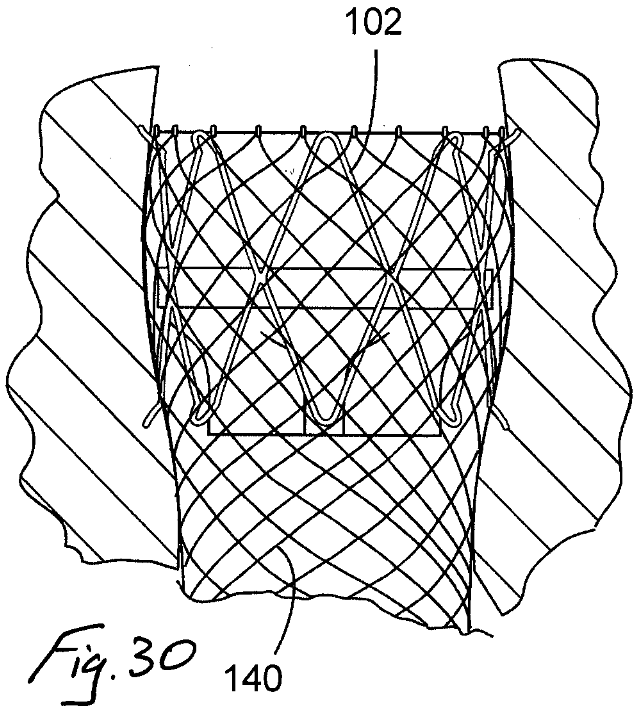


Fig. 30

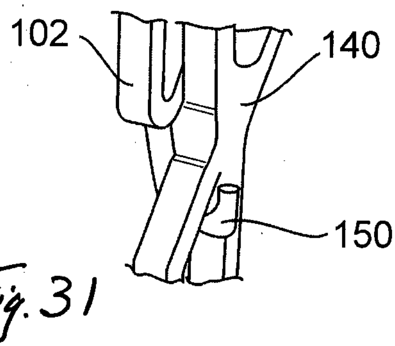


Fig. 31

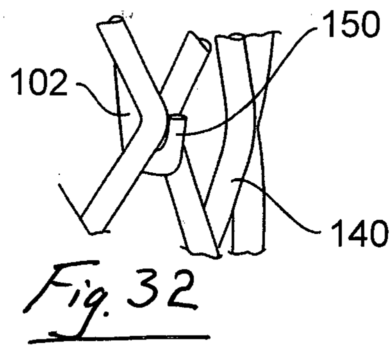


Fig. 32

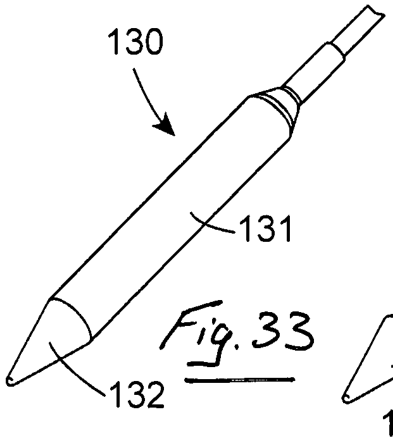


Fig. 33

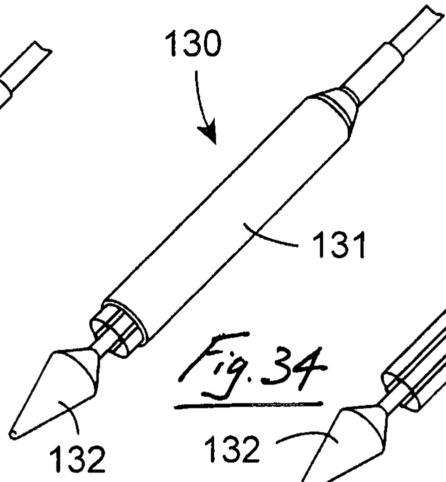


Fig. 34

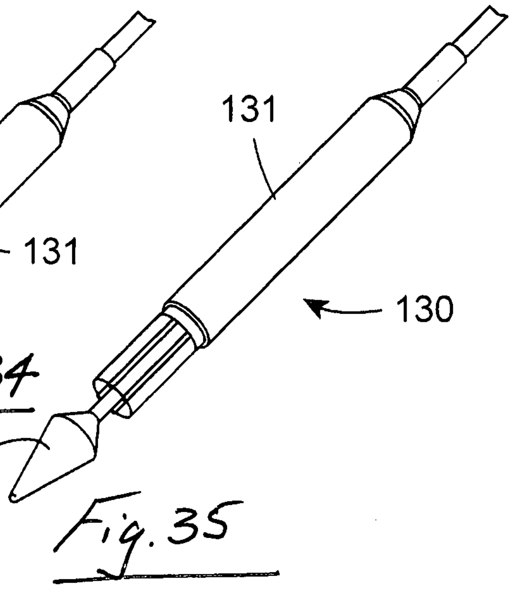


Fig. 35

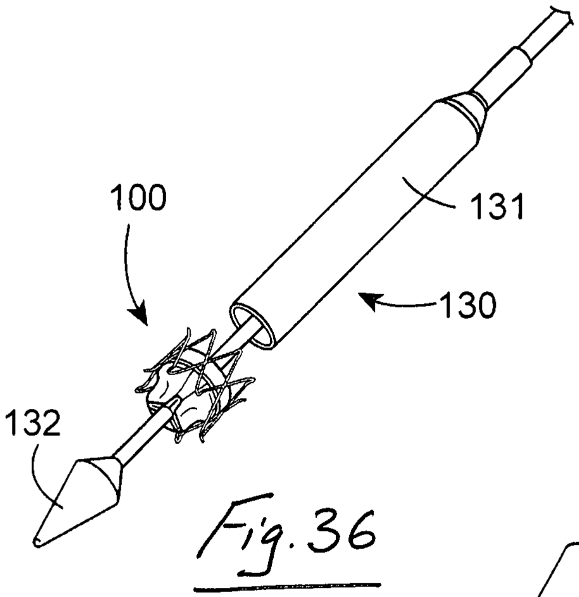


Fig. 36

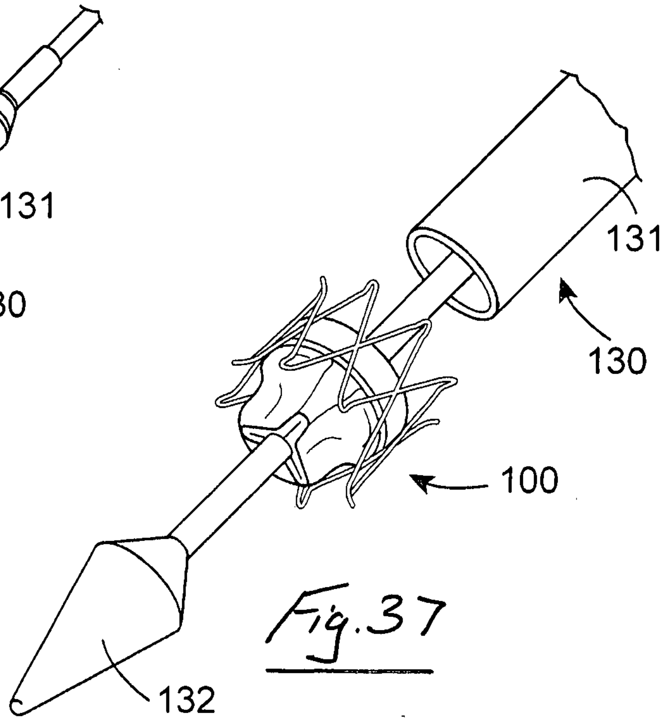


Fig. 37

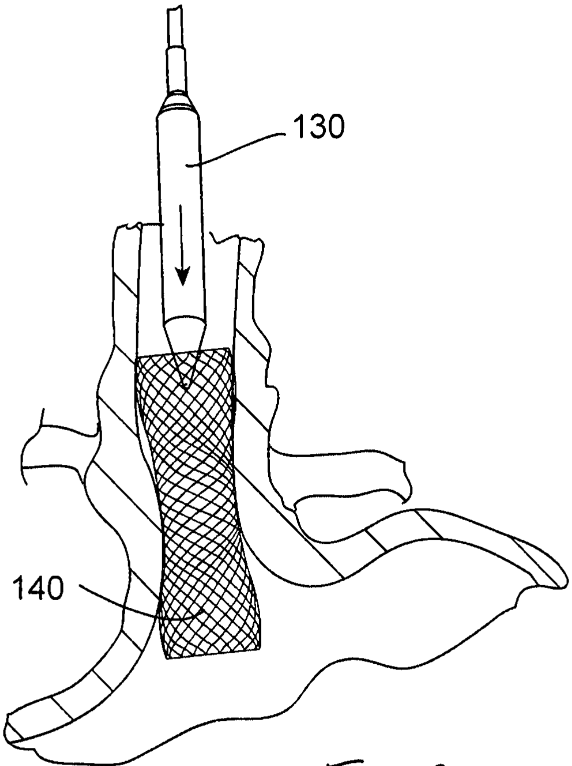


Fig. 38

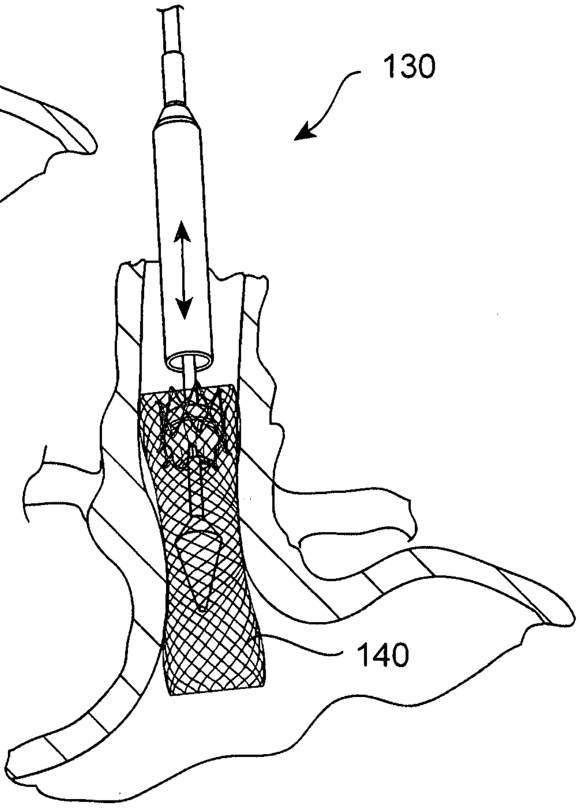


Fig. 39

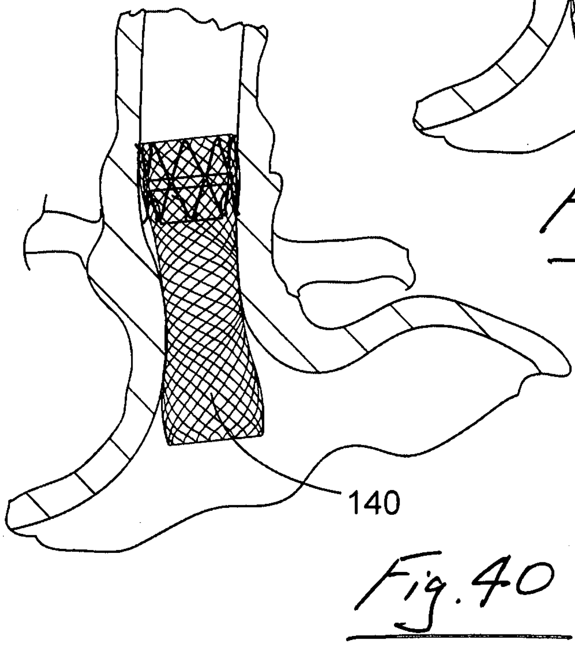


Fig. 40

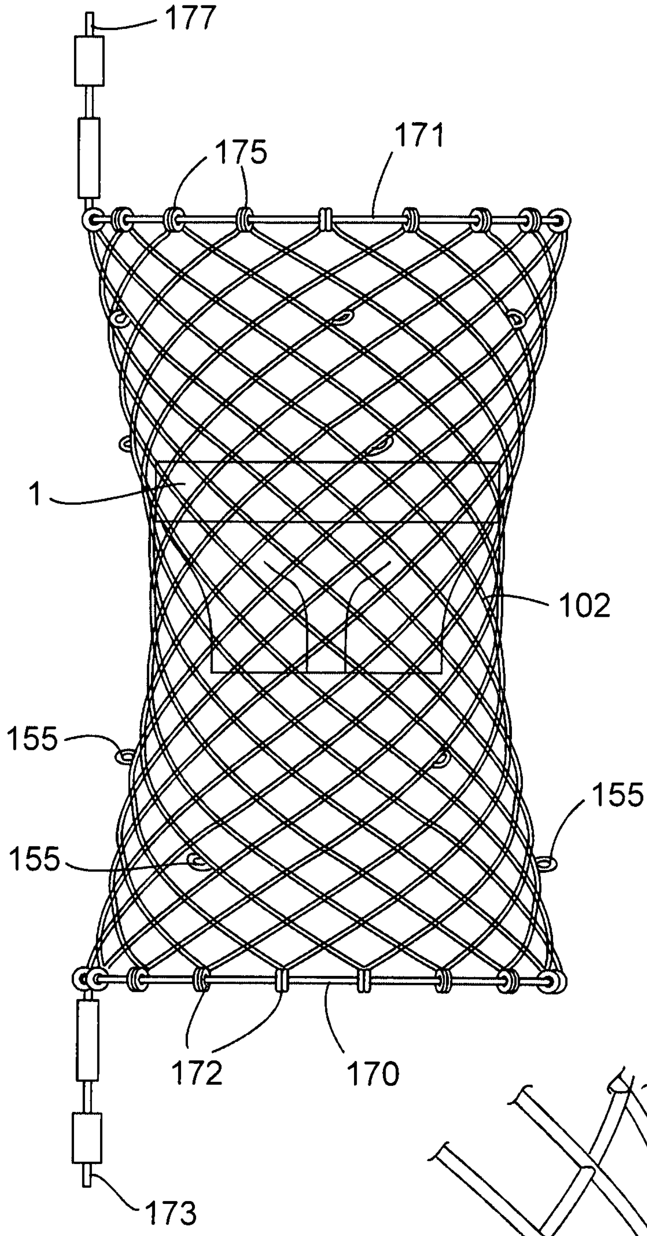


Fig. 41

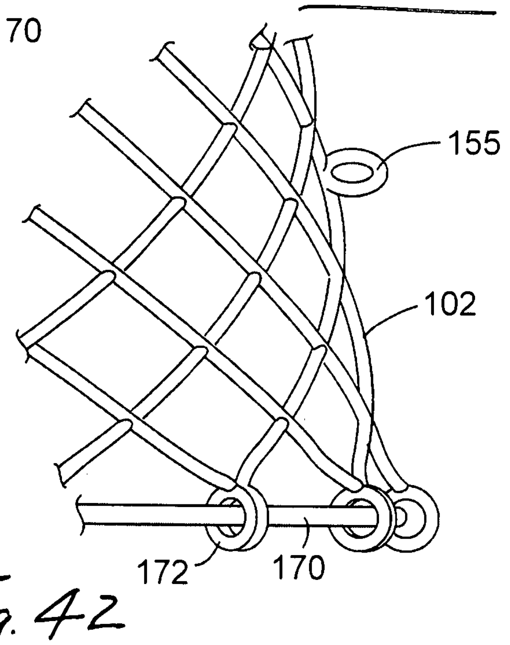


Fig. 42

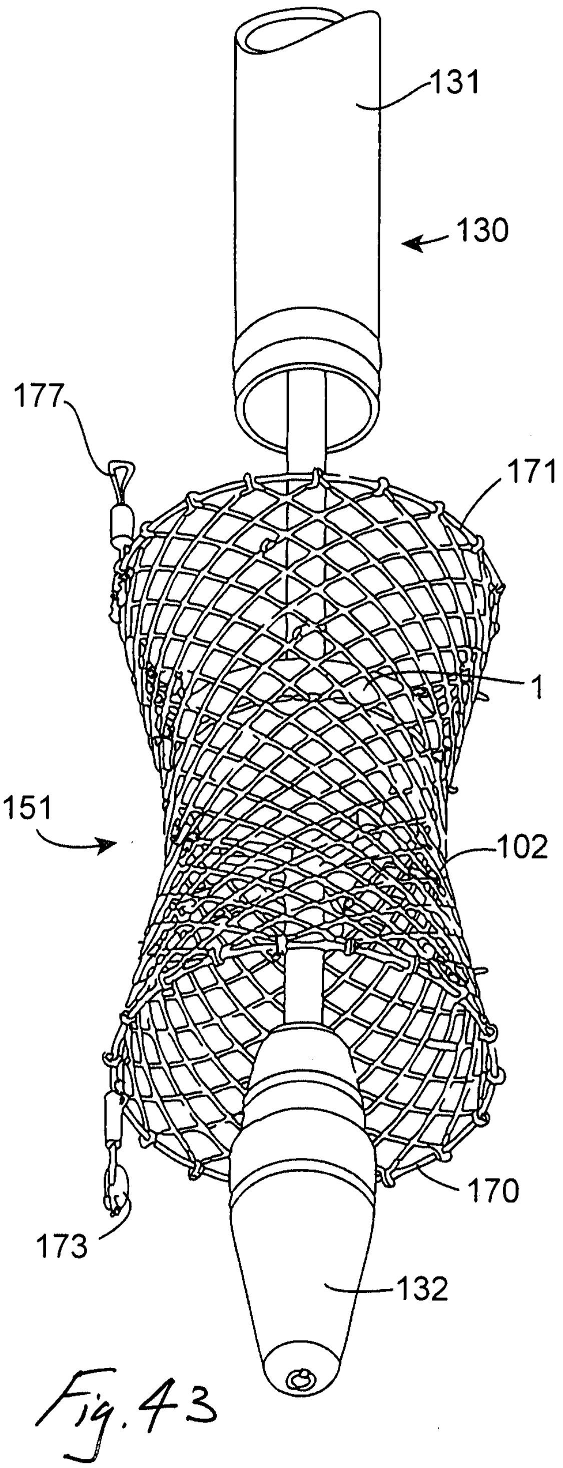


Fig. 43

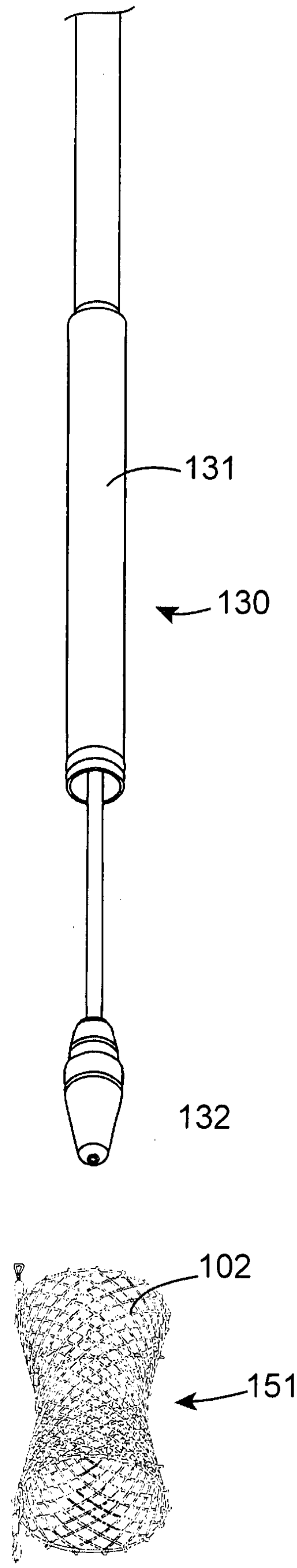


Fig. 44

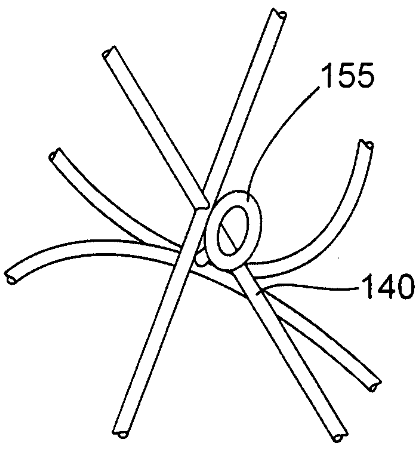


Fig. 45

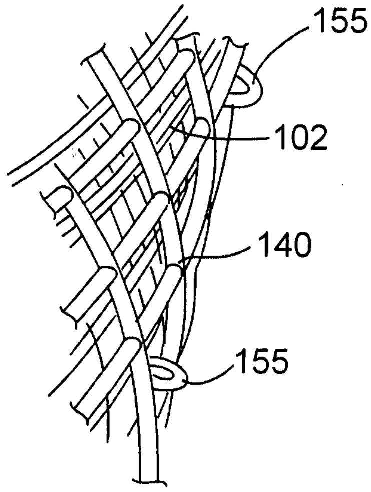


Fig. 46

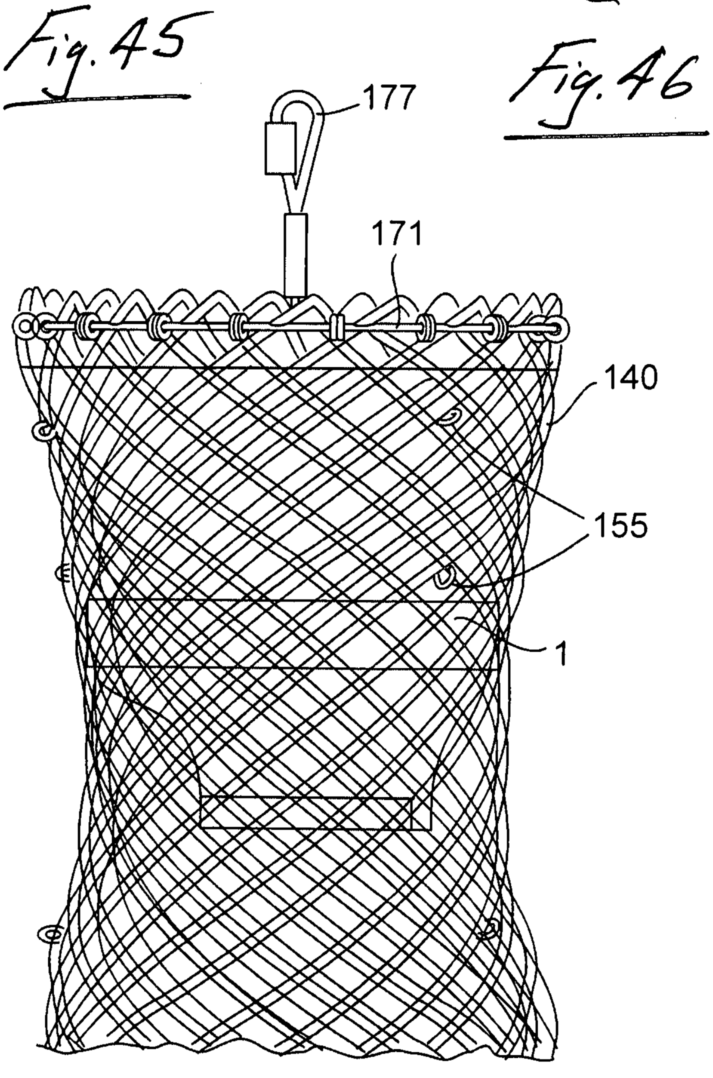


Fig. 47

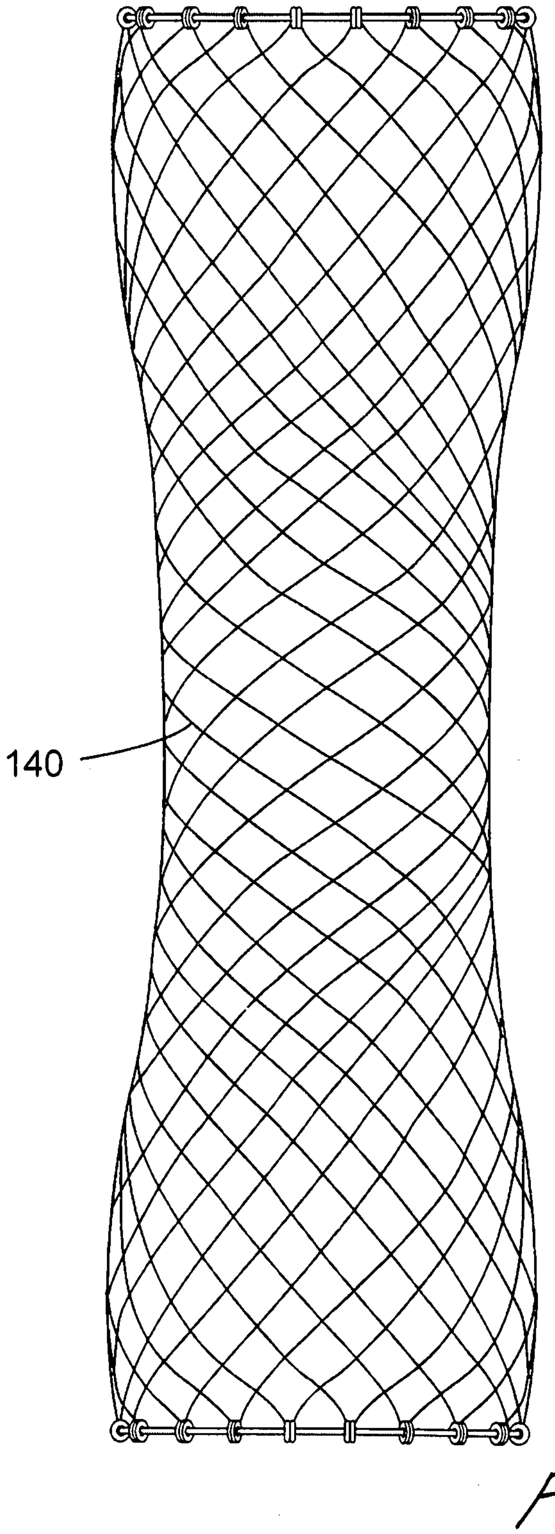


Fig.48

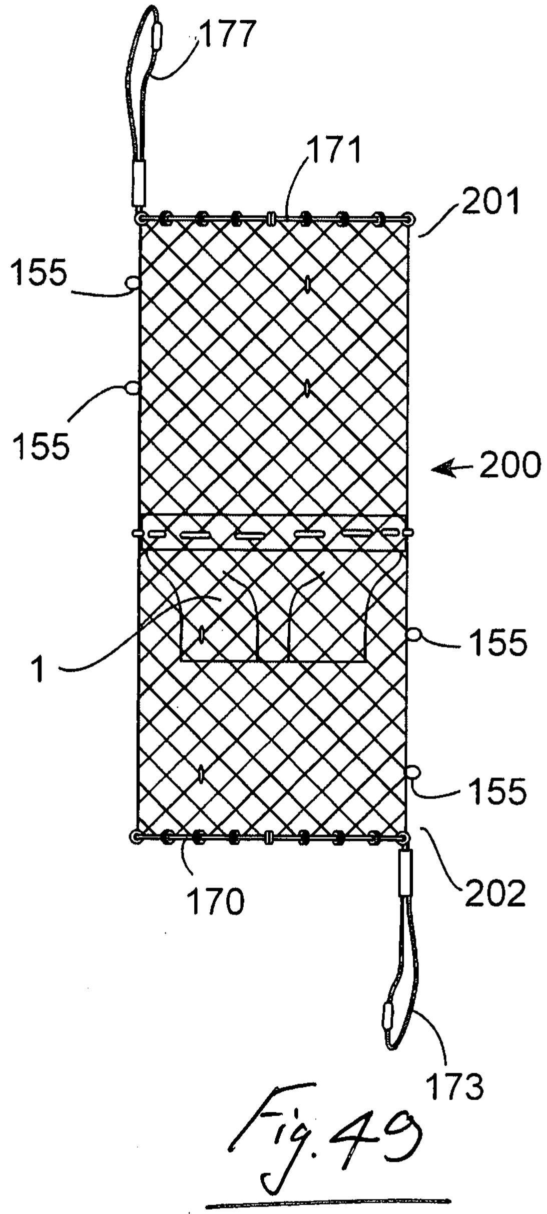


Fig.49

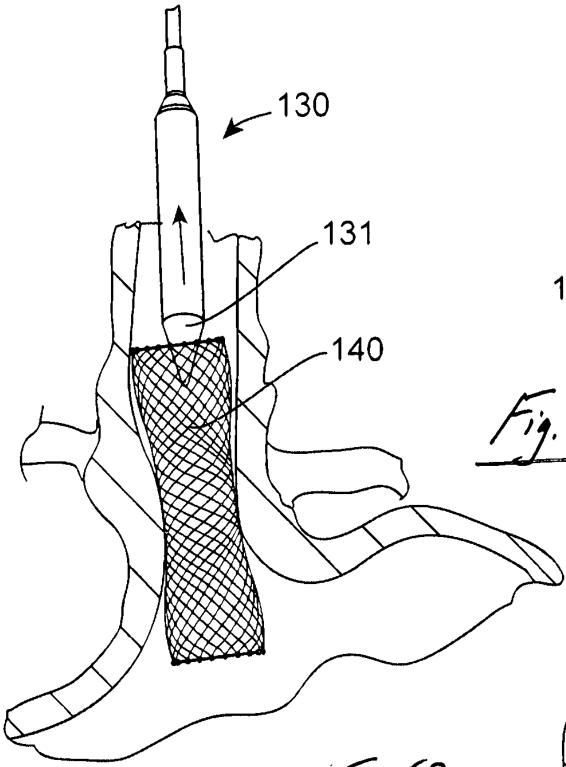


Fig. 53

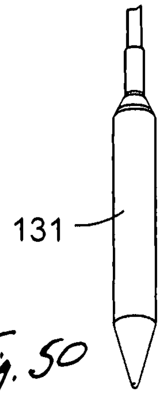


Fig. 50

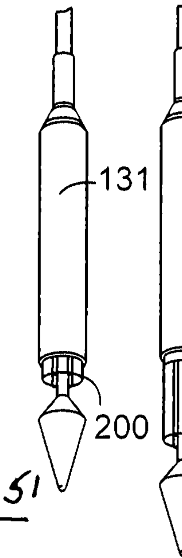


Fig. 51

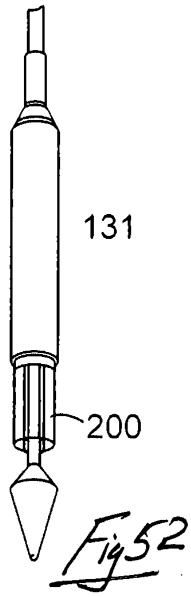


Fig. 52

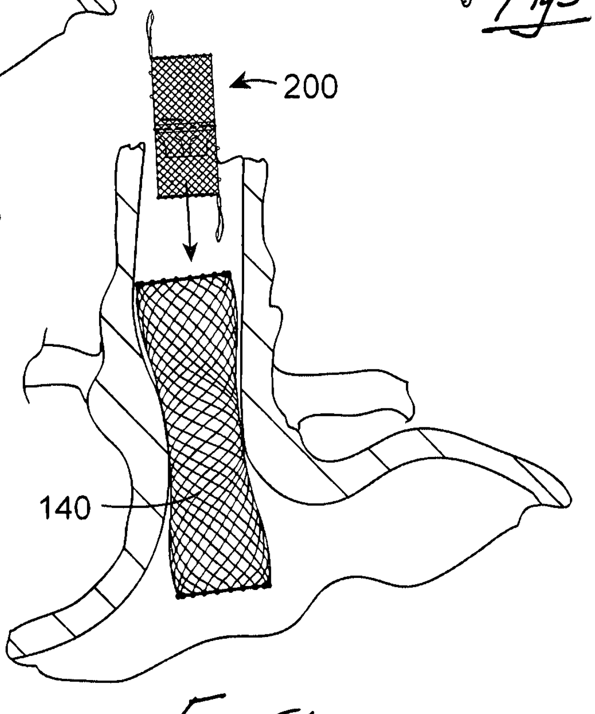


Fig. 54

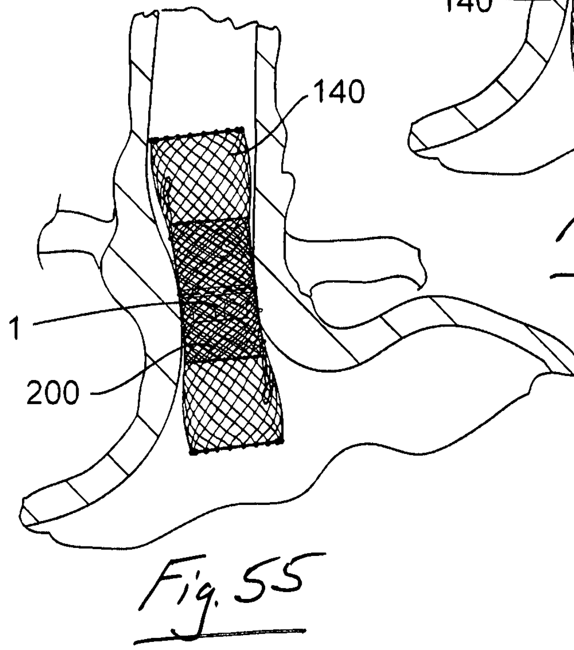


Fig. 55

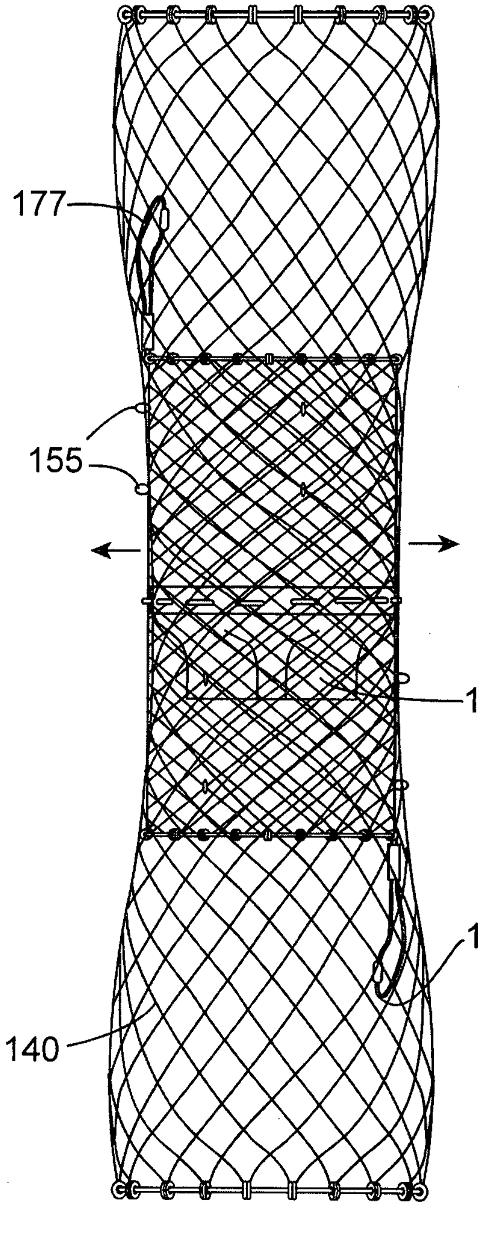


Fig. 56

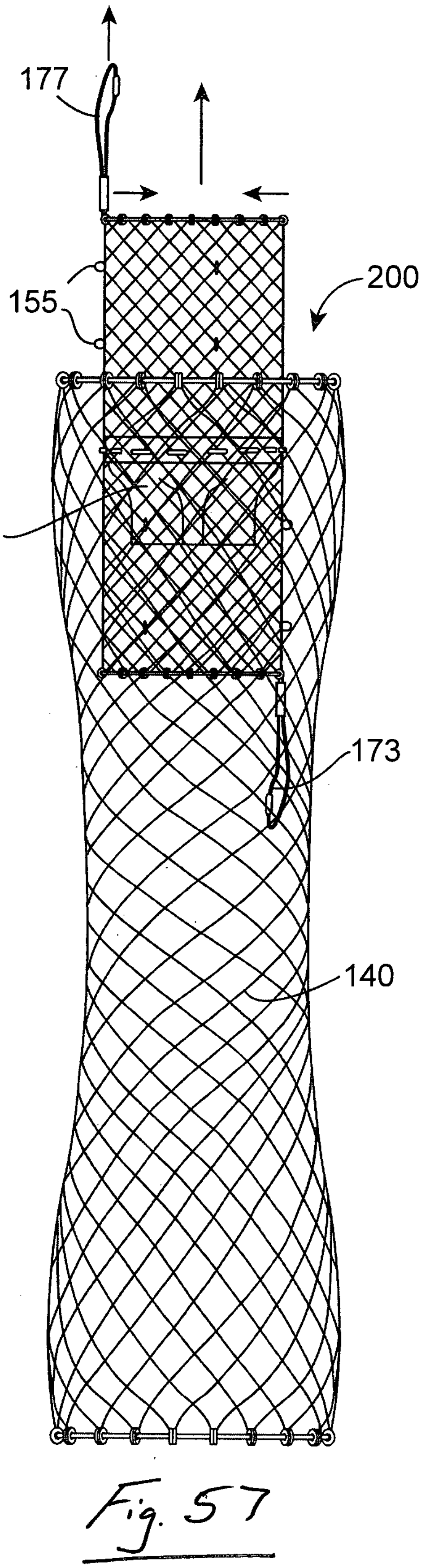


Fig. 57

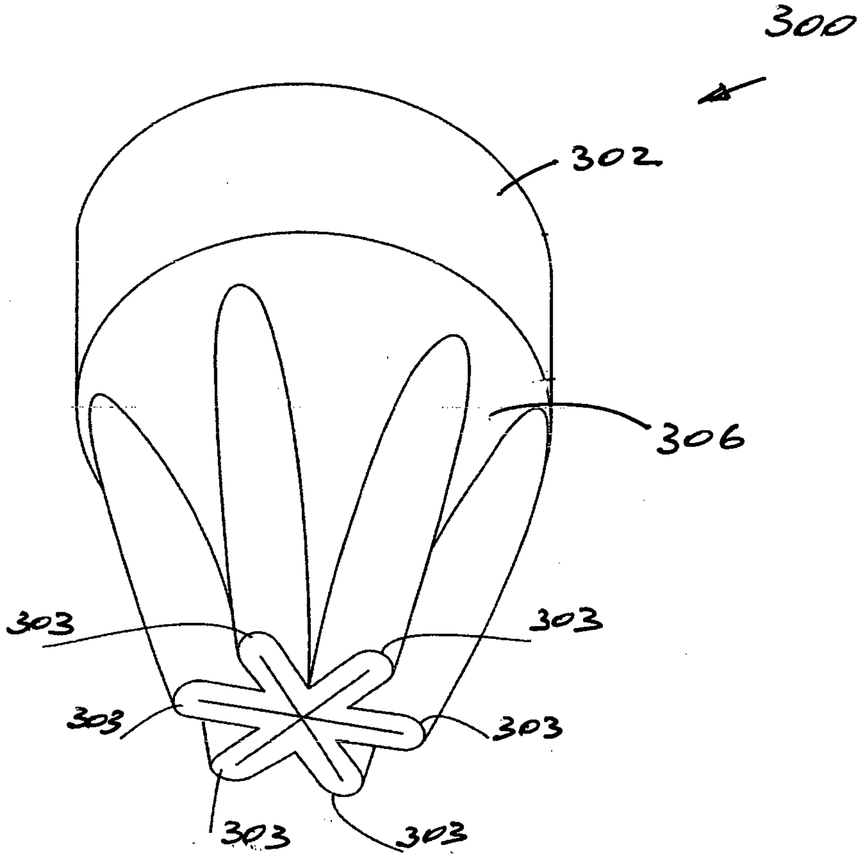


Fig. 58

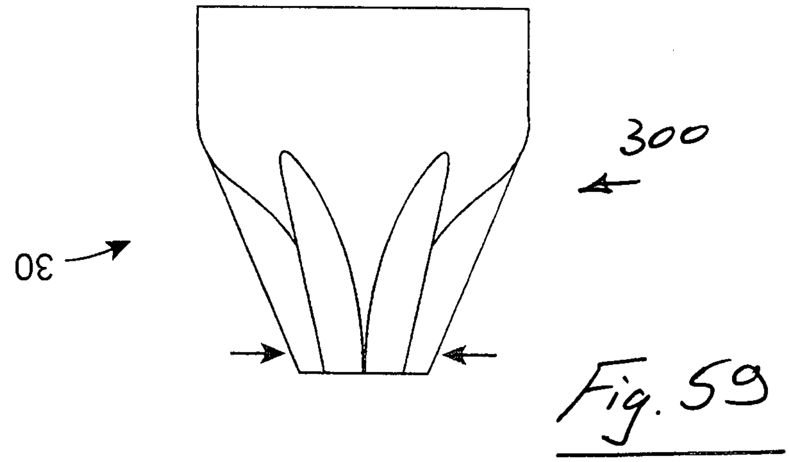


Fig. 59

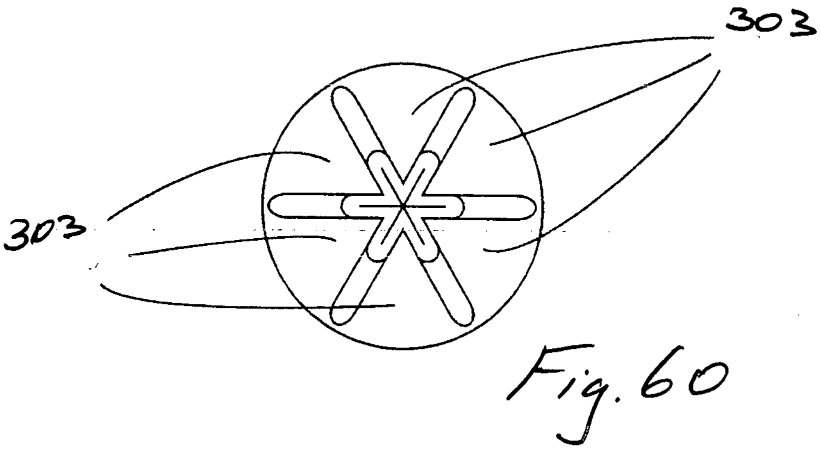


Fig. 60

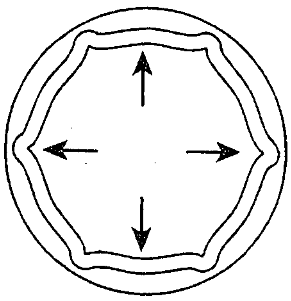

Fig. 61

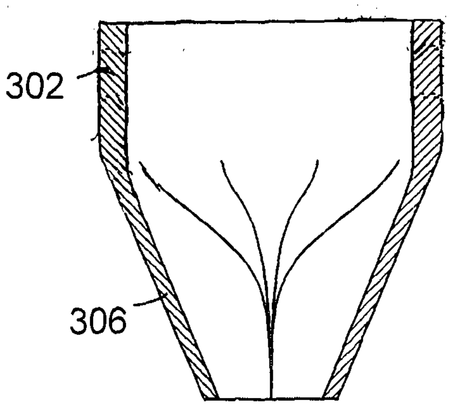

Fig.62

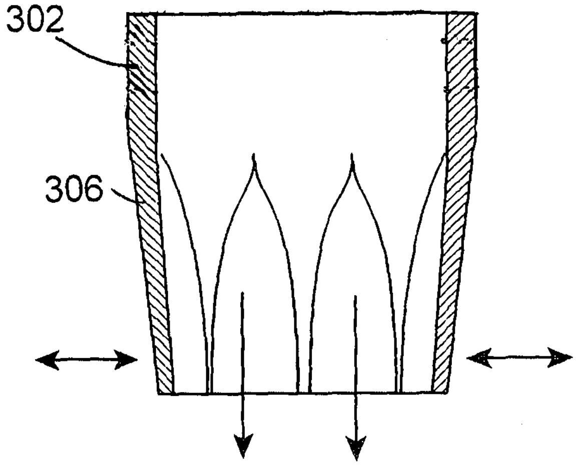

Fig.64

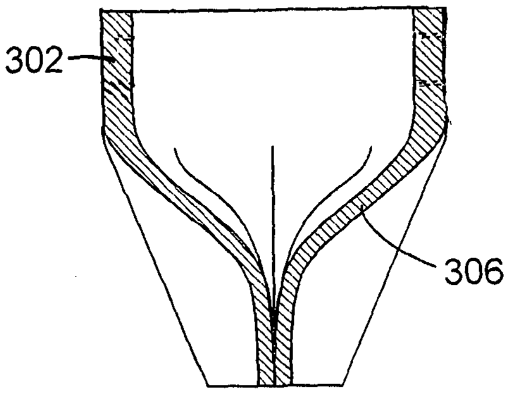

Fig.63

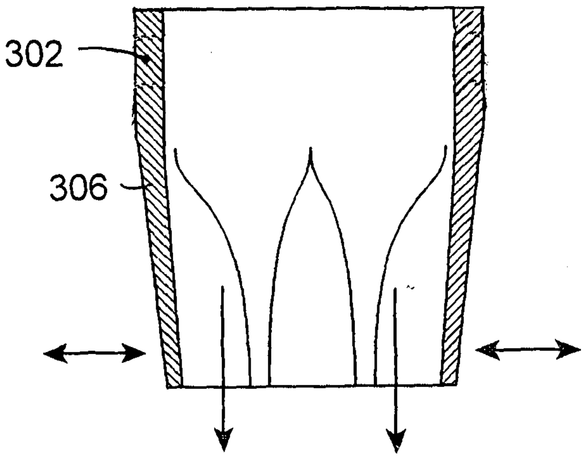

Fig.65

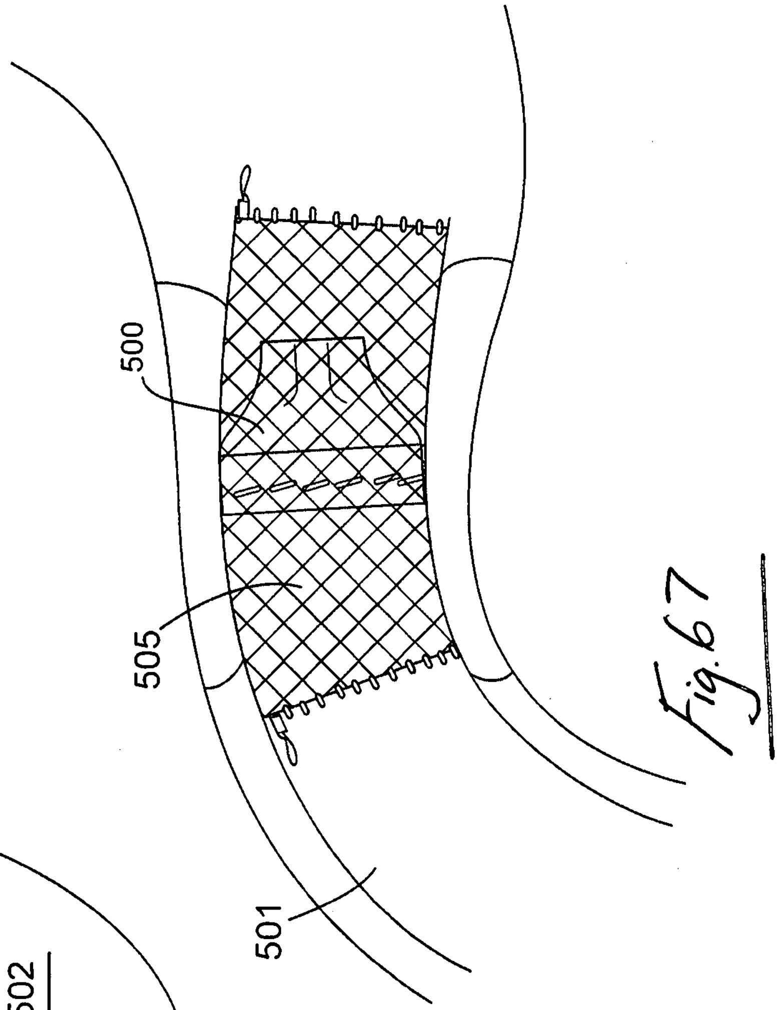


Fig.67

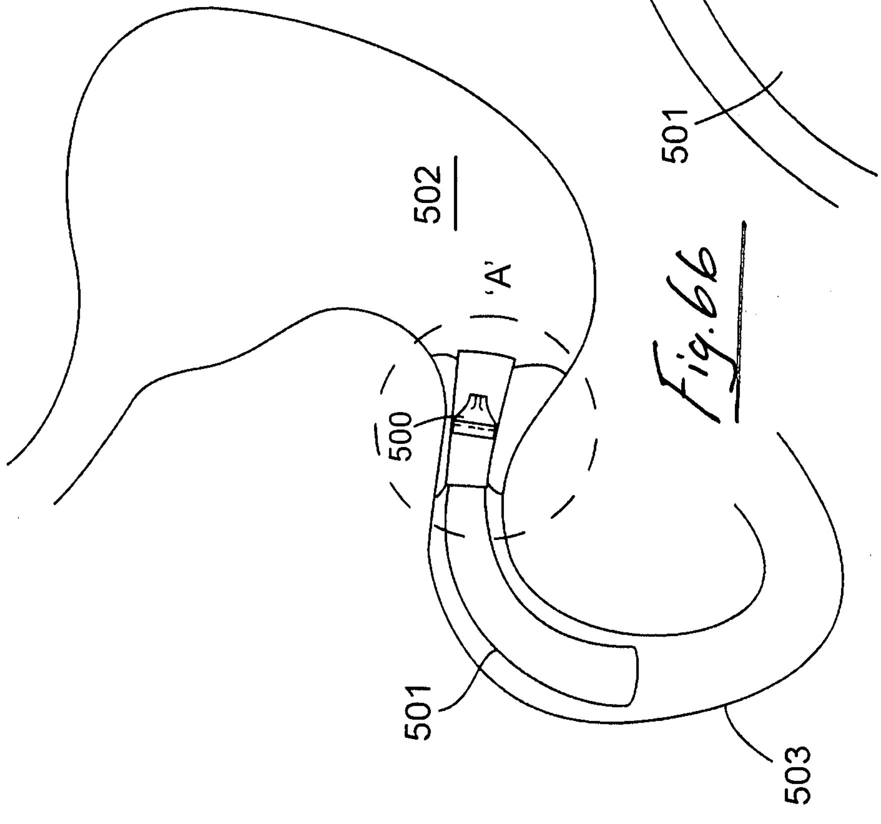


Fig.66

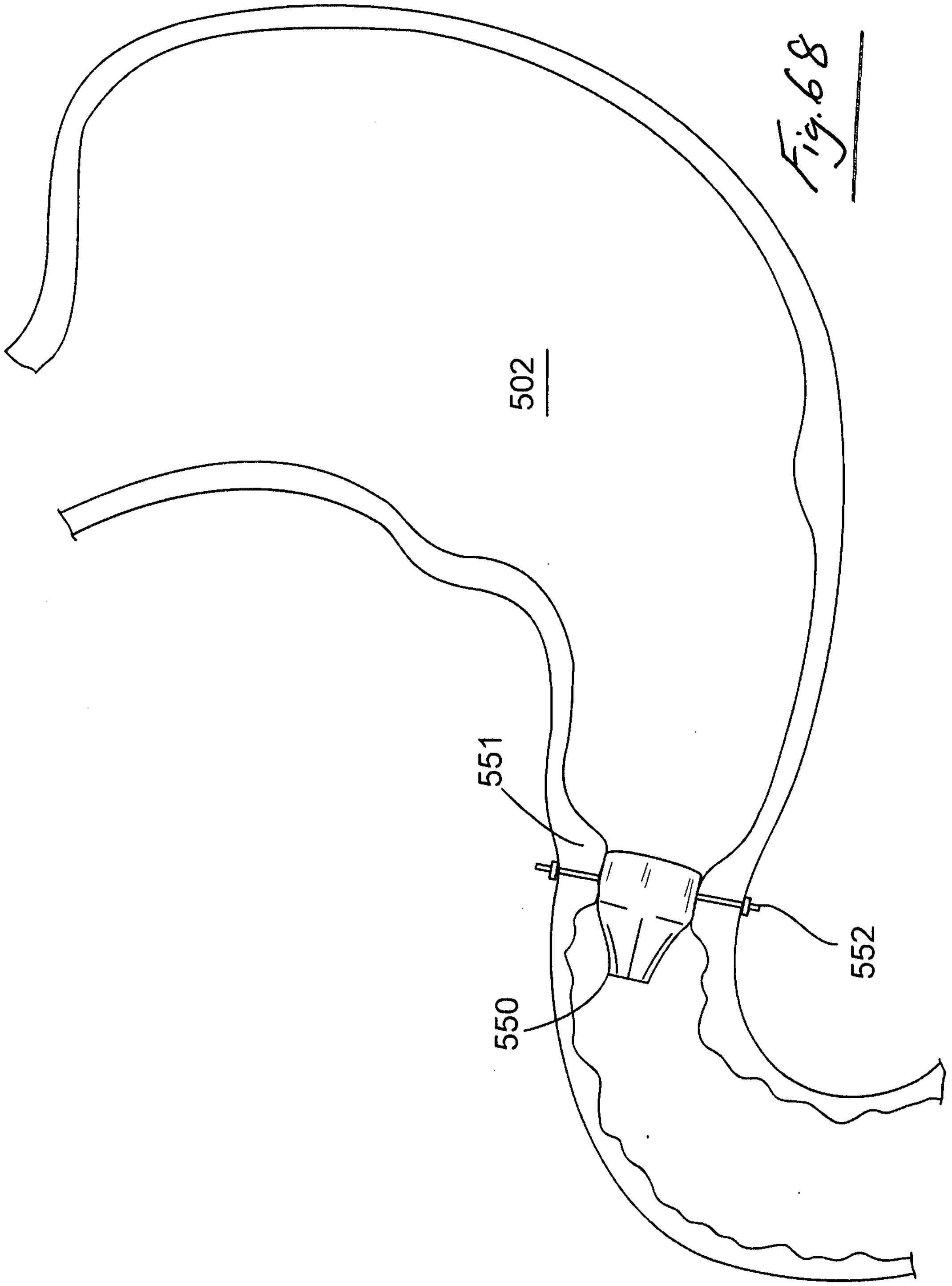


Fig. 68

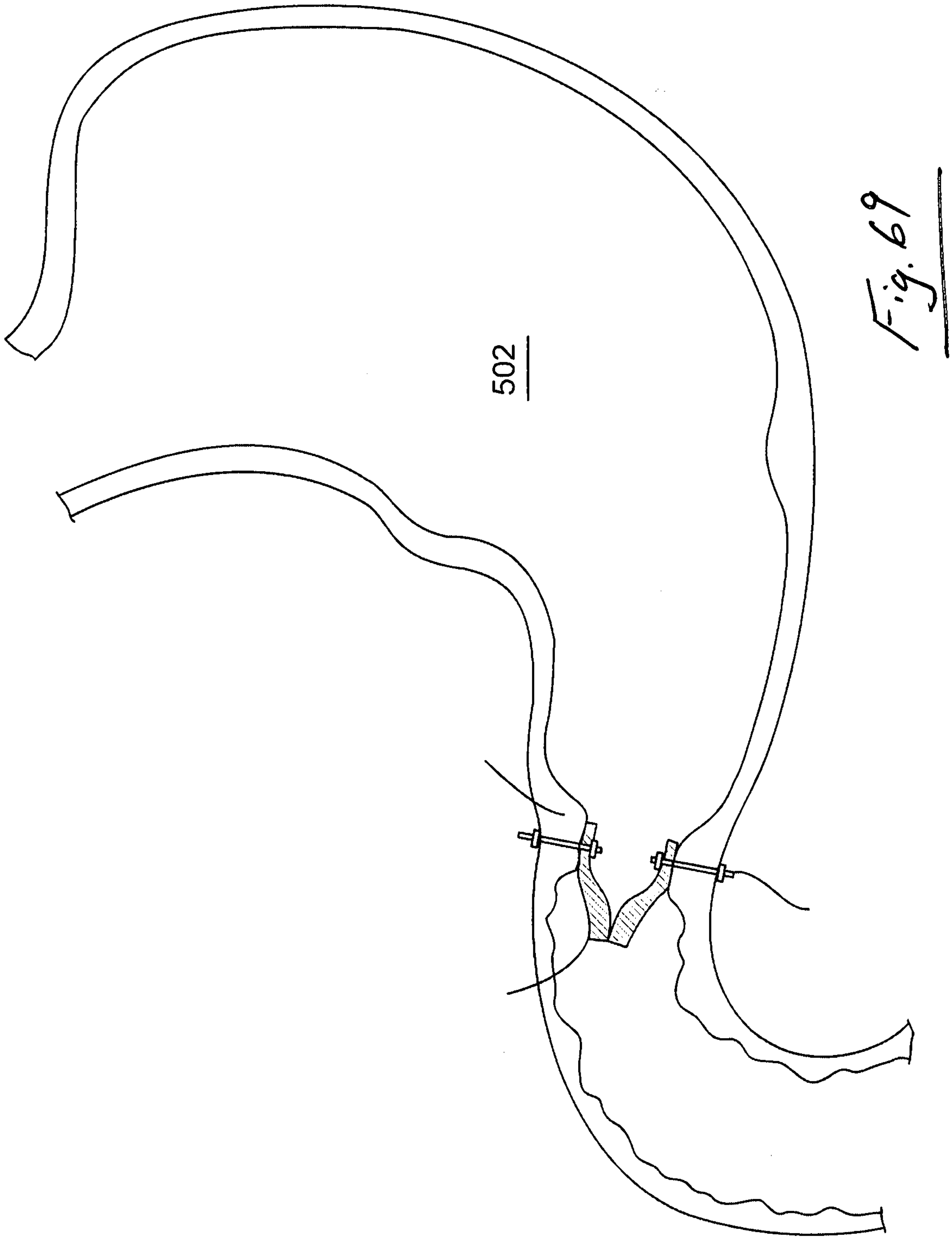


Fig. 69

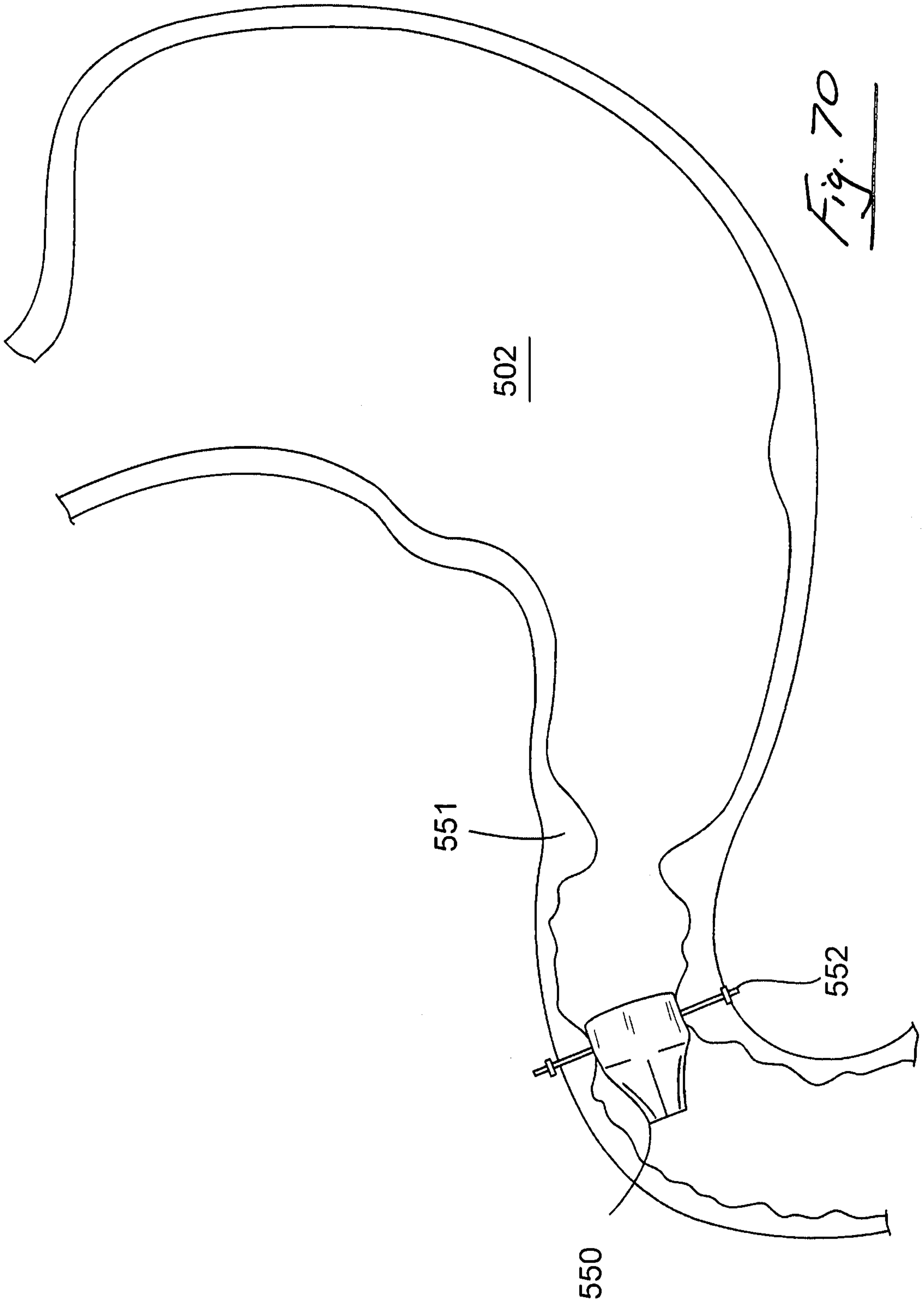


Fig. 70

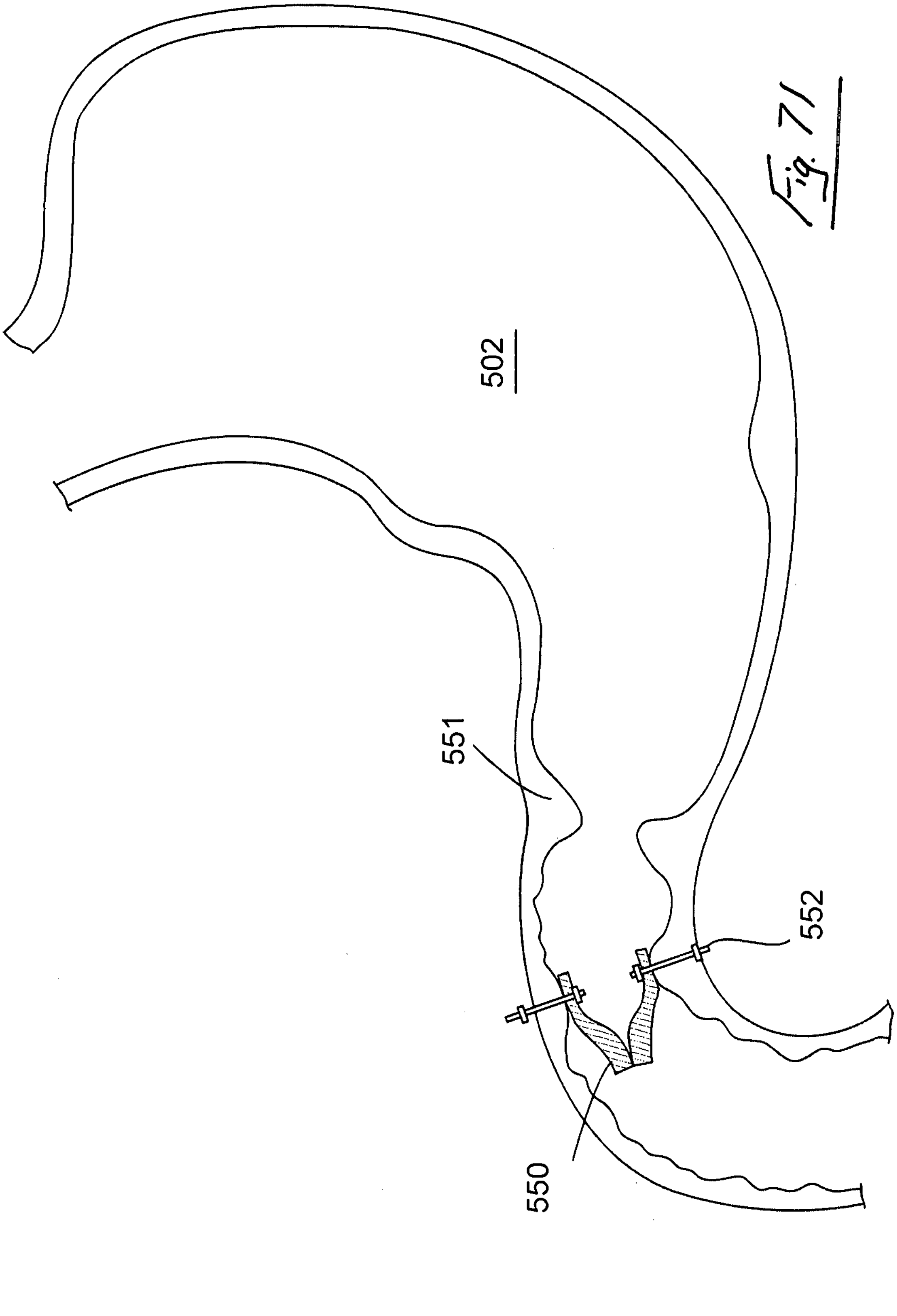


Fig. 71

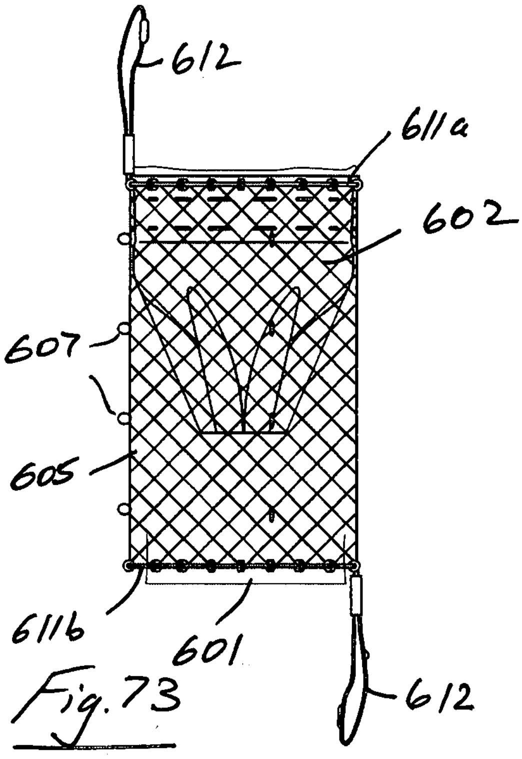


Fig.73

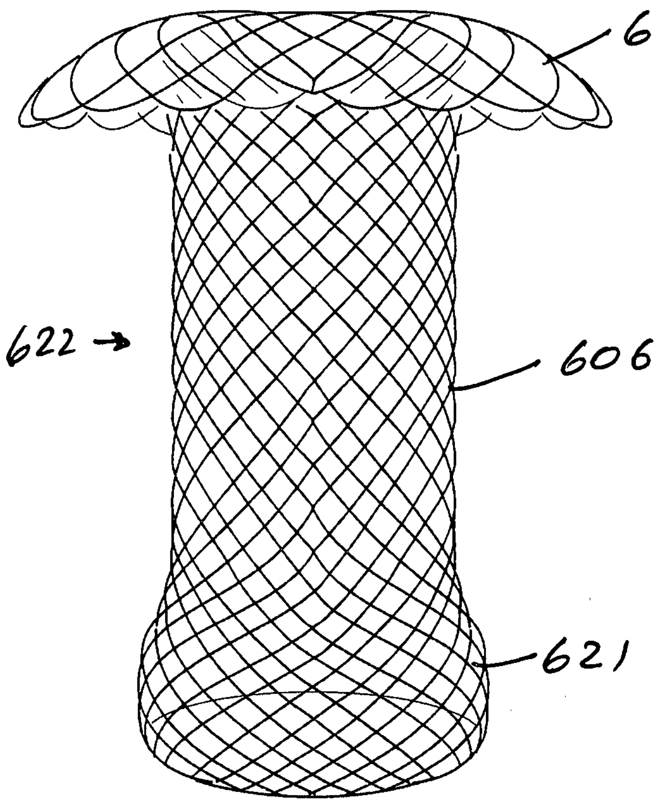


Fig.72

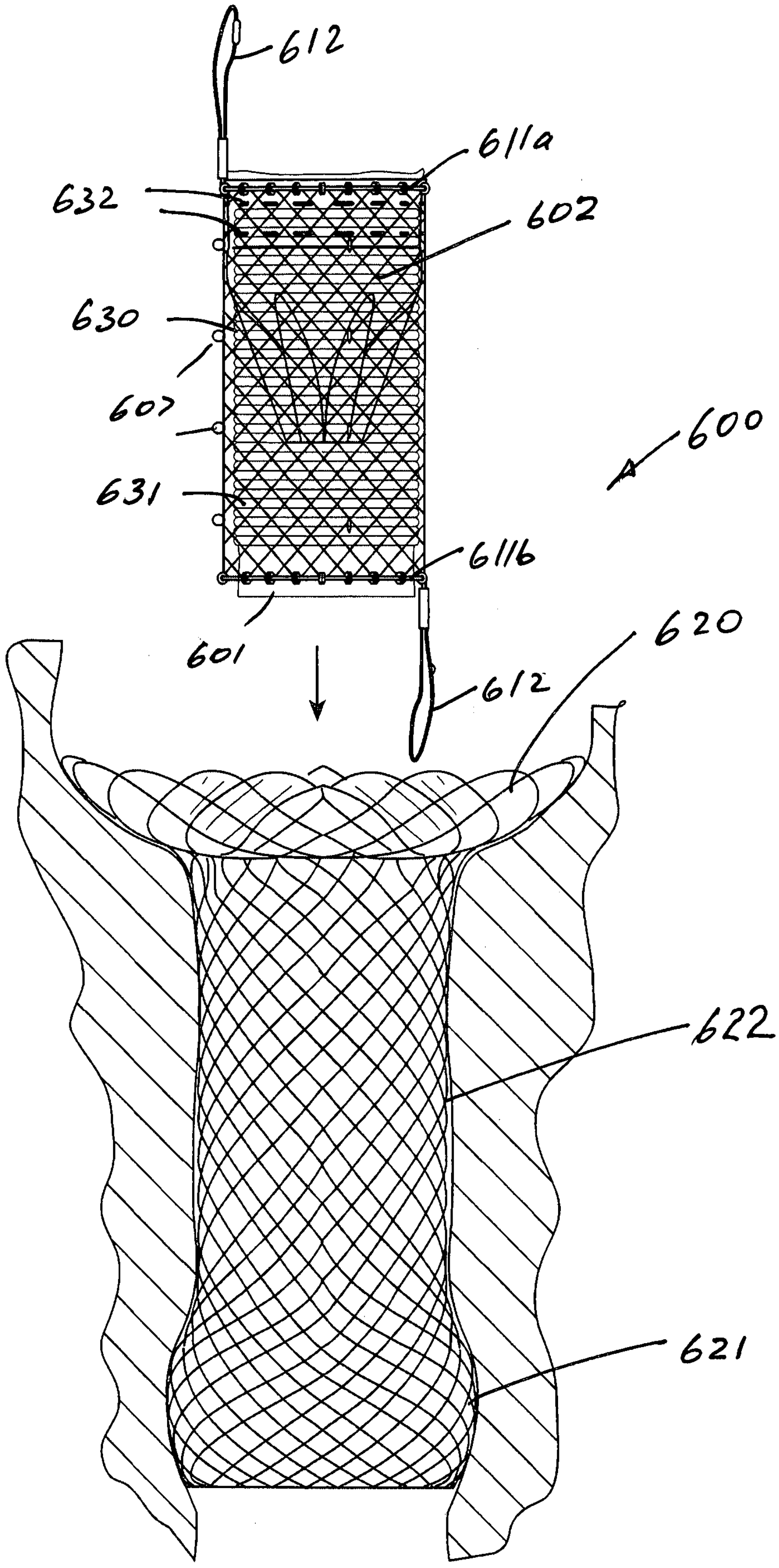


Fig. 74

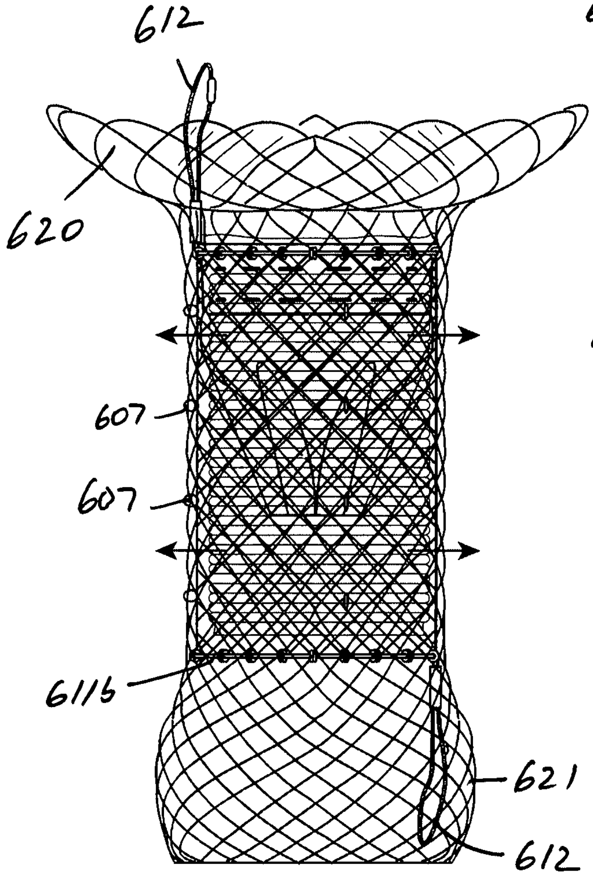


Fig. 75

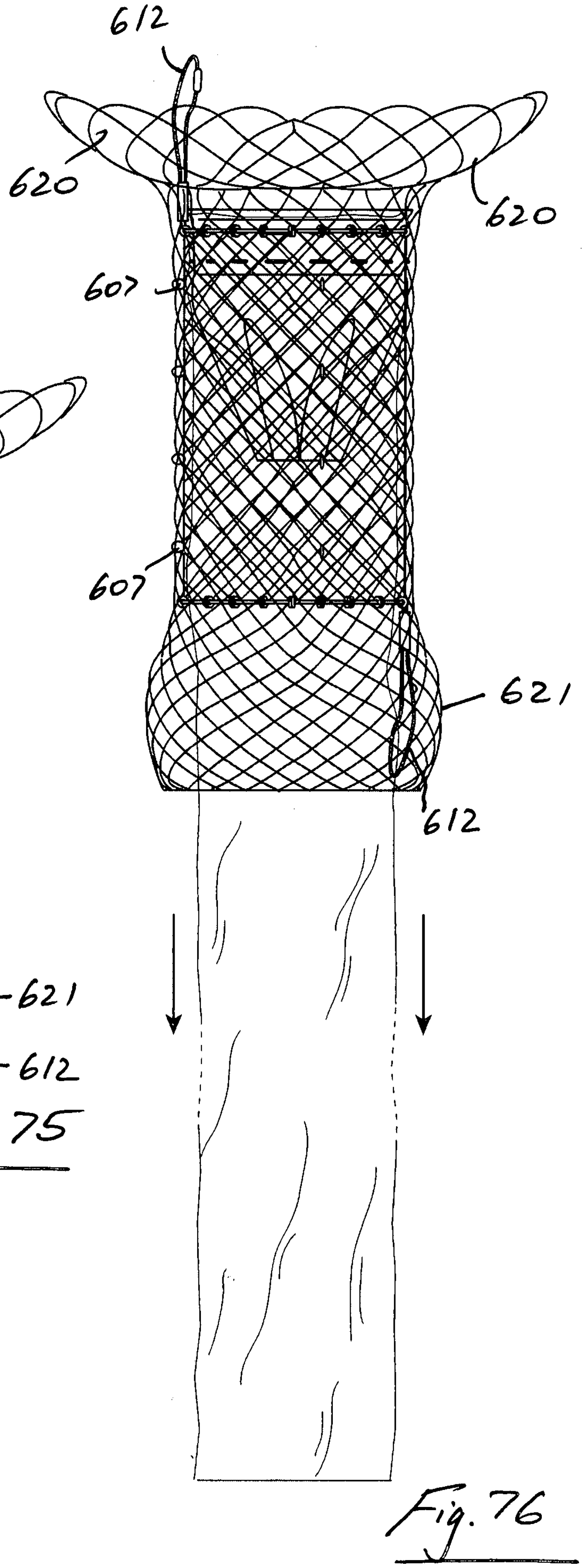


Fig. 76

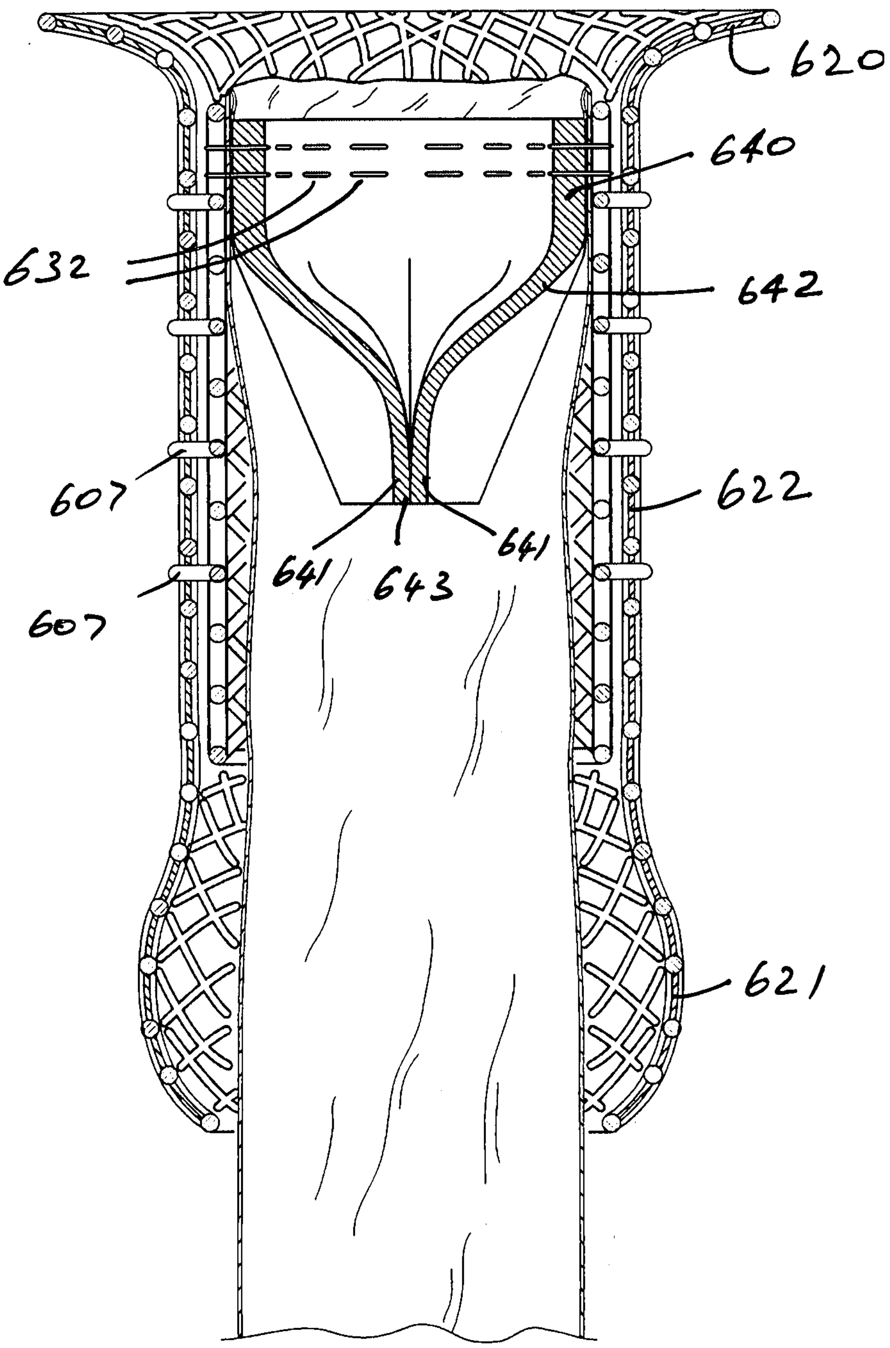


Fig. 77

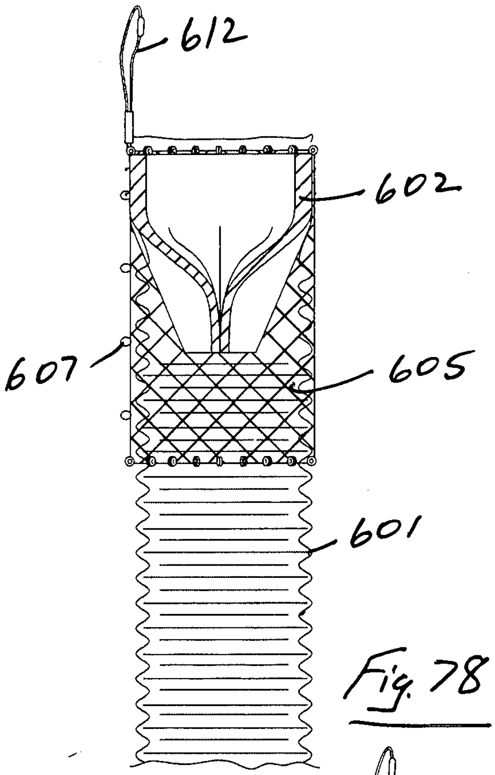


Fig. 78

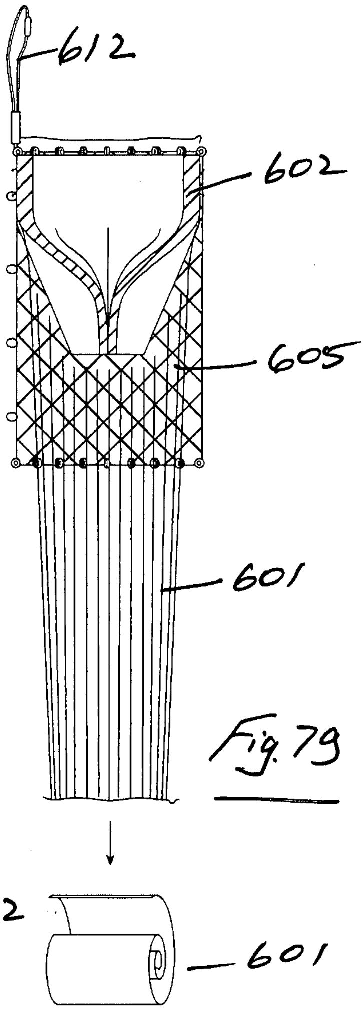


Fig. 79

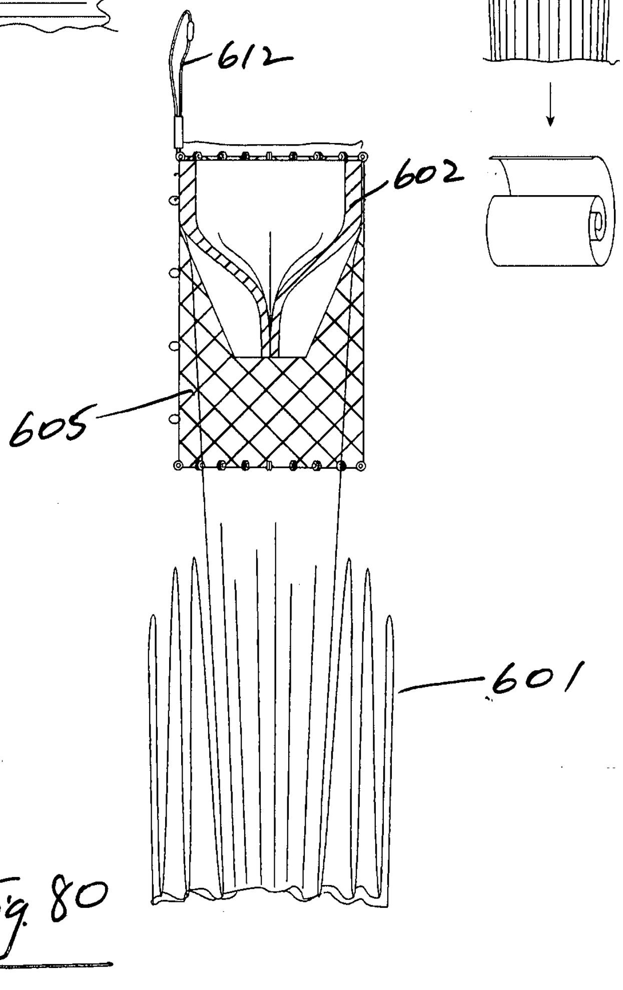


Fig. 80

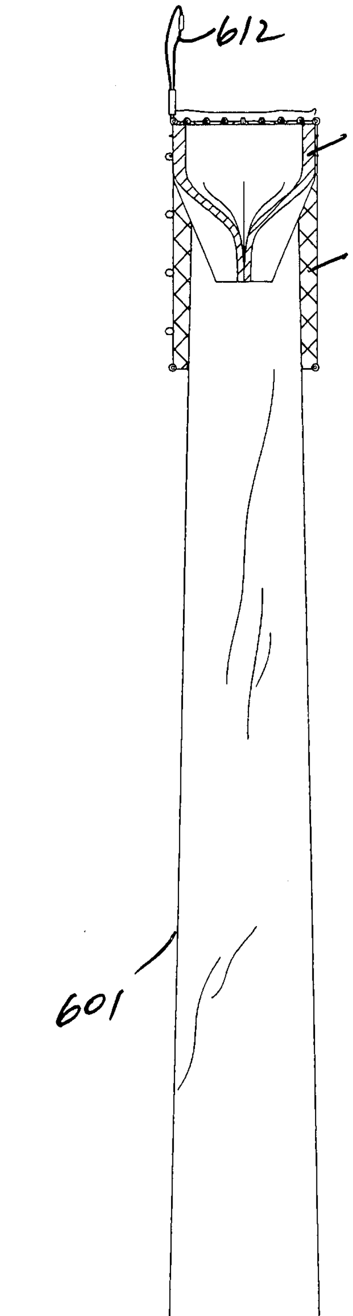


Fig.81

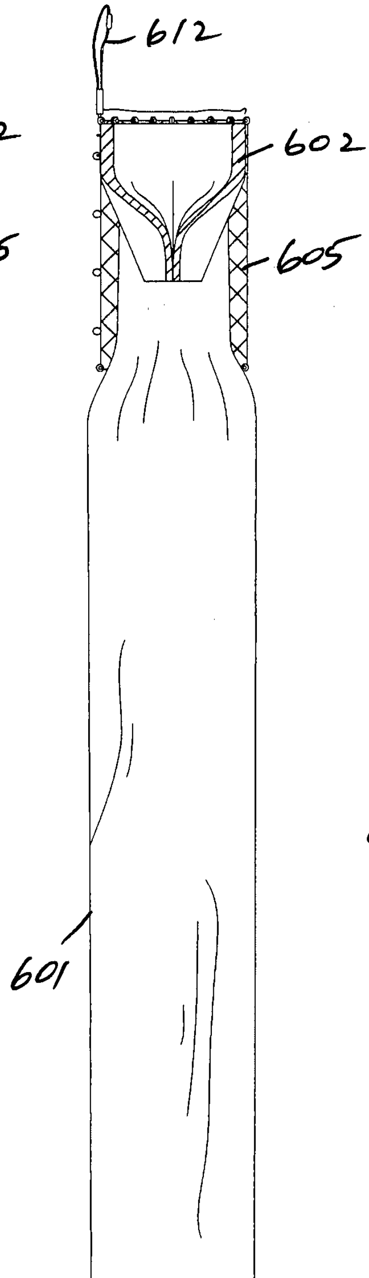


Fig.82

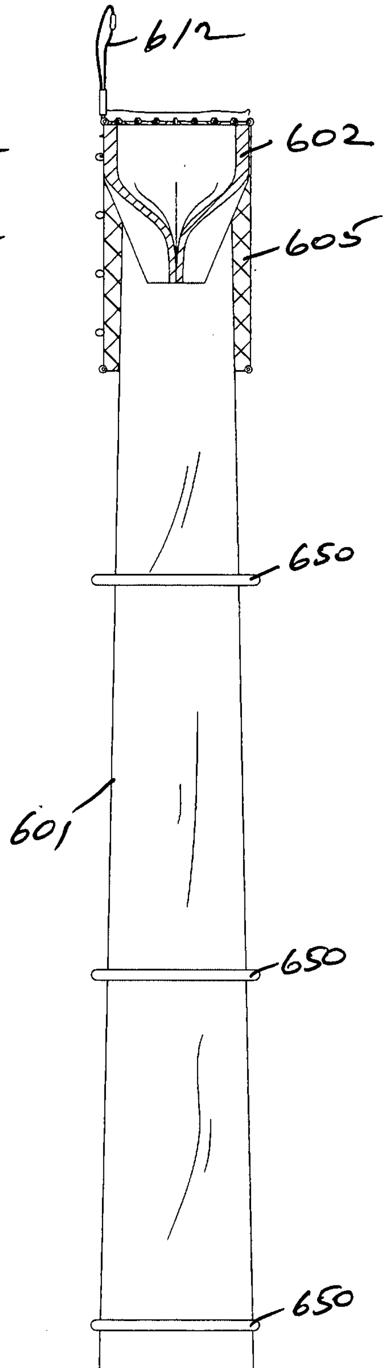


Fig.83

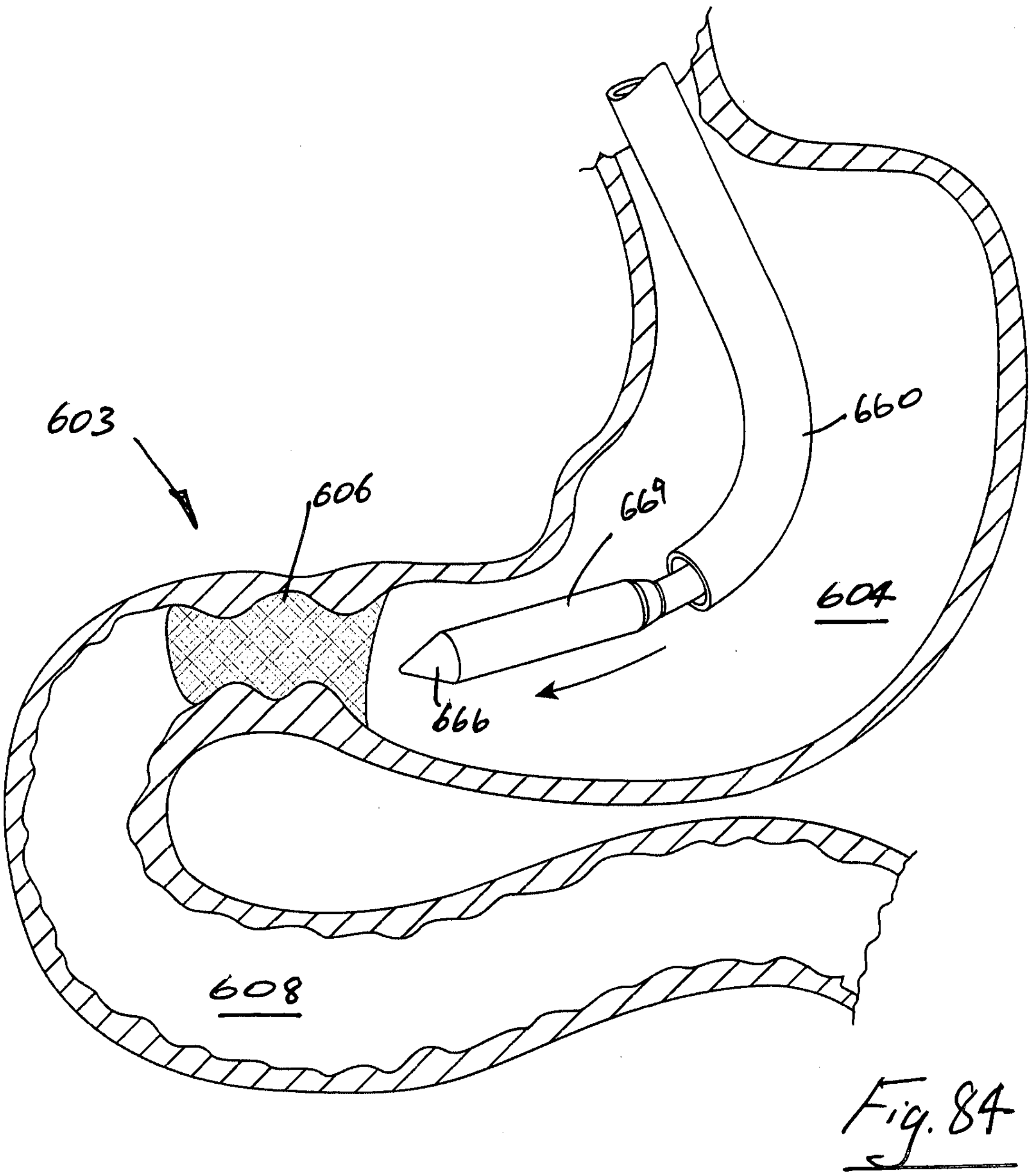


Fig. 84

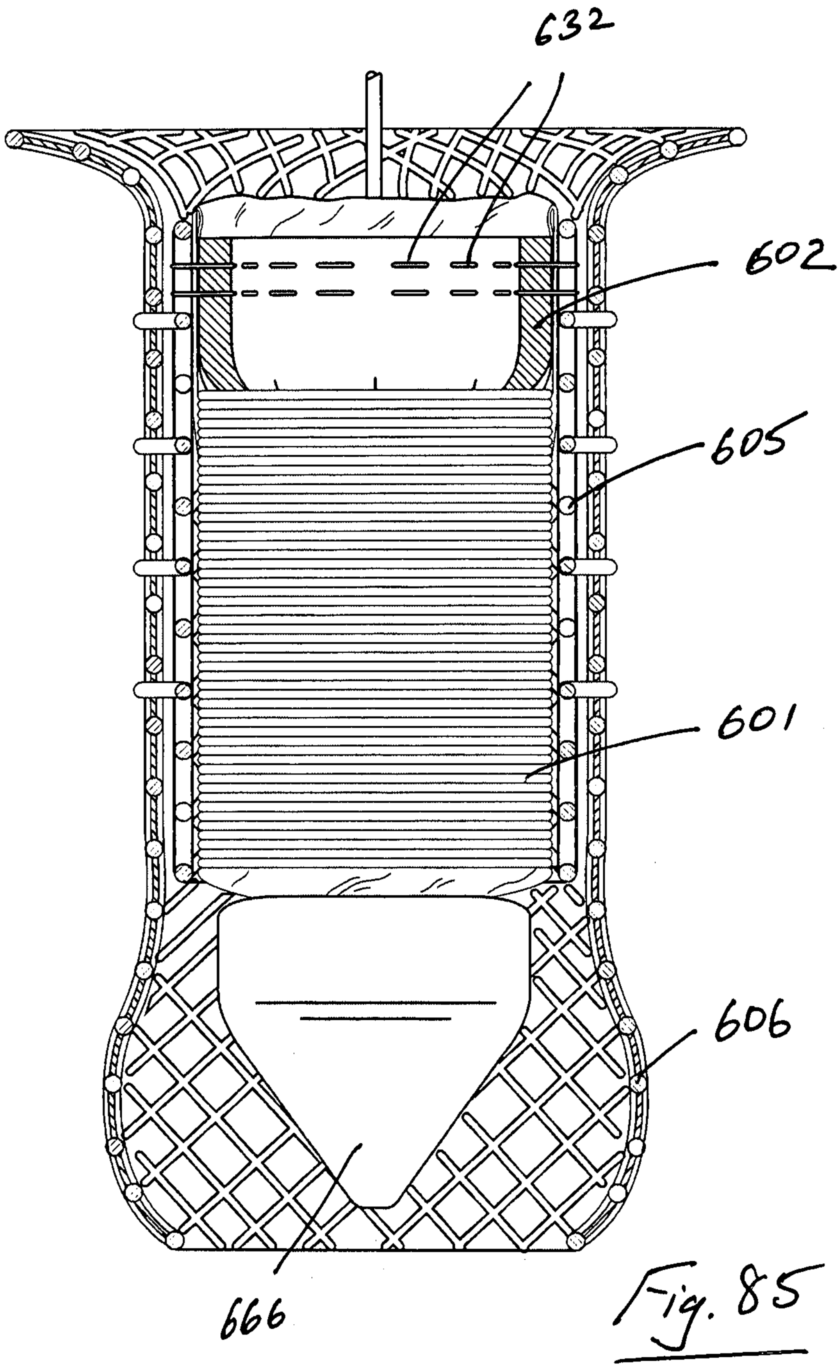


Fig. 85

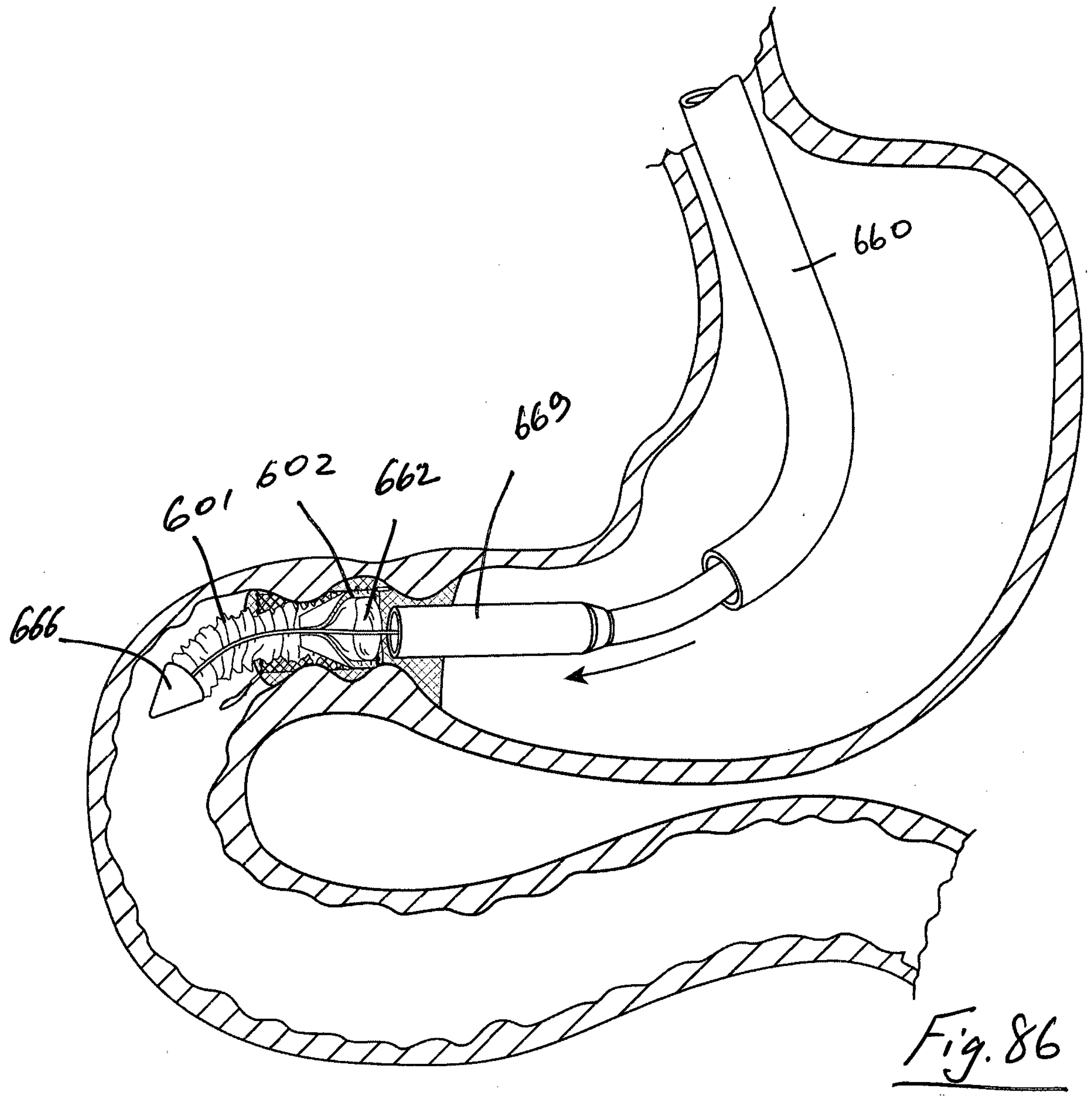


Fig. 86

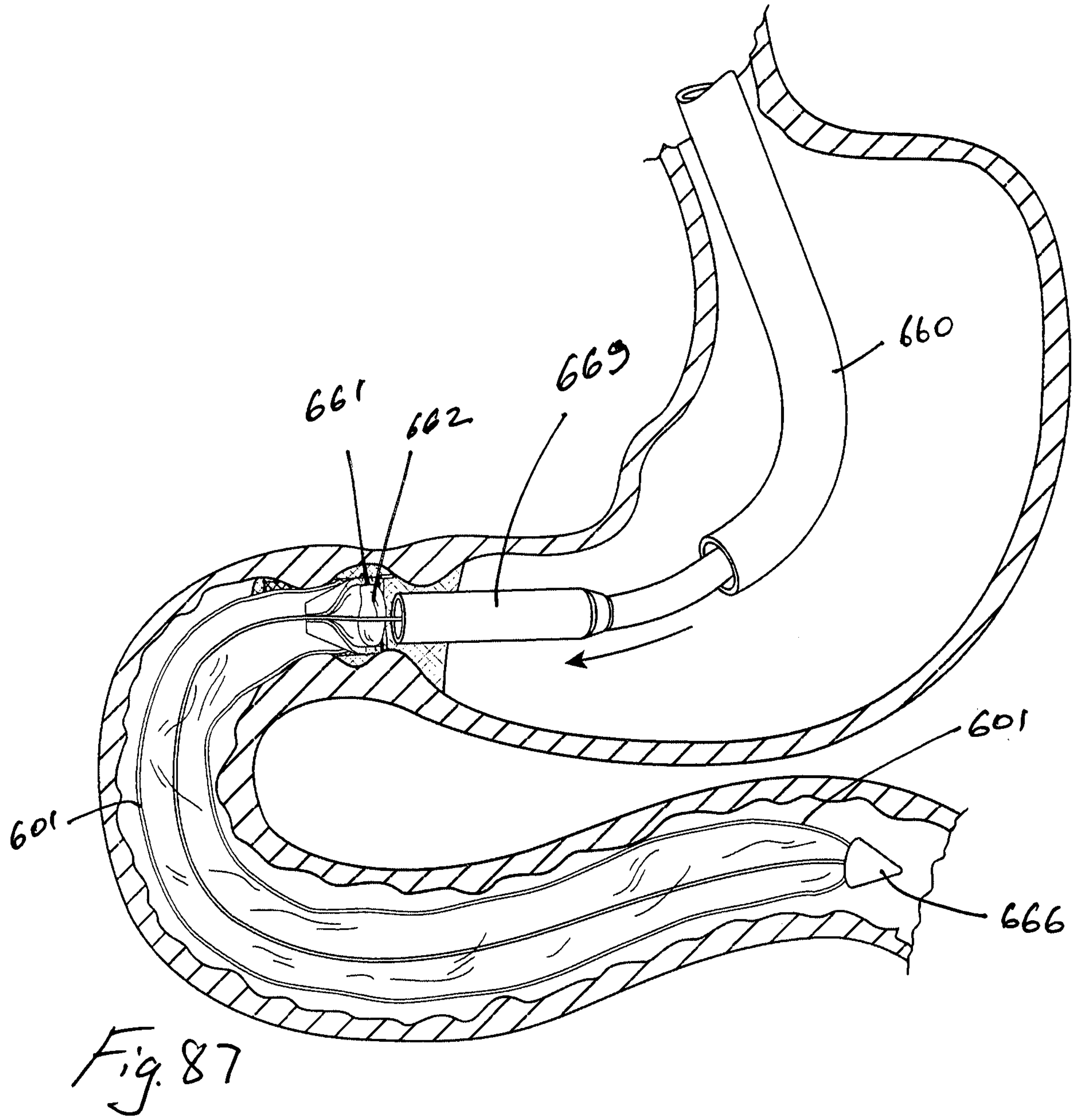


Fig. 87

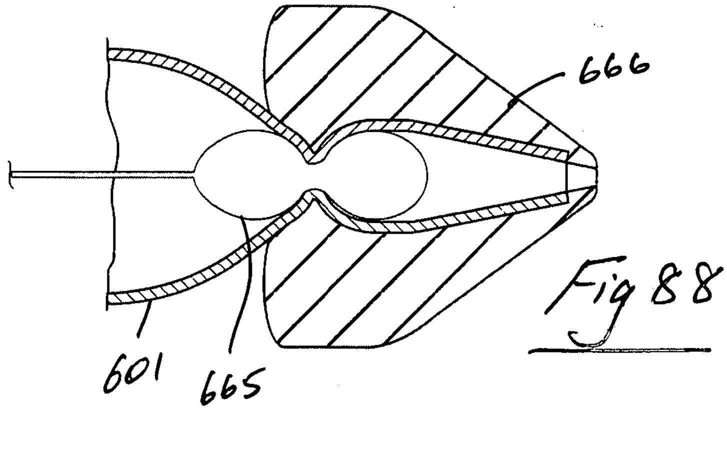


Fig 88

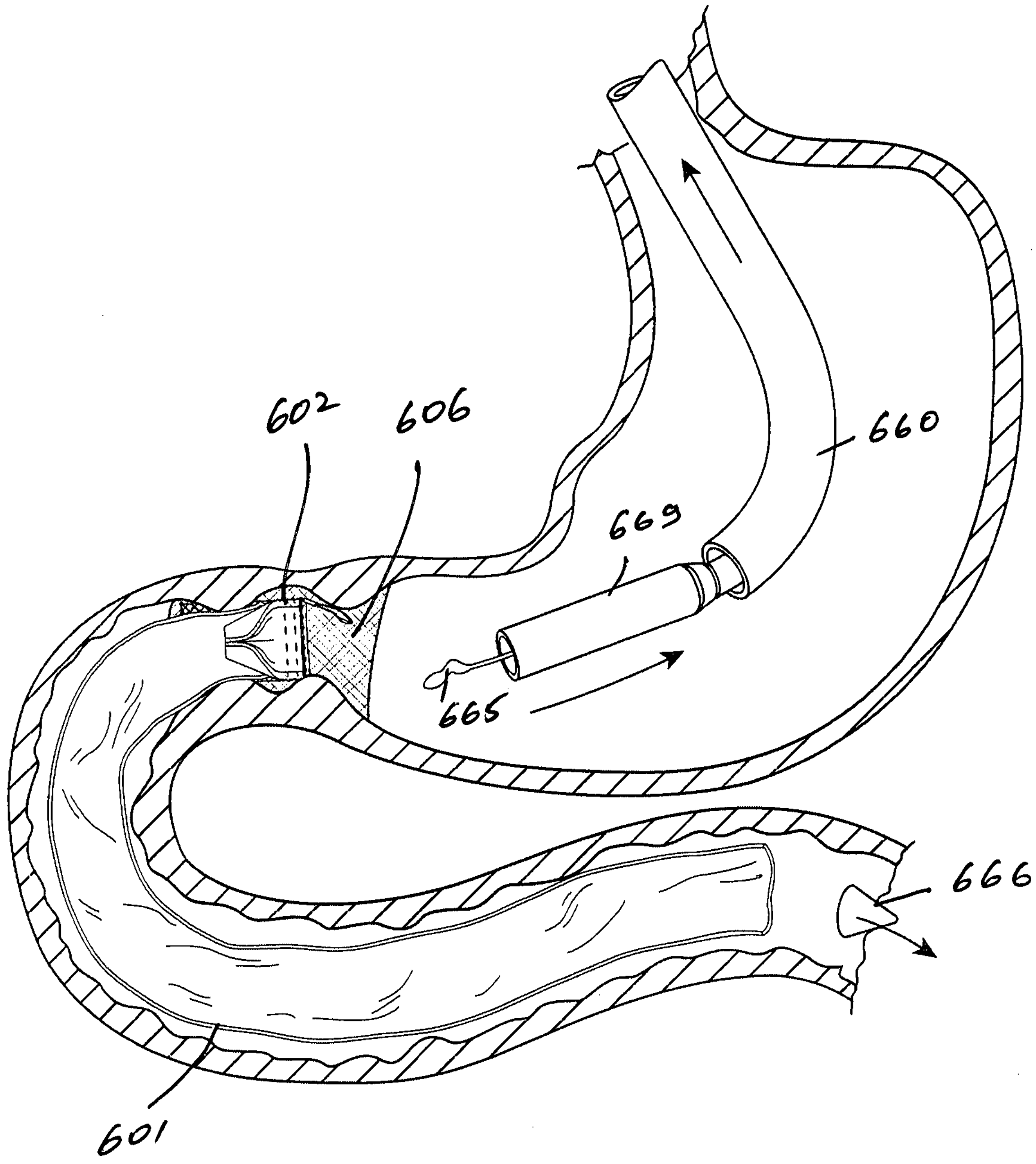


Fig. 89

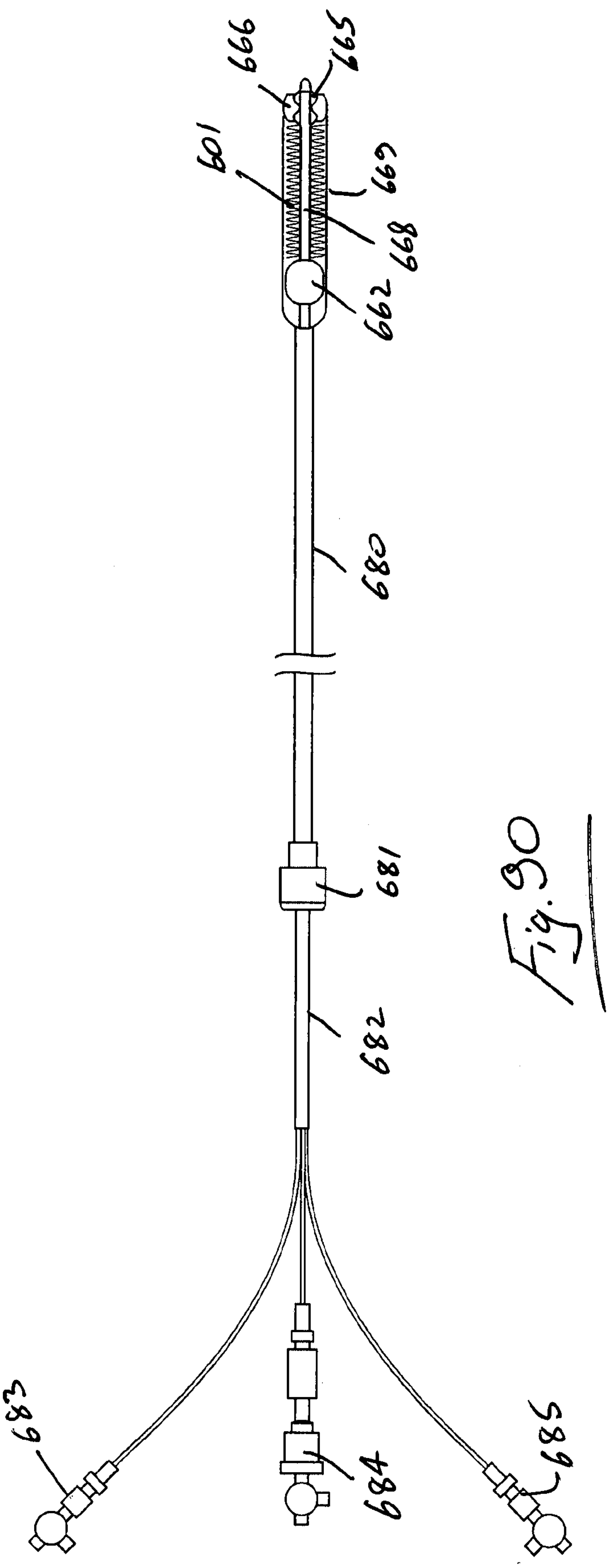


Fig. 90

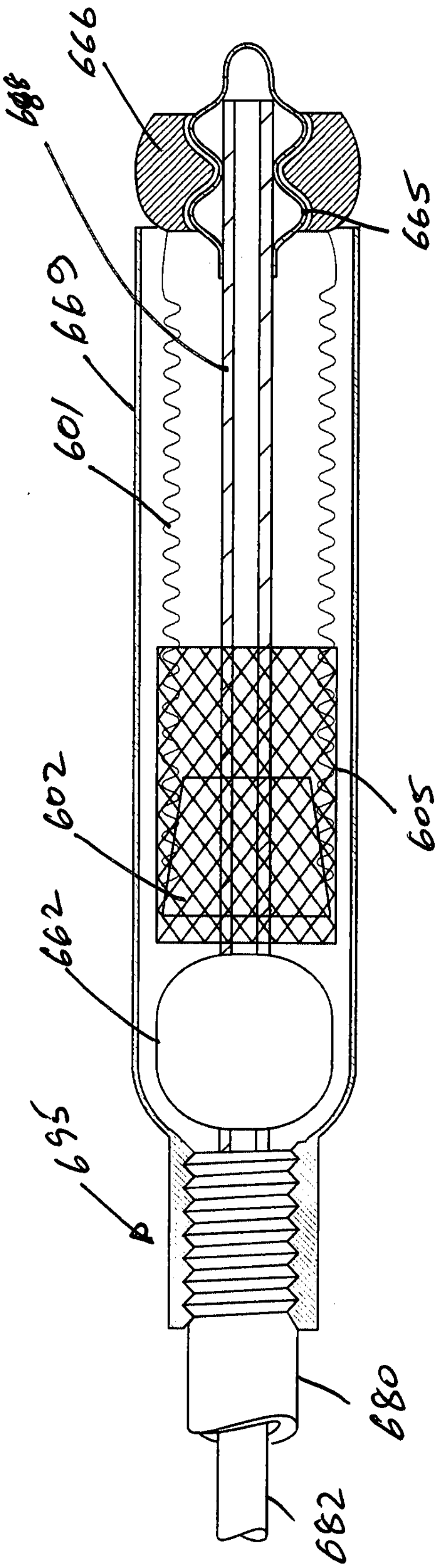


Fig. 91

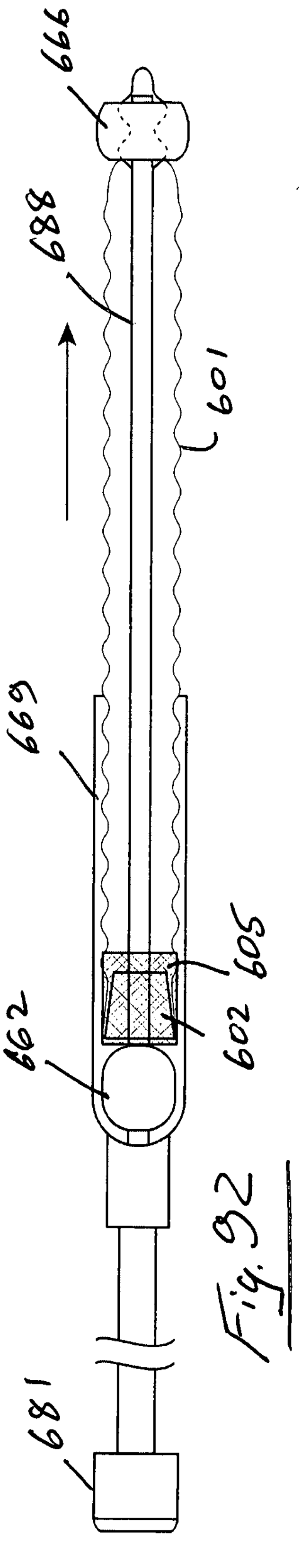


Fig. 92

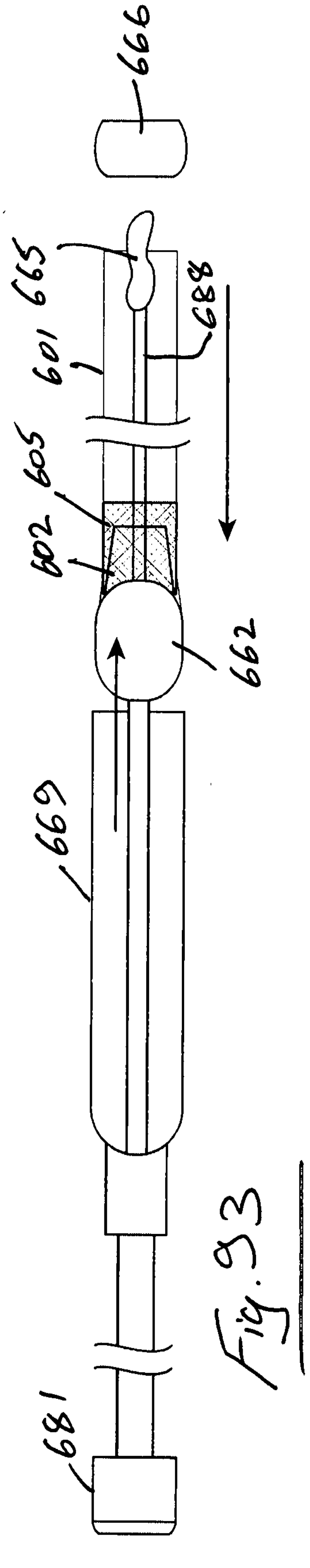


Fig. 93

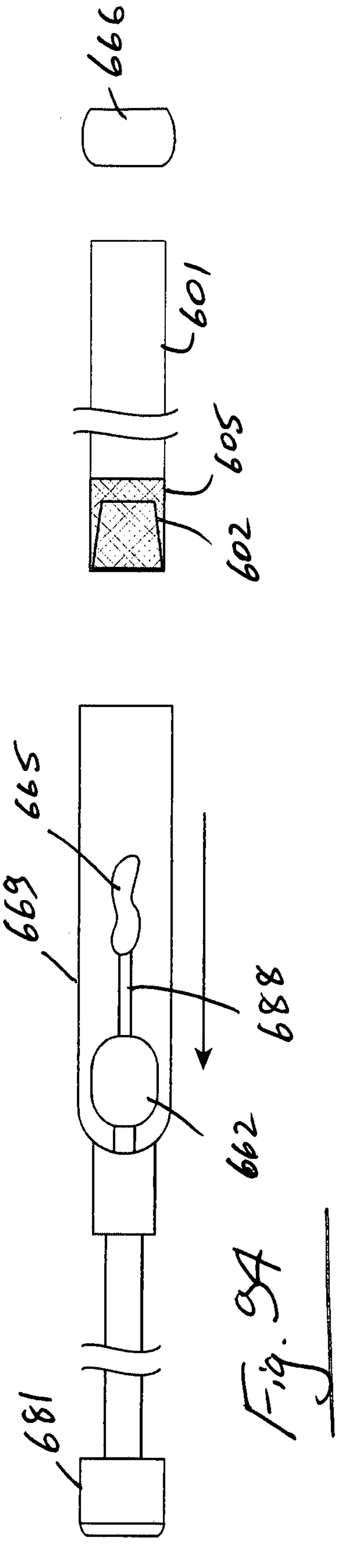


Fig. 94

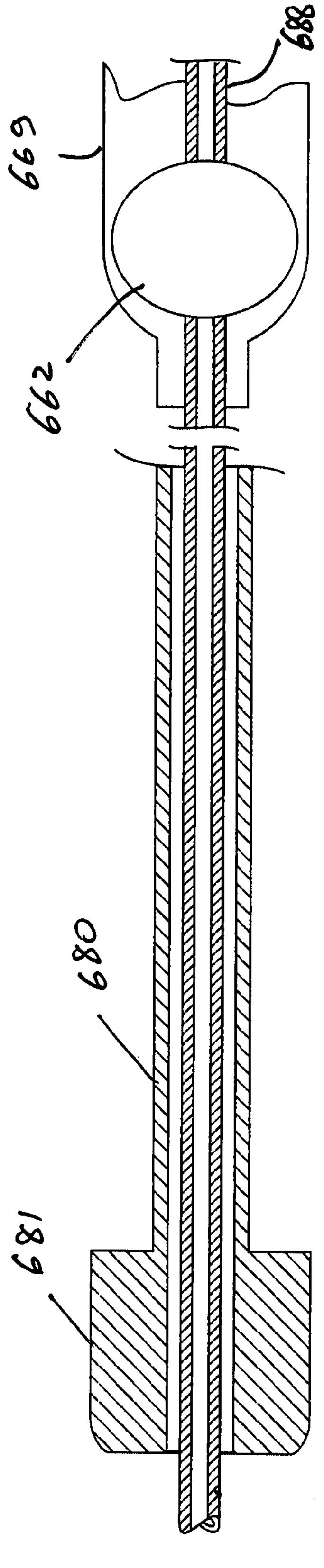


Fig. 95

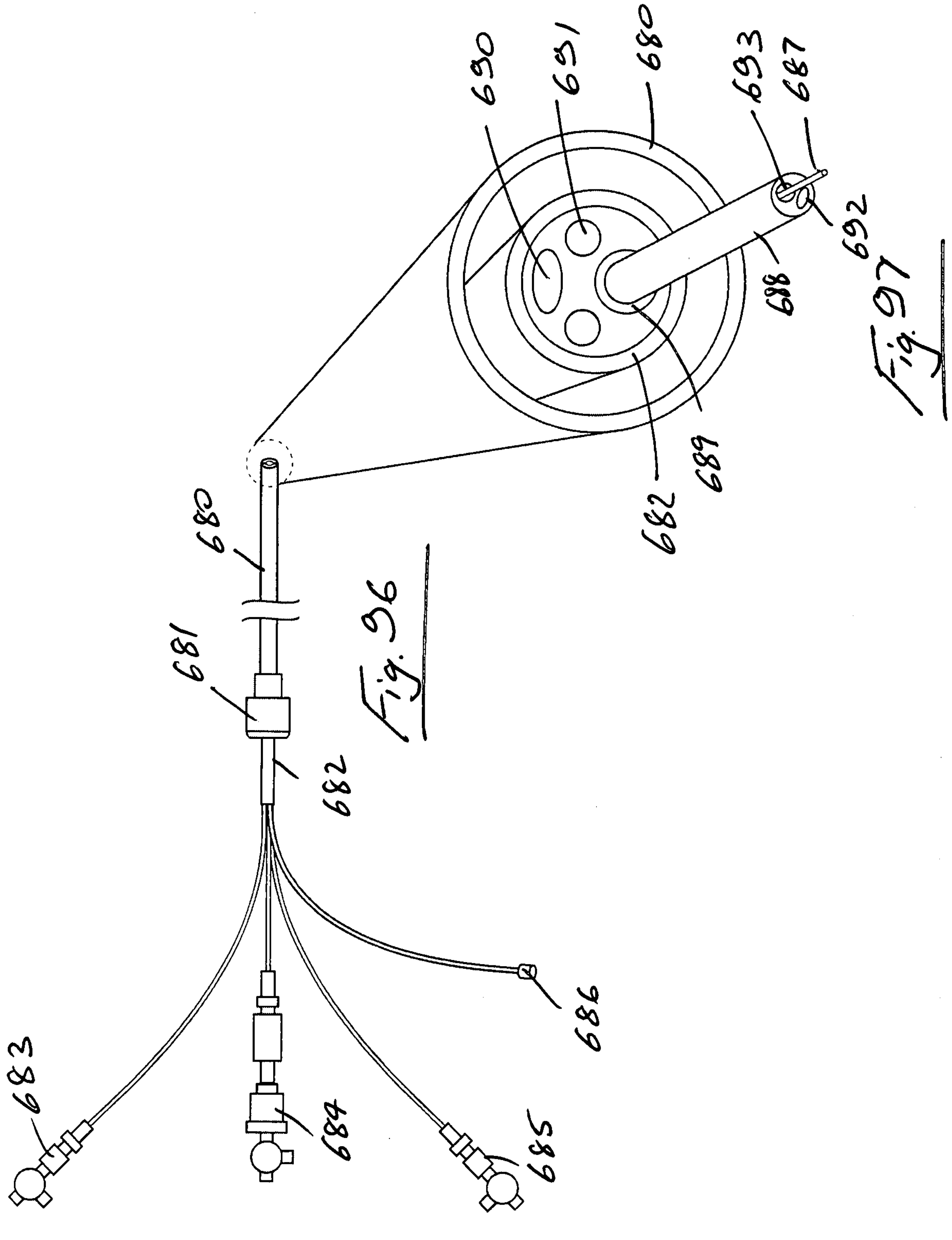

Fig. 96

Fig. 97

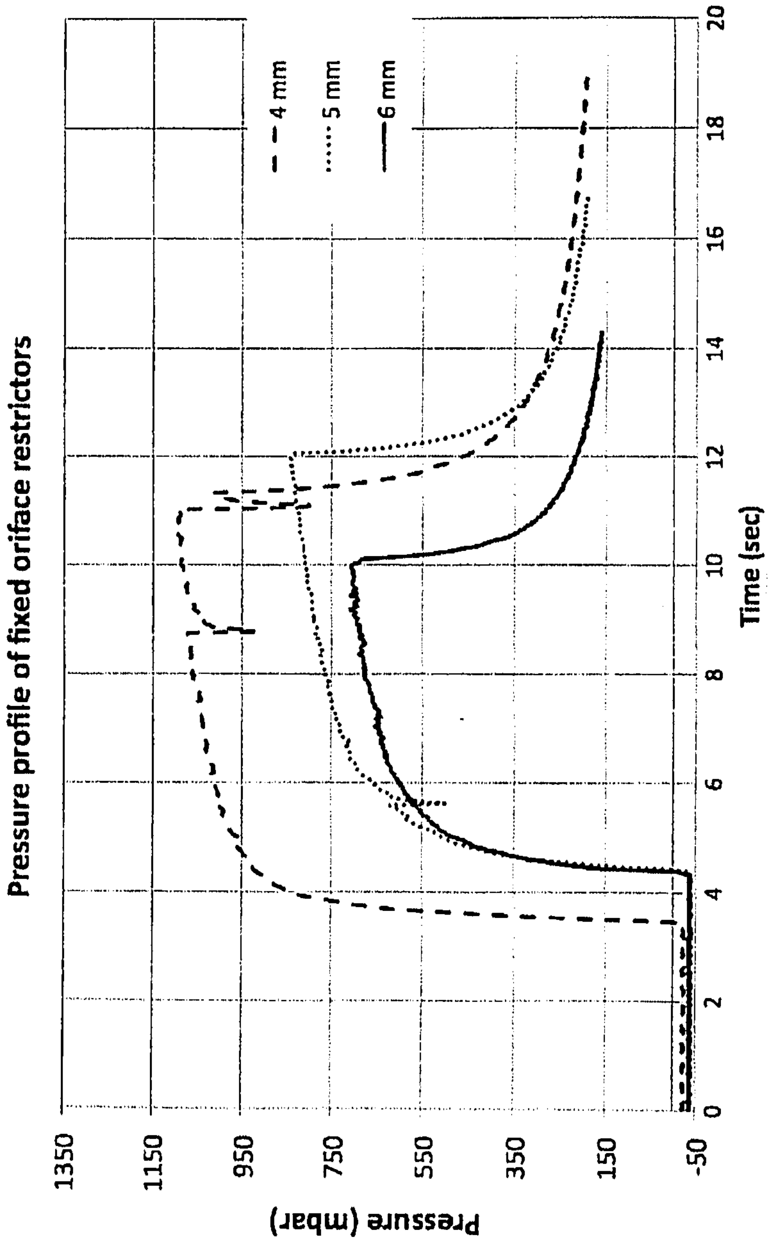
Pressure profile of fixed oriface restrictors
Pressure (mbar)
Time (sec)
4 mm
5 mm
6 mm
1350
1150
950
750
550
350
150
-50
0
2
4
6
8
10
12
14
16
18
20

Fig. 98

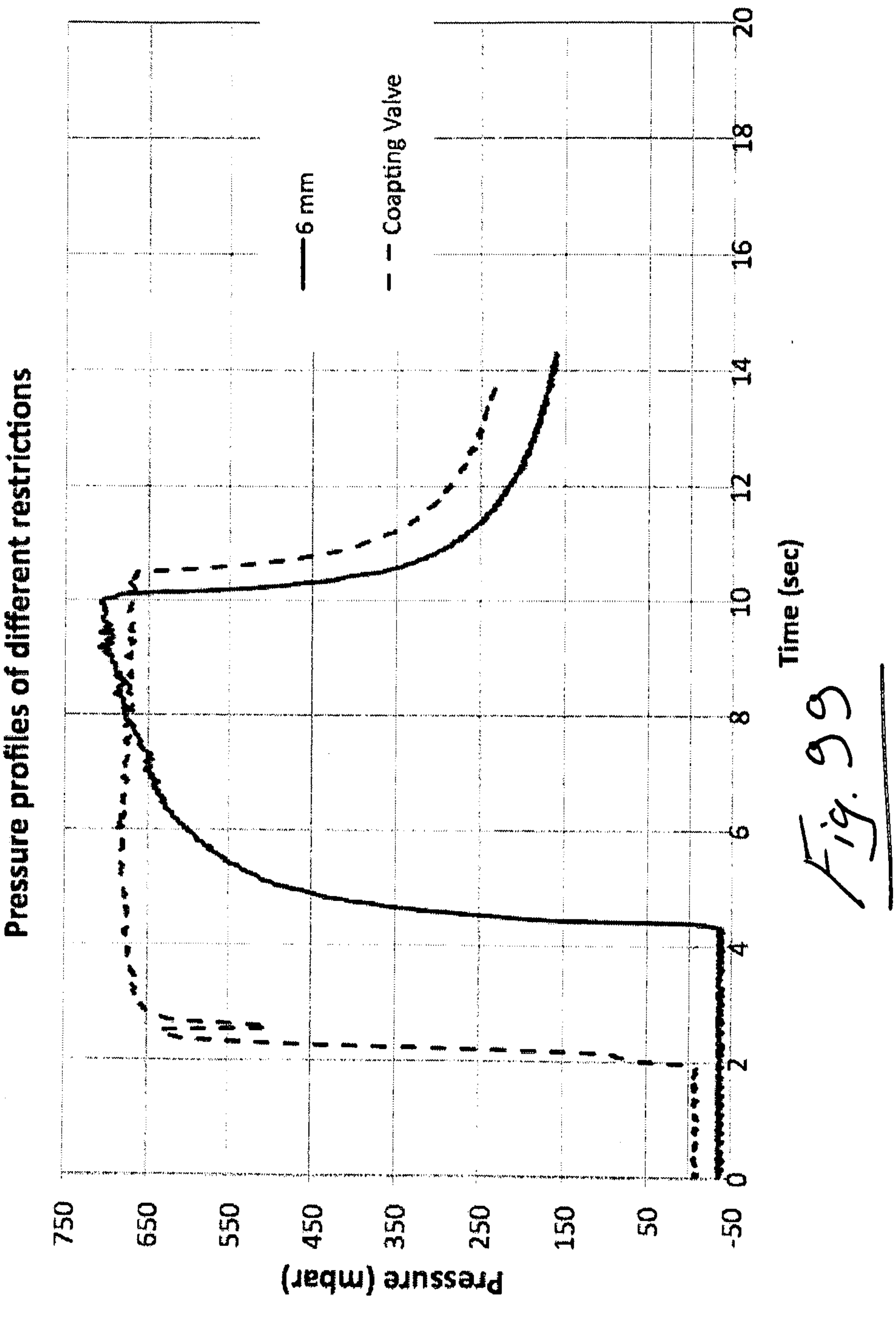
Pressure profiles of different restrictions
Pressure (mbar)
750
650
550
450
350
250
150
50
-50
Time (sec)
0
2
4
6
8
10
12
14
16
18
20
6 mm
Coapting Valve

Fig. 99

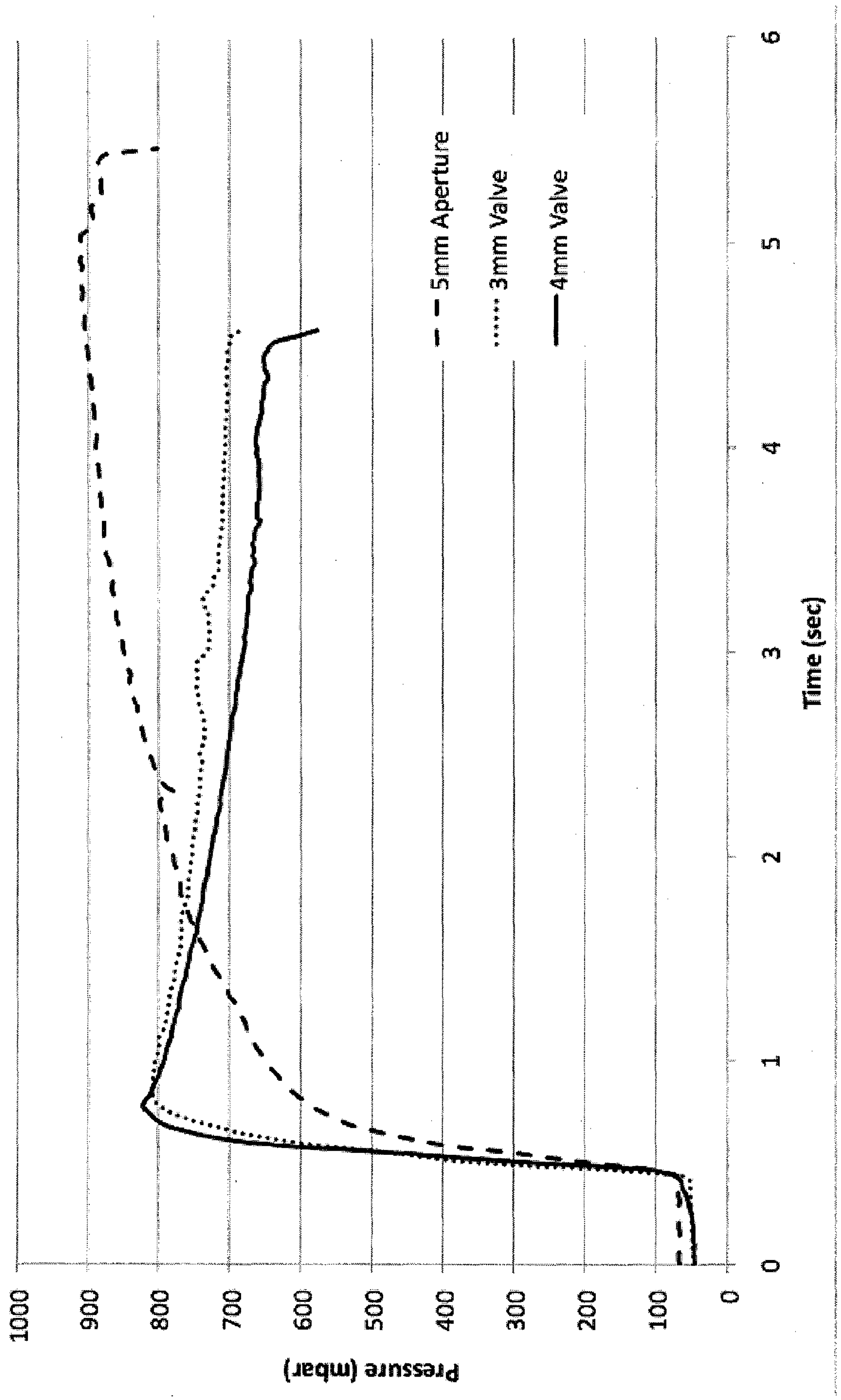
Pressure (mbar)
1000
900
800
700
600
500
400
300
200
100
0
Time (sec)
0
1
2
3
4
5
6
5mm Aperture
3mm Valve
4mm Valve

Fig. 100

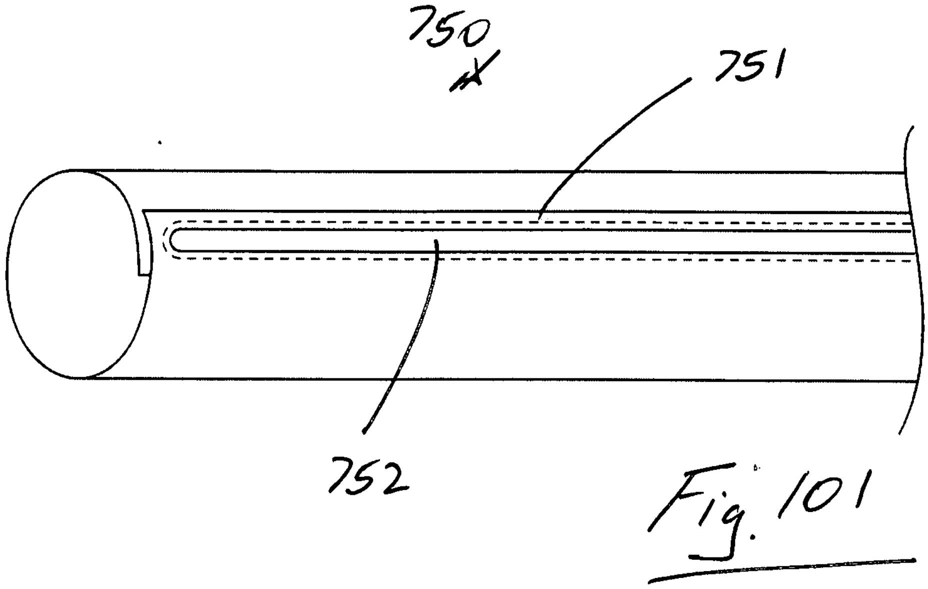


Fig. 101

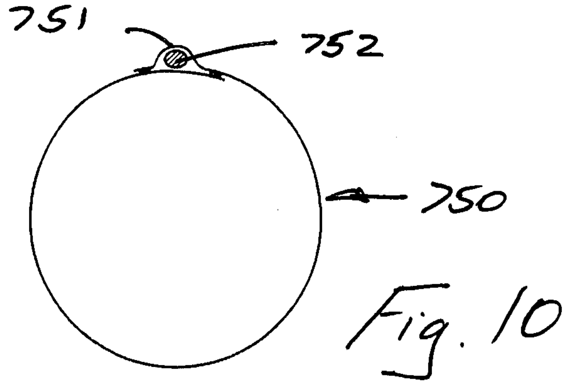


Fig. 102

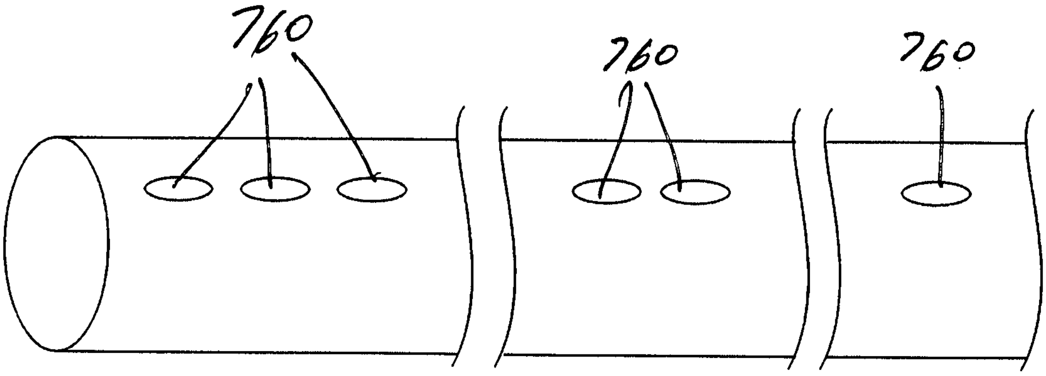


Fig. 103

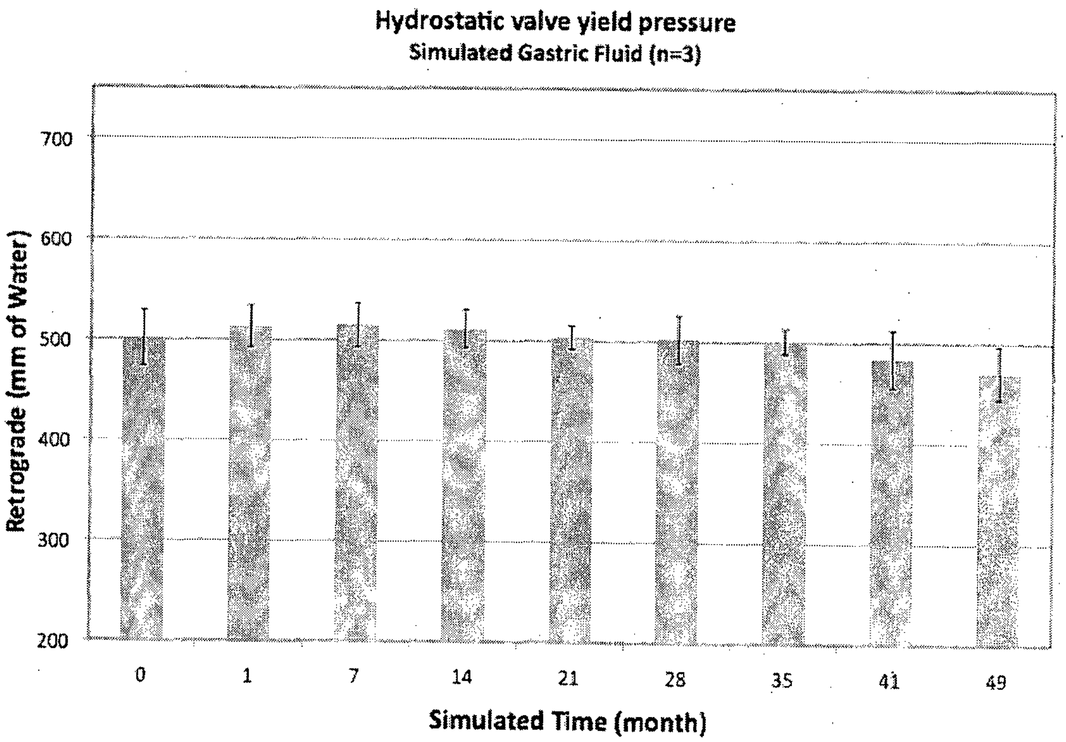
Hydrostatic valve yield pressure
Simulated Gastric Fluid (n=3)
700
600
500
400
300
200
Retrograde (mm of Water)
0
1
7
14
21
28
35
41
49
Simulated Time (month)

Fig. 104

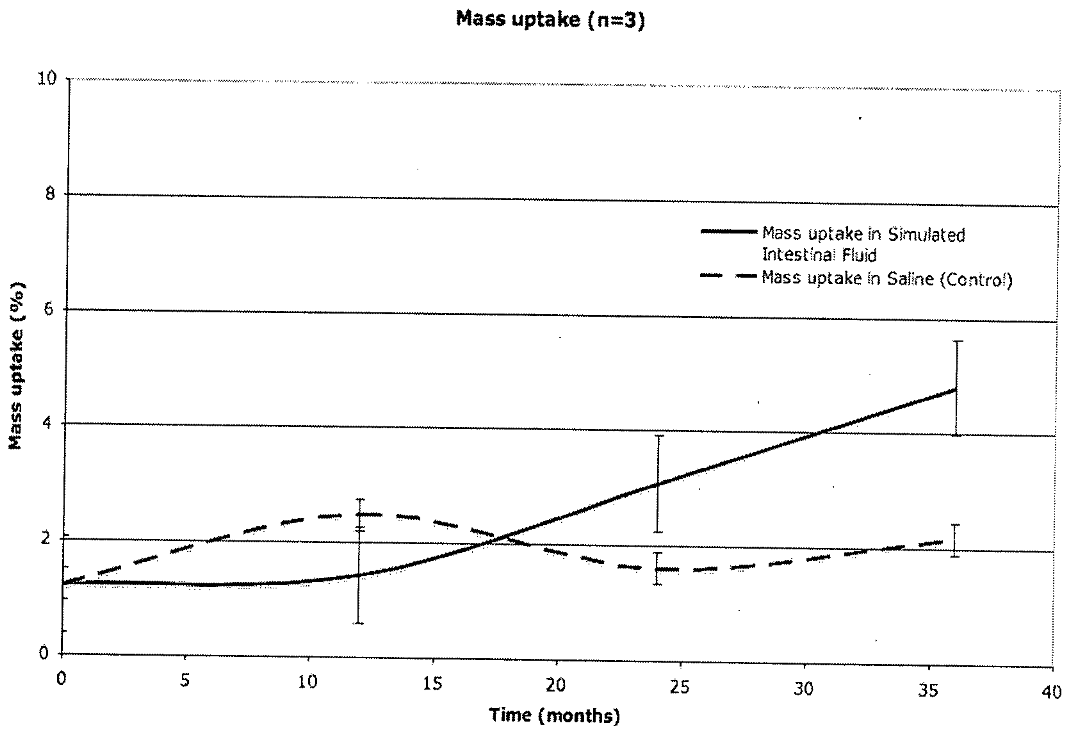
Mass uptake (n=3)
Mass uptake (%)
Time (months)
0
2
4
6
8
10
0
5
10
15
20
25
30
35
40
Mass uptake in Simulated Intestinal Fluid
Mass uptake in Saline (Control)

Fig. 105

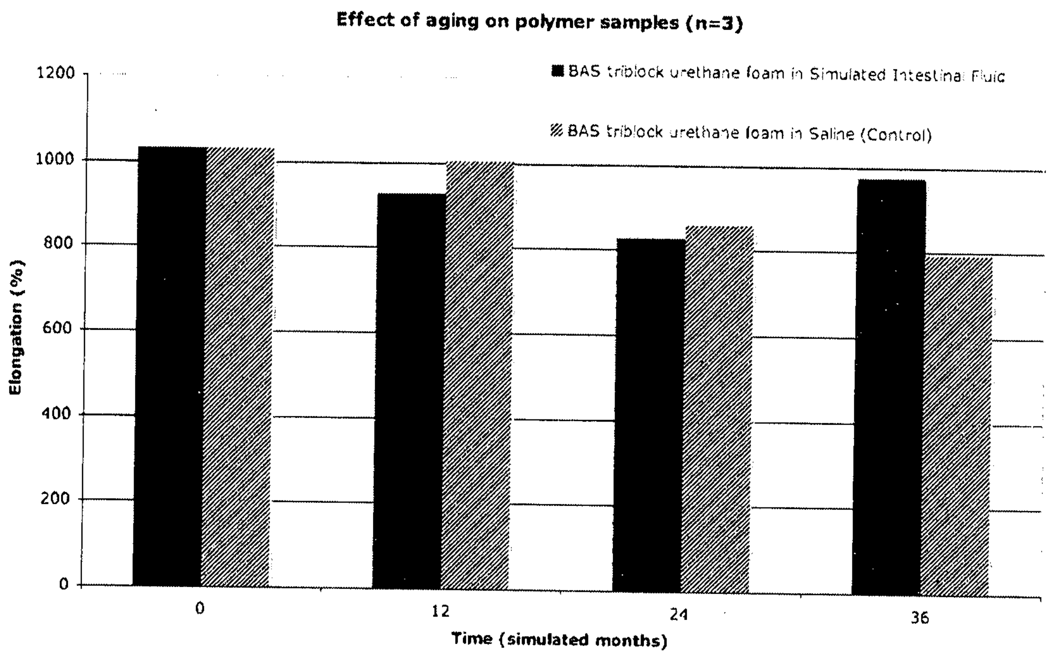
Effect of aging on polymer samples (n=3)
BAS triblock urethane foam in Simulated Intestinal Fluid
BAS triblock urethane foam in Saline (Control)
Elongation (%)
1200
1000
800
600
400
200
0
0
12
24
36
Time (simulated months)

Fig. 106

Prior Art

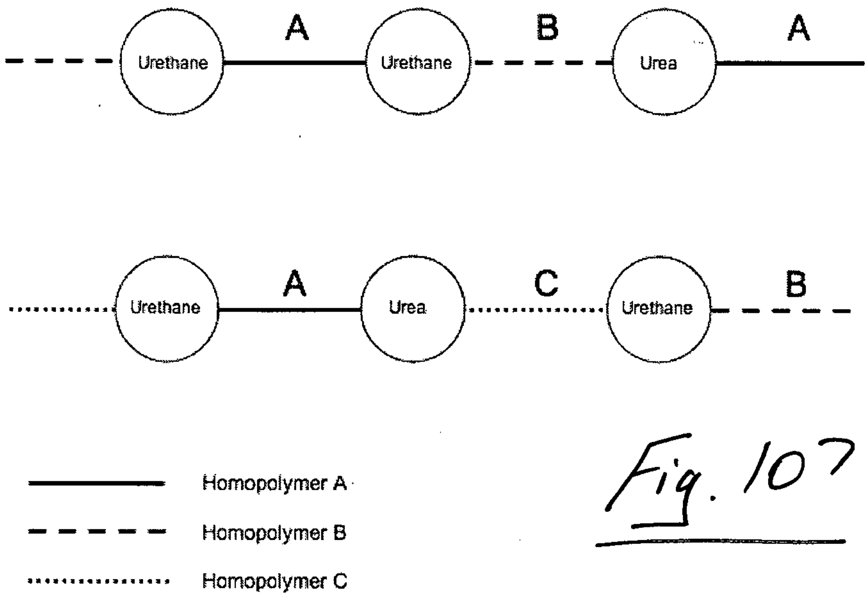


Fig. 107

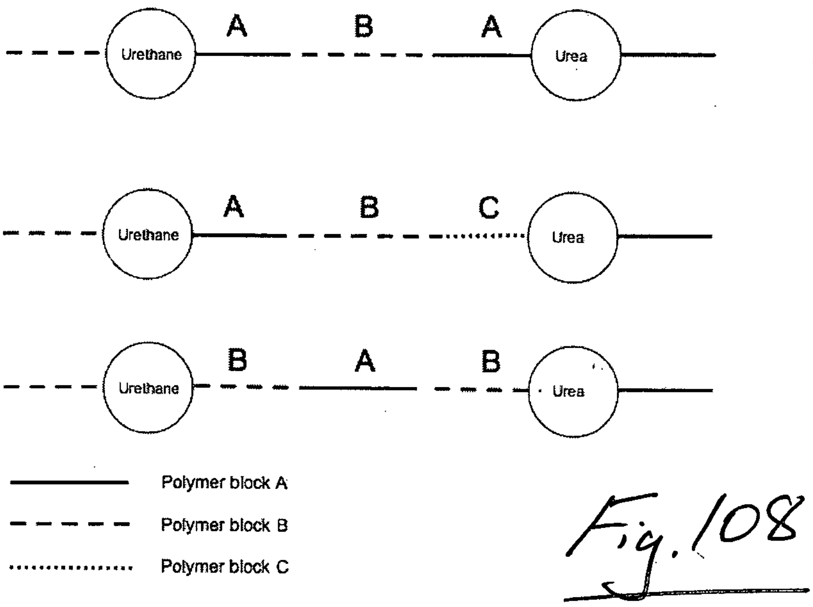


Fig. 108

A B A

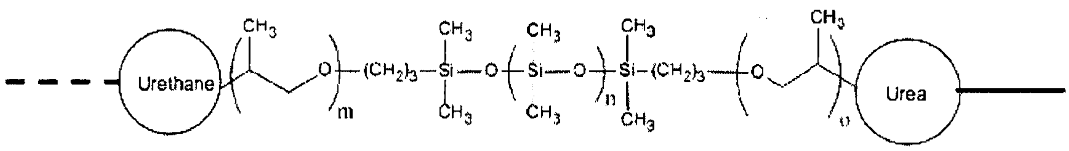

Or

A B C

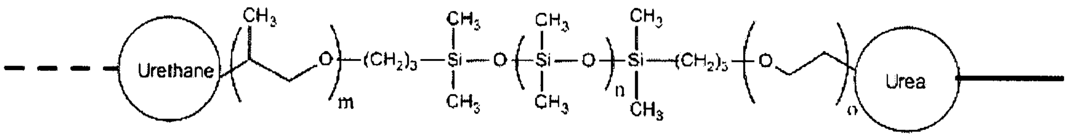

Fig. 109

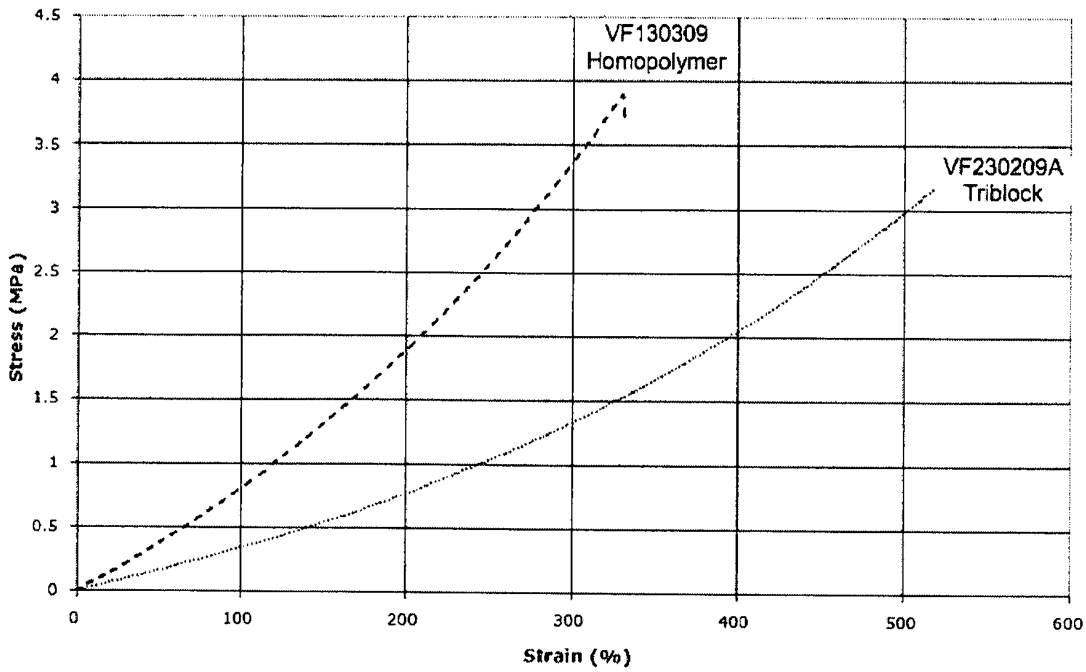


Fig. 110

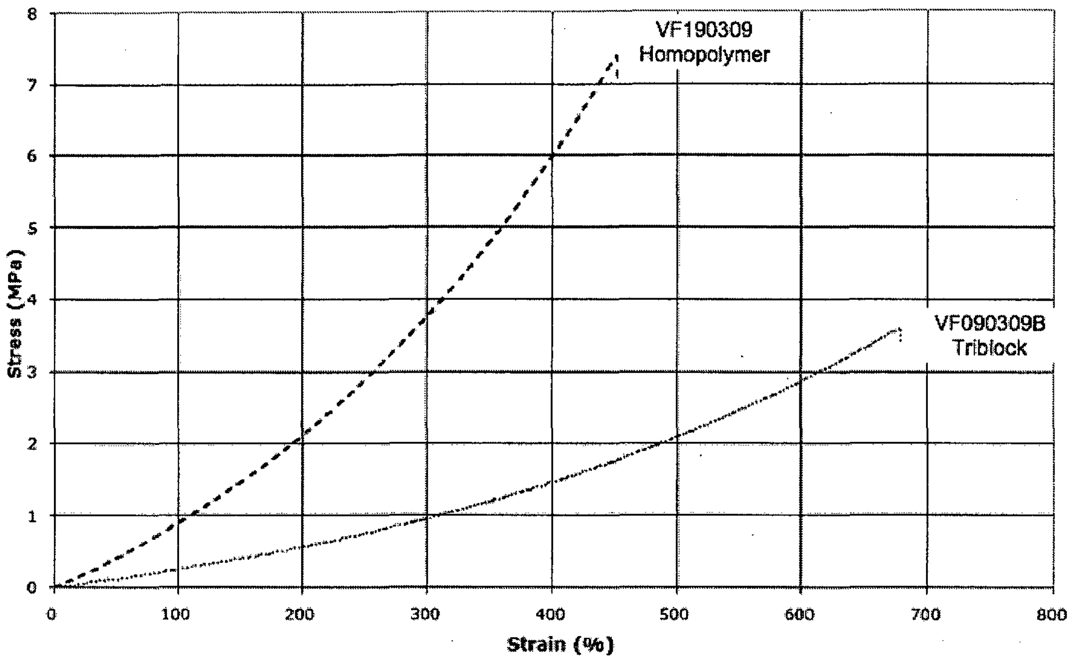


Fig. 111

Tensile strength change

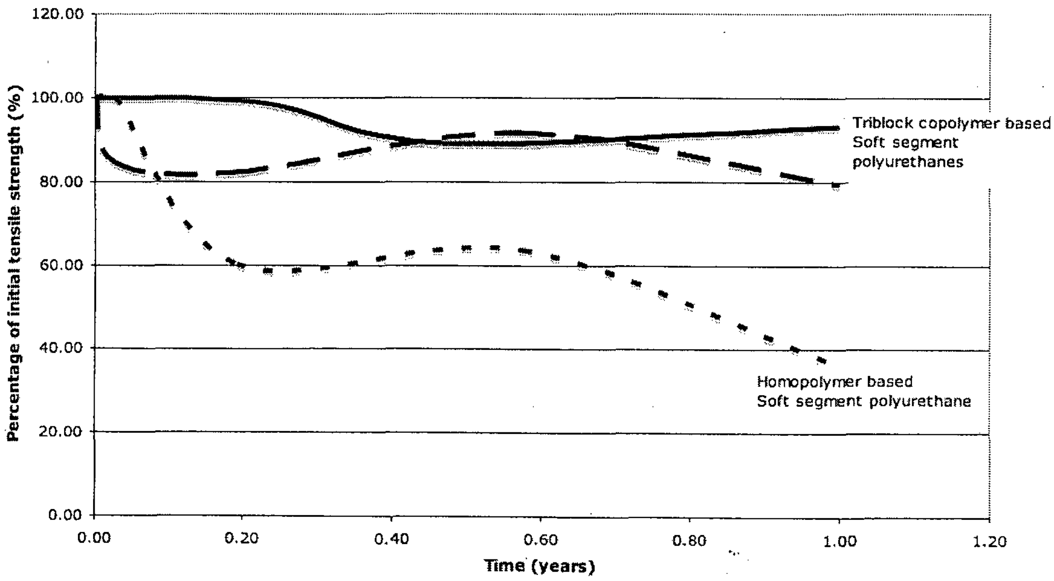


Fig. 112

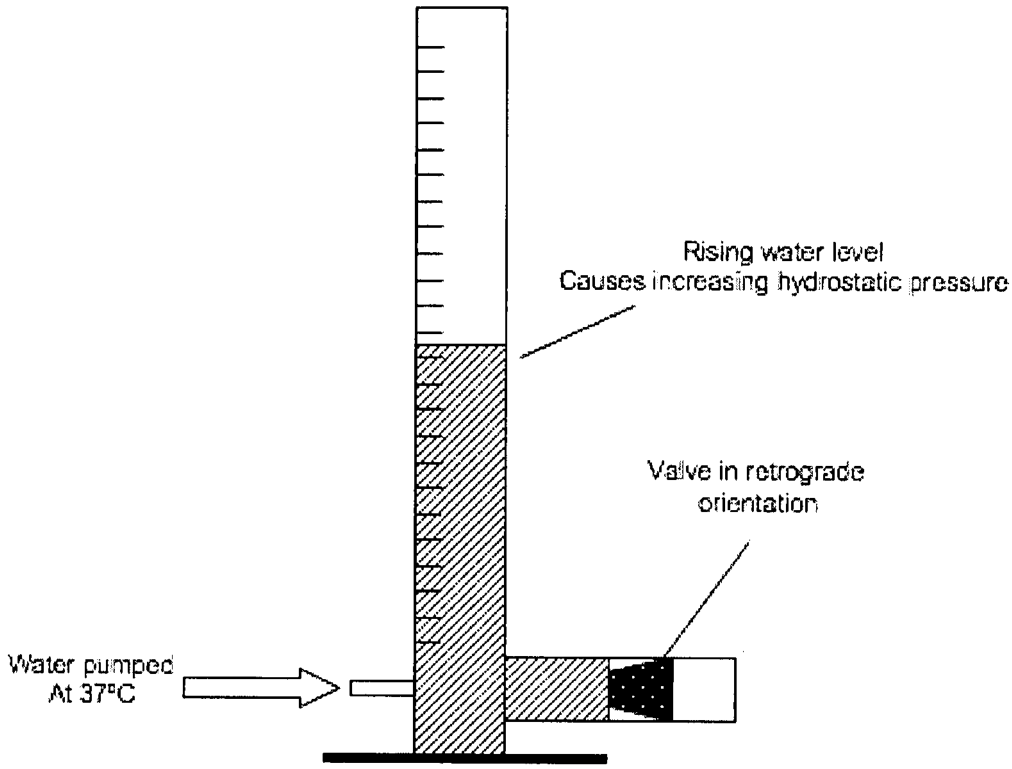
Rising water level
Causes increasing hydrostatic pressure
Valve in retrograde
orientation
Water pumped
At 37°C

Fig. 113

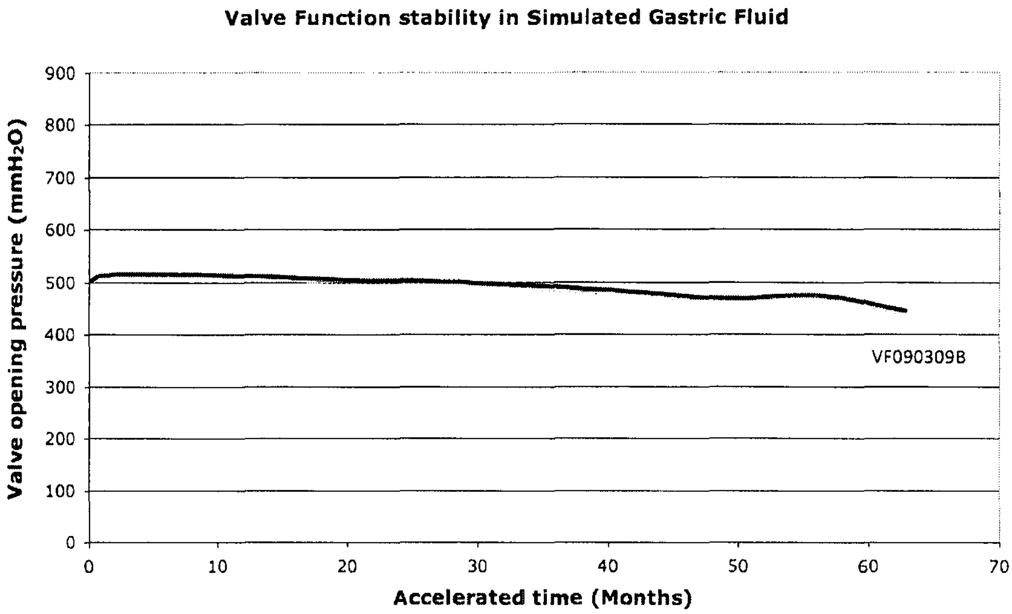


Fig. 114

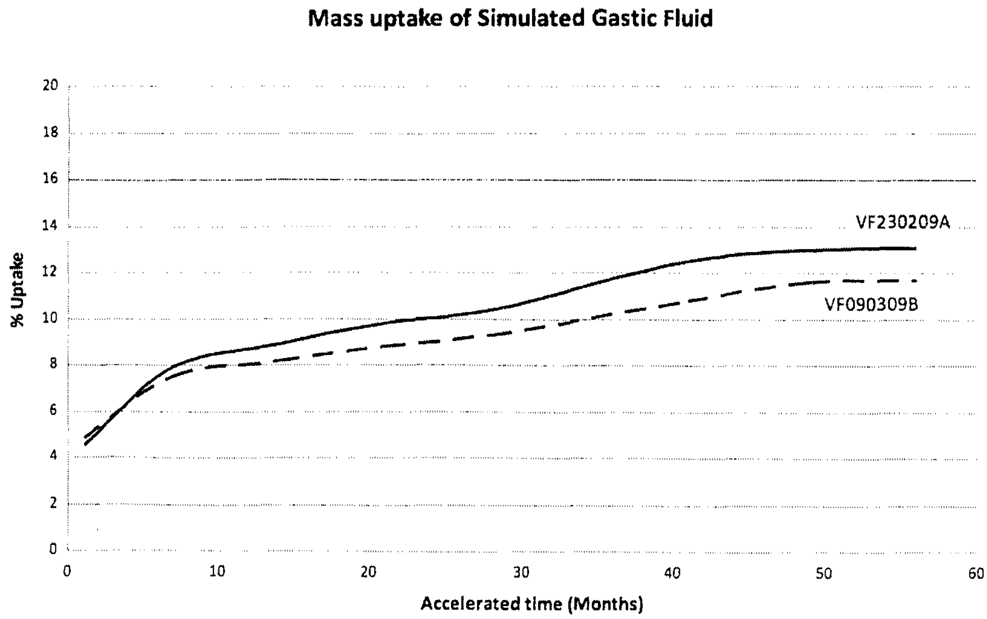


Fig. 115

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2009126268 A
- US 2008109087 A
- US 2007198048 A
- WO 2007107990 A
- US 2005267499 A
- US 2005125075 A
- WO 2008121409 A
- US 2006064983 A
- US 20050125075 A
- US 20070198048 A
- US 20100137998 A

### Non-patent literature cited in the description

- Periodic Table of the Elements, CAS version, Handbook of Chemistry and Physics
- **THOMAS SORRELL.** Organic Chemistry. University Science Books, 1999
- March's Advanced Organic Chemistry. John Wiley & Sons, 2001
- **T. W. GREENE ; P. G. M. WUTS.** Protecting Groups in Organic Synthesis. John Wiley & Sons, 1999
- **ISMAIL, J. ; BANCEWICZ, J. BAROW.** Yield pressure, anatomy of the cardia and gastro-oesophageal reflux. *British Journal of Surgery,* 1995, vol. 82, 943-947
- **DEMEESTER TR ; JOHNSON LF ; JOSEPH GJ et al.** Patterns of Gastroesophageal Reflux in Health and Disease. *Ann. Surg.,* October 1976, 459-469
- **PANDOLFINO JE ; RICHTER JE ; OURS T et al.** Ambulatory Esophageal pH Monitoring Using a Wireless System. *Am. J. Gastro,* 2003, vol. 98, 4
- **MAHMOOD Z ; MCMAHON BP ; ARFIN Q et al.** Results of endoscopic gastroplasty for gastroesophageal reflux disease: a one year prospective follow-up. *Gut,* 2003, vol. 52, 34-9
- **PARK PO ; KJELLIN T ; APPEYARD MN et al.** Results of endoscopic gastroplasty suturing for treatment of GERD: a multicentre trial. *Gastrointest endosc,* 2001, vol. 53, AB115
- **FILIPI CJ ; LEHMAN GA ; ROTHSTEIN RI et al.** Transoral flexible endoscopic suturing for treatment of GERD: a multicenter trial. *Gastrointest endosc,* 2001, vol. 53, 416-22
- **ARTS J ; SLOOTMAEKERS S SIFRIM D et al.** Endoluminal gastroplication (Endocinch) in GERD patient's refractory to PPI therapy. *Gastroenterology,* 2002, vol. 122, A47
- **THOMAS SORRELL.** Organic Chemistry. Science Books, 1999